# EUROPEAN PATENT APPLICATION

(11) **EP 4 663 228 A2**
(43) Date of publication of application: **17.12.2025**
(21) Application number: 25210360.1
(22) Date of filing: 25.02.2021
(51) Int. Cl.: A61M 25/00

(54) **EXPANDABLE SHEATH FOR INTRODUCING AN ENDOVASCULAR DELIVERY DEVICE INTO A BODY**

(30) Priority: 27.02.2020 US 202062982253 P; 03.11.2020 US 202063109171 P
(62) Divisional of application: 21713225.7
(71) Applicant: Edwards Lifesciences Corporation, Irvine, CA 92614 (US)
(72) Inventor: Tran, Sonny, IRVINE, CA, 92614 (US); Lee, Jeong Soo, IRVINE, CA, 92614 (US); Saleh, Nasser William, IRVINE, CA, 92614 (US); Tamir, Ilan, IRVINE, CA, 92614 (US); Scherb, Daniel J., IRVINE, CA, 92614 (US); Fine, Maxwell Harrison, IRVINE, CA, 92614 (US); Gowdar, Alpana Kiran, IRVINE, CA, 92614 (US); Tran, Tri D., IRVINE, CA, 92614 (US); Avery, Neal H., IRVINE, CA, 92614 (US); Ghanbari, Sarah, IRVINE, CA, 92614 (US); Trinh, Uy D., IRVINE, CA, 92614 (US); Mak, Sovanpheap, IRVINE, CA, 92614 (US); Bulman, Erik, IRVINE, CA, 92614 (US); Mercado, Raymond, IRVINE, CA, 92614 (US)
(74) Representative: Eisenführ Speiser

(57) **Abstract**

Aspects of an expandable sheath can be used in conjunction with a catheter assembly to introduce a prosthetic device, such as a heart valve, into a patient. Such aspects can minimize trauma to the vessel by allowing for temporary expansion of a portion of the introducer sheath to accommodate the delivery apparatus, followed by a return to the original diameter once the prosthetic device passes through. Some aspects can include a sheath with a variable diameter inner liner wound in a spiral slidable configuration and an outer layer. The disclosed sheath is configured to expand from a predetermined rest diameter *dᵣ* to an expanded diameter *dₑ* during application of a radial outward force by passage of a medical device through the sheath.

## Description

### CROSS-REFERENCE TO RELATED APPLICATIONS

This application claims the benefit of U.S. Provisional Application No. 62/982,253, filed February 27, 2020, and U. S. Provisional Application No. 63/109,171, filed November 3, 2020, the contents of which are incorporated herein by reference in its entirety

### FIELD

The present application concerns aspects of a sheath for use with catheter-based technologies for repairing and/or replacing heart valves, as well as for delivering a prosthetic device, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (*e.g*., the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath, and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

However, delivery and/or removal of prosthetic devices and other material to or from a patient still poses a significant risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

Accordingly, there remains a need in the art for an improved introducer sheath for endovascular systems used for implanting valves and other prosthetic devices.

### SUMMARY

Aspects of the present expandable sheath can minimize trauma to the vessel by allowing for temporary expansion of a portion of the introducer sheath to accommodate a delivery system, followed by a return to the original diameter once the delivery system passes through. Some aspects can comprise a sheath with a smaller profile than that of prior art introducer sheaths. Furthermore, certain aspects can reduce the length of time a procedure takes, as well as reduce the risk of a longitudinal or radial vessel tear or plaque dislodgement because only one sheath is required, rather than several different sizes of sheaths. Aspects of the present expandable sheath can require only a single vessel insertion, as opposed to requiring multiple insertions for the dilation of the vessel.

In one aspect, disclosed is a sheath for introducing a prosthetic device comprises an inner liner and an outer layer. At least a portion of the sheath can be designed or configured to locally expand from a first diameter (rest diameter) to a second diameter (expanded diameter) as the prosthetic device is pushed through a lumen of the sheath, and then at least partially return to the first diameter once the prosthetic device has passed through.

Also disclosed herein is an aspect directed to a sheath for delivering a medical device, wherein the sheath has a proximal and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; an outer layer having an inner surface and an outer surface and extending about at least a portion of the variable diameter inner liner such that the inner surface of the outer layer is positioned adjacent to the outer surface of the inner liner, wherein the outer layer comprises: a braid or coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

Also disclosed herein is an aspect comprising methods of making a sheath having a proximal and a distal end. In certain aspect, the method of making such a sheath comprises forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid or coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

Also disclosed are additional or alternative aspects of a sheath for delivering a medical device, wherein the sheath has a proximal and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; an outer layer having an inner surface and an outer surface and extending about at least a portion of the variable diameter inner liner such that the inner surface of the outer layer is positioned adjacent to the outer surface of the inner liner, wherein the outer layer comprises: at least one layer of a first elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Also disclosed are aspects directed to methods of making a sheath having a proximal and a distal end and comprising: forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: at least one layer of a first elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Also disclosed herein is an aspect directed to a sheath for delivering a medical device, wherein the sheath has a proximal and a distal end and comprises:
a) an inner liner defining a lumen having a first rest diameter *dᵣ* and a second expanded diameter *dₑ*, wherein the lumen is configured to receive and pass through a medical device, wherein the inner liner comprises a sheet comprising a first portion having a first surface and an opposite second surface, wherein a first end of the first portion splits into a first segment having a first surface and an opposite second surface and a third segment having a first surface and an opposite second surface, and wherein a second end of the first portion extends into a second segment having a first surface and an opposite second surface, wherein the sheet is rolled into a spiral configuration, such that at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment, wherein the first surface of the first portion extends into the first surface of the first segment, the second segment and the third segment, and wherein the second surface of the first portion extends into the second surface of the second and the third segments, wherein each segment is configured to slidably move along each other upon passage of the medical device through the lumen; wherein the sheet comprises a polymer layer; and b) an outer layer.

Also disclosed herein is an aspect directed to a sheath for delivering a medical device, wherein the sheath has a proximal end and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; wherein the sheet comprises a polymer layer; an outer layer having a predetermined thickness and having an inner surface and outer surface, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In one aspects, disclosed herein is a sheath for delivering a medical device, wherein the sheath has a proximal and a distal end and comprises: a variable diameter inner liner comprising a sheet comprising a polymer layer comprising a compound material, wherein the compound material comprises a polyolefin present in an amount from greater than 0 wt% to less than 100 wt % based on a total weight of the compound and a lubricious filler present in an amount from about 5 wt % to about 20 wt% of a total weight to the compound material, wherein the sheet is rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface, wherein the inner surface of the outer layer is disposed on the outer surface of the spiral configuration of the inner liner; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In one aspect, disclosed herein is a sheath for delivering a medical device, wherein the sheath has a proximal and a distal end, wherein the sheath comprises a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface, wherein a lubricious liner is disposed between the outer surface of the inner liner and the inner surface of the outer layer, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Still in further aspect, disclosed is a sheath for delivering a medical device, wherein the sheath has a proximal and a distal end and comprising: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; a tie layer disposed at the inner surface of the sheet, such that when the sheet is in the spiral configuration, the tie layer is disposed between the overlapping portions and at at least an innermost surface of the lumen; and/or a tie layer disposed at the outer surface of the sheet such that when the sheet is in the spiral configuration, the tie layer is disposed between the overlapping portions and at an outermost surface of the inner liner, at least one lubricious liner disposed on the tie layer; and an outer layer having a predetermined thickness and having an inner surface and outer surface, wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Also disclosed herein is an aspect directed to a sheath for delivering a medical device, wherein the sheath has a proximal and a distal end and comprising: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an elongated tube forming an outer layer of the sheath that is positioned at at least the proximal end of the sheath and extending along at least a portion of a length of the sheath, having an inner surface and an outer surface, and wherein the elongated tube comprises a) a first polymer layer, wherein the first polymer layer comprises a first compound composition comprising i) from greater than 0 wt% to less than 100 wt% of a polymer comprising a polyether block amide, a polyurethane, or a combination thereof based on a total weight of the first compound composition; ii) less than about 65% of an inorganic filler based on a total weight of the first compound composition; and iii) up to about 20 % of a solid lubricant filler based on a total weight of the first compound composition; and b) a second polymer layer comprising polyurethane; wherein the elongated tubing is a bump tubing and is a coextrusion of the first and the second polymers; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In still further aspects disclosed is a sheath for delivering a medical device, wherein the sheath has a proximal and a distal end and comprising: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an elongated tube forming an outer layer of the sheath that is positioned at at least the proximal end of the sheath and extending along at least a portion of a length of the sheath, having an inner surface and an outer surface, and wherein the elongated tube comprises a) a first polymer layer, wherein the first polymer layer comprises a first compound composition comprising i) from greater than 0 wt% to less than 100 wt% of a polymer comprising a polyether block amide, a polyurethane, or a combination thereof based on a total weight of the first compound composition; ii) less than about 65% of an inorganic filler based on a total weight of the first compound composition; and iii) up to about 20 % of a solid lubricant filler based on a total weight of the first compound composition; and b) a second polymer layer comprising polyurethane; wherein the second polymer layer at least partially overlies the first polymer layer; and wherein the first polymer is disposed at the proximal end of the sheath and has a length that is shorter than the length of the second polymer layer; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Also disclosed herein an aspect of a sheath for delivering a medical device, wherein the sheath has a proximal and a distal end and comprising: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface, wherein at least a portion of an innermost surface of the outer layer is bonded to at least a portion of an outermost surface the inner liner, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In some aspects, disclosed is a sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface; and a reinforcing jacket having a proximal end and a distal end and wherein the reinforcing jacket is disposed over at least a portion of an outer surface of the outer layer; wherein the reinforcing jacket comprises an elastomer and a reinforcing element; and wherein the distal end of the reinforcing jacket substantially seamlessly bonded to at least a portion of the outer surface of the outer layer; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In yet other aspects disclosed is a sheath for delivering a medical device, where the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface and extending from the proximal end of the sheath to the distal end of the sheath; and a ballooning guard having a proximal end and a distal end and is disposed over at least a portion of the outer layer and wherein the ballooning guard is configured to remain outside of a subject's vessel and to maintain hemostasis; wherein the proximal end of the ballooning guard is connected to a most proximal portion of the outer layer and/or a hub of the sheath wherein the distal end of the ballooning guard radially circumscribes at least a portion of the outer layer and wherein the distal end is not bonded to the outer layer; and wherein the ballooning guard is configured to adjust a length of the guard as a function of an insertion depth of the inner liner and outer layer of the sheath into a subject's vessel

Still further disclosed are aspects directed to methods of making a sheath having a proximal and a distal end and comprising: a) forming an inner liner by: i) providing a double lumen tubing comprising two channels extruded in a single-tube configuration; wherein the double lumen comprises at least one polymer layer; wherein a first channel has an inner surface and an outer surface; wherein a second channel has an inner surface and an outer surface; wherein the second channel is positioned within the first channel such that at least a portion of a circumference of the first channel and at least a portion of a circumference of the second channel have at least one shared inner surface and at least one shared outer surface; and wherein the outer surface of the first channel defines an outer surface of the double-lumen tubing; ii) longitudinally cutting the first channel at a portion of the circumference of the first channel that is not shared with the circumference of the second channel to form a first sheet having a first edge and a second edge and the second channel disposed longitudinally along at least a portion of the first sheet; such that at least a portion of the circumference of the second channel has at least a portion that has a shared surface with the first sheet along a length of the sheet and along a length of the second channel; iii) longitudinally cutting the second channel at a portion of the circumference of the second channel that abuts the shared surface with the first sheet to form a second sheet having a first edge and a second edge, wherein the second edge is defined by a portion of the shared surface with the first sheet; iv) rolling the first and the second sheets into a spiral configuration such that: I) the shared surface between the second and the first sheets form a first portion of an inner liner having a first surface and an opposite second surface; II) at least a portion of the second sheet forms a first segment of an inner liner having a first surface and an opposite second surface; III) wherein a portion of the first sheet adjacent to the second end of the second sheet and extending to the first end of the first sheet forms a second segment of an inner liner having a first surface and an opposite second surface; and IV) wherein a portion of the first sheet adjacent the second end of the second sheet and extending to the second end of the first sheet forms a third segment of an inner liner having a first surface and an opposite second surface; wherein in the spiral configuration, at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment; wherein the first surface of the first portion extends into the first surface of the first segment, the second segment and the third segment, and wherein the second surface of the first portion extends into the second surface of the second and the third segments, and b) disposing an outer layer to form the sheath; wherein the formed sheath is configured to extend from a first rest diameter *dᵣ* to a second expanded diameter *dₑ* and back to a diameter substantially identical to the first rest diameter dᵣ upon passage of the medical device by slidably moving each segment along each other.

Also disclosed herein is a method of making a sheath having a proximal and a distal end and comprising: a) forming a variable diameter inner liner by: i) providing an elongated single lumen tubing comprising at least one polymer layer; ii) longitudinally cutting at least a portion of a circumference of the elongated single lumen tubing to form a sheet having a first longitudinal edge and an opposite second longitudinal edge and having an inner surface and an outer surface; iii) forming a variable diameter inner liner by rolling the sheet in a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; and b) disposing an outer layer over at least a portion of the outer layer of the inner liner to form the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

In some aspects disclosed is a method of forming a sheath comprising: a) positioning an elongated tubing on a mandrel; b) disposing an amount of a lubricant on an outer surface of the elongated tubing in a predetermined pattern; c) curing the lubricant; d) cutting an elongated tubing at a portion of a circumference of the tubing along a length of the tubing to form a sheet having an outer surface and inner surface and a first longitudinal edge and a second longitudinal edge; wherein the inner surface forms a sheath lumen, and at least a portion of the outer surface comprises the lubricant disposed in the predetermined pattern; e) rolling the sheet into a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet; f) disposing an outer layer having an inner surface and an outer surface over the outer surface of the inner liner such that the lubricant is disposed between at least a portion of the inner surface of the outer layer and at least a portion of the outer surface of the inner layer to form the sheath; wherein the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

While in other aspects disclosed is a method of making a sheath having a proximal end and a distal end and comprising: a) extruding a tubular body to form an elongated tubing comprising a compound material, wherein the compound material comprises a polyolefin present in an amount from greater than 0 wt% to less than 100 wt % based on a total weight of the compound and a lubricious filler present in an amount from about 5 wt % to about 20 wt% of a total weight to the compound material; b) cutting the elongated tubing at at least a portion of the circumference along a length of the elongated tubing to form a sheet; c) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and d) positioning an outer layer having a predetermined thickness and having an inner surface and outer surface, wherein the inner surface of the outer layer is disposed on the outer surface of the spiral configuration of the inner liner; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In still further aspect disclosed is a method of making a sheath having a proximal end and a distal end and comprising: a) co-extruding a polymer layer and a tie layer to form an elongated tubing; b) positioning at least one lubricious liner at an outer surface of the inner liner; c) cutting the elongated tubing at at least a portion of the circumference along a length of the elongated tubing to form a sheet; d) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and e) positioning an outer layer having a predetermined thickness and having an inner surface and outer surface on the least one lubricious liner to form the sheath; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

Still in a further aspect, disclosed is a method of making a sheath having a proximal end and a distal end and comprising: a) co-extruding a polymer layer and a tie layer to form an elongated tubing; b) positioning at least one lubricious liner on the tie layer; c) cutting the elongated tubing at at least a portion of the circumference along a length of the elongated tubing to form a sheet; d) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; such that when the sheet is in the spiral configuration, the tie layer is disposed between the overlapping portions and at at least an inner surface of the lumen; and/or such that when the sheet is in the spiral configuration, the tie layer is disposed between the overlapping portions and at an outermost surface of the inner liner; e) positioning an outer layer having a predetermined thickness and having an inner surface and outer surface to form the sheath, wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

Additionally or alternatively, disclosed is a method of making a sheath having a proximal end and a distal end and comprising: a) co-extruding an elongated bump tubing comprising a first polymer layer and a second polymer layer; wherein i) the first polymer layer comprises: a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; ii) the second polymer layer comprises a polyurethane; wherein the first polymer layer defines an inner surface of the tubing and the second polymer layer defines an outer surface of the tubing; b) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and c) disposing the elongated bump tubing on the sheath such that the elongated bump tubing forms an outer layer of the sheath, and wherein the elongated tube is positioned at at least the proximal end of the sheath and extending along at least a portion of a length of the sheath, wherein the elongated bump tubing is configured to reversibly expand from an initial diameter d₀ in an unexpended position to an expanded diameter dₑ in an expanded position upon passage of a medical device; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

Also disclosed is a method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing a first polymer layer comprising a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; wherein the first polymer layer is disposed over a proximal portion of the inner liner and has a length from about 5 cm to about 15 cm; c) disposing a second polymer layer comprising polyurethane over the first polymer layer, wherein the second polymer layer extends along a length of the sheath; wherein the first polymer layer and the second polymer layer together form an outer layer of the sheath that is configured to reversibly expand from an initial diameter d₀ in an unexpended position to an expanded diameter dₑ in an expanded position upon passage of a medical device; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

Alternatively or in addition, disclosed is a method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and b) disposing an outer layer over the inner liner to form the sheath; c) positioning the sheath on a mandrel configured to rotate; d) aligning the mandrel with a laser beam configured to move along a longitudinal axis of the sheath to a predetermined distance at conditions effective to form the bond; e) forming a bond at a predetermined portion of the sheath between at least a portion of an outermost surface of the inner liner and at least a portion of an innermost surface of the outer layer, wherein the predetermined portion is substantially free of a lubricant and/or a tie layer; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

Alternatively or in addition, disclosed is a method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and b) disposing an outer layer over the inner liner to form the sheath; c) positioning the sheath on a mandrel configured to rotate; d) inserting the mandrel into a radial compression head bonder comprising a collapsible aperture configured to compress the sheath to a predetermined diameter and wherein the radial compression head bonder comprises a plurality of dies, wherein at least one of a plurality of dies is heated to form the bond at the preterminal portion of the sheath; e) forming a bond at a predetermined portion of the sheath between at least a portion of an outermost surface of the inner liner and at least a portion of an innermost surface of the outer layer, wherein the predetermined portion is substantially free of a lubricant and/or a tie layer; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

In addition or in the alternative, disclosed is a method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and b) disposing an outer layer over at least a portion of the inner liner to form the sheath; c) disposing a reinforcing jacket having a proximal end and a distal end over at least a portion of the outer layer; wherein the reinforcing jacket comprises an elastomer and a reinforcing element; and d) substantially seamlessly bonding the distal end of the reinforcing jacket to at least a portion of the outer layer, wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

In addition or in the alternative disclosed is a method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and b) disposing an outer layer over at least a portion of the inner liner to form the sheath; c) disposing a ballooning guard having a proximal end and a distal end over at least a portion of the outer layer, wherein the ballooning guard is configured to remain outside of a subject's vessel and to maintain hemostasis; d) connecting the proximal end of the ballooning guard to a most proximal portion of the outer layer and/or a hub of the sheath, wherein the distal end of the ballooning guard radially circumscribes at least a portion of the outer layer and wherein the distal end is not bonded to the outer layer; and wherein the ballooning guard is configured to adjust a length of the guard as a function of an insertion depth of the inner liner and outer layer of the sheath into a subject's vessel; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

The foregoing and other features and advantages of the disclosure will become more apparent from the following detailed description, which proceeds with reference to the accompanying figures.

### BRIEF DESCRIPTION OF THE DRAWINGS

**FIGURE 1** is an elevation view of a sheath according to the present disclosure along with an endovascular delivery apparatus for implanting a prosthetic valve.
**FIGURES 2A** and **2B** are section views of aspects of a sheath for introducing a prosthetic device into a patient, and **FIGURE 2C** is a perspective view of one component of such a sheath.
**FIGURES 3A, 3B,** and **3C** are elevation views of three aspects of a sheath according to the present disclosure, having a uniform and varying rest *dᵣ* diameters.
**FIGURE 4A-4D** illustrates partial elevation views of various exemplary aspects of a braid structure with various PIC according to the present disclosure.
**FIGURES 5A** and **5B** illustrate a section view of one aspect of an exemplary inner liner: **FIGURE 5A** depicts an unexpanded configuration, while **FIGURE 5B** depicts an expanded configuration.
**FIGURES 6A-6E** **and 6H** show section views of various aspects of exemplary sheaths. **FIGURES 6F, 6G,** and **6I** show perspective views of various aspects of exemplary sheaths.
**FIGURE 7** illustrates a block diagram of one aspect of a method of making a sheath according to the present disclosure.
**FIGURE 8** illustrates a block diagram of another aspect of a method of making a sheath according to the present disclosure.
**FIGURES 9A-9K** illustrate section or side views of various method steps of the methods shown in **FIGURES 7-8****.**
**FIGURE 10** is an elevation view of an expandable sheath according to the present disclosure and representative housing.
**FIGURE 11** is an enlarged cutaway view of the distal end of the sheath of **FIGURE 10****.**

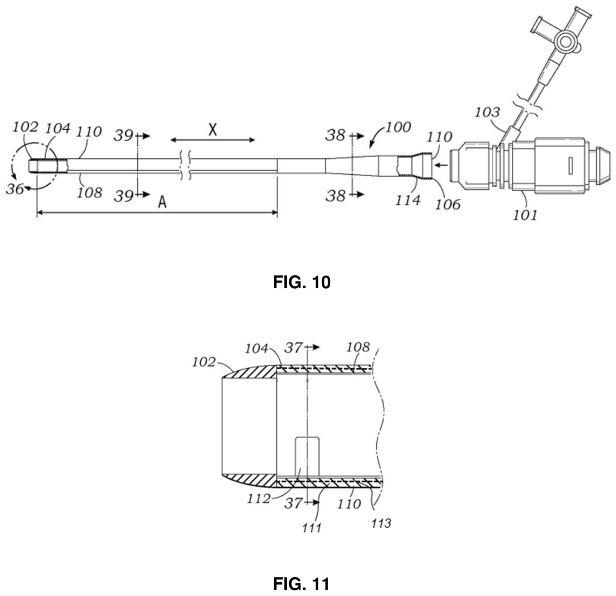

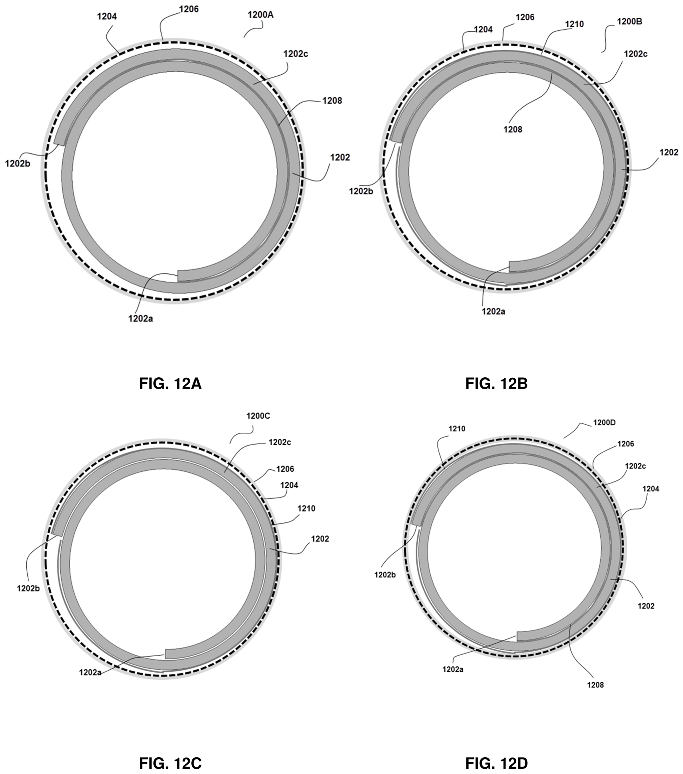

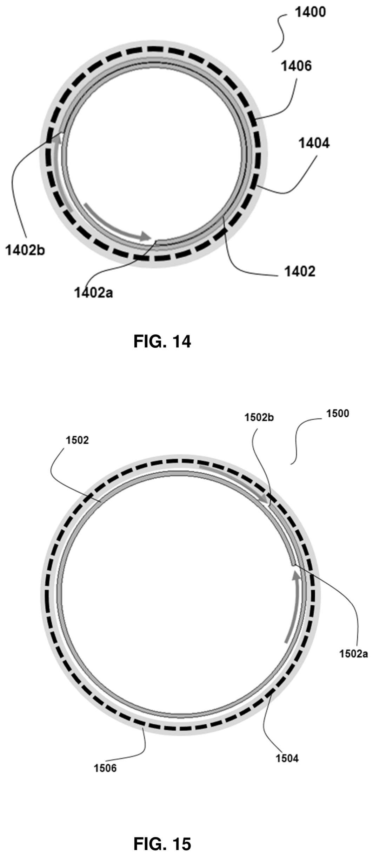

**FIGURE 12A-12D** are section views of the distal end of the exemplary sheath of **FIGURE 14****,** taken along line 37-37 in **FIGURE 11****;** **FIGURE 12A** shows a section view of the exemplary sheath with a lubricant disposed between the sliding and overlaying portions of the sheet and the braid or coil that is not embedded in the elastomeric polymer layer; **FIGURE 12B** shows a section view of the exemplary sheath with a lubricant disposed between the sliding and overlaying portions of the sheet and a lubricant disposed between the inner liner and outer layer, where the braid or coil that is not embedded in the elastomeric polymer layer; **FIGURE 12C** shows a section view of the exemplary sheath with a lubricant disposed between the inner liner and outer layer, where the braid or coil that is not embedded in the elastomeric polymer layer; with and without the lubricant; **FIGURE 12D** shows a section view of the exemplary sheath with a lubricant disposed between the sliding and overlaying portions of the sheet and a lubricant disposed between the inner liner and outer layer, where the braid or coil is at least partially embedded in the elastomeric polymer layer.

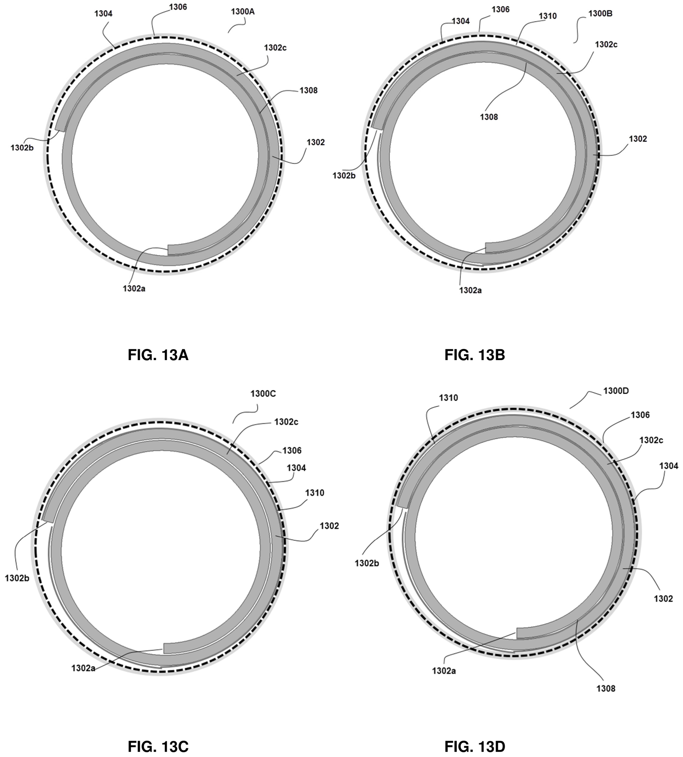

**FIGURE. 13** is a section view of a proximal section of the sheath of **FIGURE 10****,** taken along line 38-38 in **FIGURE 11****;** **FIGURE 13A** shows a section view of the exemplary sheath with a lubricant disposed between the sliding and overlaying portions of the sheet and the braid or coil that is not embedded in the elastomeric polymer layer; **FIGURE 13B** shows a section view of the exemplary sheath with a lubricant disposed between the sliding and overlaying portions of the sheet and a lubricant disposed between the inner liner and outer layer, where the braid or coil that is not embedded in the elastomeric polymer layer; **FIGURE 13C** shows a section view of the exemplary sheath with a lubricant disposed between the inner liner and outer layer, where the braid or coil that is not embedded in the elastomeric polymer layer; with and without the lubricant; **FIGURE 13D** shows a section view of the exemplary sheath with a lubricant disposed between the sliding and overlaying portions of the sheet and a lubricant disposed between the inner liner and outer layer, where the braid or coil is at least partially embedded in the elastomeric polymer layer.
**FIGURES 14** is a section view of the sheath of **FIGURE 35** in a rest (unexpanded) configuration, taken along line 39-39 in **FIGURE 11****.**
**FIGURE 15** shows a section view of the sheath in **FIGURE 14** in an expanded configuration.
**FIGURE 16** shows experimental data for an exemplary sheath in one aspect.
**FIGURE 17** shows experimental data for an exemplary sheath in another aspect.
**FIGURE 18** depicts an inner liner of an exemplary sheath in one aspect in a collapsed and unexpended configuration.
**FIGURE 19** depicts a cross-sectional view of an exemplary sheath in one aspect showing the inner liner, as shown in **FIG.18** and an outer layer.
**FIGURES 20A-C** depict exemplary manufacturing steps of the inner liner, as shown in **FIG. 18****.**
**FIGURES 21A-B** depicts an exemplary sheath in one aspect showing an inner liner and an outer layer in a collapsed and unexpended configuration: **FIG. 21** **A** illustrates a cross-sectional schematic of the sheath and **FIG. 21B** depicts a sideview schematic of the sheath.
**FIGURES 22A-22B** depict an exemplary sheath in one aspect: FIG. 22A shows a schematic of a cross-sectional view of the sheath in a collapsed configuration (left) and an expanded configuration (right); **FIG. 22B** shows snapshots of an exemplary sheath expanding during the passage of a medical device.
**FIGURE 23** depicts a cross-sectional view schematic of an exemplary sheath in one aspect.
**FIGURES 24A-24B** depict a cross-sectional view schematic of an exemplary sheath in one aspect.
**FIGURES 25A-25H** depict various inner liner combinations for an exemplary sheath: **FIGS. 25A-25D** show a cross-sectional view of various liners prior to forming an exemplary inner liner; **FIGS. 25E-25H** show various cross-sectional views of an exemplary inner liner in a spiral configuration.
**FIGURE 26** depicts a side view of an exemplary lubricant pattern disposed on an exemplary inner liner in one aspect.
**FIGURE 27** depicts a cross-sectional view of an exemplary sheath in collapsed and in an expanded configuration with bonding between an inner liner and outer liner.
**FIGURES 28A-C** depict various methods of forming a bond between an inner liner and an outer layer of an exemplary sheath.
**FIGURES 29A-F** depict schematics of a reinforcing jacket effect (**FIGS.25A-****D**) and a ballooning guard effect on patient anatomy (**FIGS. 25A-B** and **25E-F**).
**FIGURES 30A-B** depict schematics of a reinforcing jacket present in an exemplary sheath in one aspect.

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "polymer" includes aspects having two or more such polymers unless the context clearly indicates otherwise.

It is also to be understood that the terminology used herein is for the purpose of describing aspects only and is not intended to be limiting. As used in the specification and in the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

References in the specification and concluding claims to parts by weight of a particular element or component in a composition or article, denotes the weight relationship between the element or component and any other elements or components in the composition or article for which a part by weight is expressed. Thus, in a composition or a selected portion of a composition containing 2 parts by weight of component X and 5 parts by weight component Y, X and Y are present at a weight ratio of 2:5, and are present in such ratio regardless of whether additional components are contained in the composition.

A weight percent of a component, unless specifically stated to the contrary, is based on the total weight of the formulation or composition in which the component is included.

Ranges can be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It should be further understood that the endpoints of each of the ranges are significant both in relation to the other endpoint, and independently of the other endpoint.

As used herein, the term "substantially," when used in reference to a composition, refers to at least about 80%, at least about 85%, at least about 90%, at least about 91%, at least about 92%, at least about 93%, at least about 94%, at least about 95%, at least about 96%, at least about 97%, at least about 98%, at least about 99%, or about 100% by weight, based on the total weight of the composition, of a specified feature or component.

As used herein, the term "substantially," in, for example, the context "substantially free" refers to a composition having less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the terms "substantially identical reference composition" or "substantially identical reference article" refer to a reference composition or article comprising substantially identical components in the absence of an inventive component. In another exemplary aspect, the term "substantially," in, for example, the context "substantially identical reference composition," refers to a reference composition comprising substantially identical components and wherein an inventive component is substituted with a common in the art component.

Further, the terms "coupled" and "associated" generally mean electrically, electromagnetically, and/or physically (*e.g*., mechanically or chemically) coupled or linked and do not exclude the presence of intermediate elements between the coupled or associated items.

As used herein, the term "atraumatic" is commonly known in the art and refers to a device or a procedure that minimized tissue injury.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, an appropriate effective amount will be readily determined by one of ordinary skill in the art using only routine experimentation.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### SHEATH

Disclosed herein one aspect of a sheath for delivering a medical device, wherein the sheath has a proximal and a distal end and comprises: a) an inner liner defining a lumen having a first rest diameter *dᵣ* and a second expanded diameter *dₑ*, wherein the lumen is configured to receive and pass through a medical device, wherein the inner liner comprises a sheet comprising a first portion having a first surface and an opposite second surface, wherein a first end of the first portion splits into a first segment having a first surface and an opposite second surface, and a third segment having a first surface and an opposite second surface, and wherein a second end of the first portion extends into a second segment having a first surface and an opposite second surface, wherein the sheet is rolled into a spiral configuration, such that at least a portion of the first surface of the second segment overlaps at least a portion of the second surface of the first segment, wherein at least a portion of the first surface of the third segment overlaps at least a portion the second surface of the second segment, and wherein at least a portion of the first surface of the third segment overlaps at least a portion of the second surface of the first segment, wherein the first surface of the first portion extends into the first surface of the first segment, the second segment and the third segment, and wherein the second surface of the first portion extends into the second surface of the second and the third segments; wherein each segment is configured to slidably move along each other upon passage of the medical device through the lumen; wherein the sheet comprises a polymer layer; and an outer layer.

In certain aspects, the rest diameter *dᵣ* can be substantially uniform along the longitudinal axis of the lumen. In yet other aspects, the rest diameter *dᵣ* varies along the longitudinal axis of the lumen and wherein the rest diameter *dᵣ* at the proximal end that is larger than the rest diameter *dᵣ* at the distal end. In one disclosed aspect, the expanded diameter *dₑ* is configured to accommodate the medical device passing through the lumen. In yet another aspect, the sheath can contract to the predetermined rest diameter *dᵣ* after passage of the medical device through the lumen.

In one disclosed aspect, the sheet of the sheath comprises a high-density polyethylene, polypropylene, polyamide, fluoropolymer, copolymers thereof, or blends thereof. In a still further aspect, the sheet can have a multilayer structure. In still further aspects, the inner surface of the sheet is at least partially ribbed.

In one exemplary aspect, the sheet is lubricious and has a coefficient of friction less than about 0.5.

In a still further aspect, an amount of a first lubricant is disposed between at least a portion of the inner liner and at least a portion of the outer layer. In yet other disclosed aspects, an amount of a second lubricant can be disposed between at least a portion of the overlying portion of the sheet and at least a portion of the sliding portions of the sheet.

In yet other disclosed aspects, the outer surface of the layer of the elastomeric polymer can define at least a portion of the outer surface of the outer layer. In some of the disclosed aspects, at least a portion of the inner surface of the layer of the elastomeric polymer is at least partially bonded to at least a portion of the outer surface of the sheet of the inner liner. In still further aspects, at least a portion of the inner surface of the layer of the elastomeric polymer can define at least a portion of the inner surface of the outer layer. While in other aspects, at least a portion of the braid or coil can define at least a portion of the inner surface of the outer layer.

In one disclosed aspect, the sheath can further comprise a first strip of the elastomeric polymer disposed along at least a portion of the longitudinal axis of the lumen between at least a portion of the outer surface of the sheet that does not comprise the overlaying portion of the sheet and the inner surface of the outer layer. In other aspects, the sheath can further comprise a second strip of the elastomeric polymer disposed between at least a portion of the outer surface of the sheet at the proximal end of the sheath and the inner surface of the outer layer. While in other aspects, the sheath can further comprise a third strip of the elastomeric polymer disposed between at least a portion of the outer surface of the sheet at the distal end of the sheath and the inner surface of the outer layer.

In still further aspects, the braid or coil is an expandable braid or coil. In still further aspects, the braid or coil can comprise at least one filament comprising stainless steel, nitinol, a polymer material, or a composite material. In certain aspects, the at least one filament can be a round filament or a flat filament. In aspects where the at least one filament comprises a polymer material, the polymer material can be polyester or nylon. In aspects where the at least one filament is round, the round filament can have a diameter of less than about 0.015". While in the aspects where the at least one filament is flat, the flat filament can have a height of less than about 0.006" and a width from greater than about 0.003" to about 0.015". In yet further aspects, the braid can have a per inch crosses (PIC) count of less than 50. In yet still further aspects, the braid's PIC can vary along the longitudinal axis of the lumen.

In still further aspects, where the at least one filament is nitinol, the nitinol is heat set at *dₑ*. In yet other aspects, where the at least one filament comprises stainless steel or nitinol, the filament can be configured to be atraumatic at least at the distal end of the sheath.

In yet other aspects, the elastomeric polymer present in the outer layer comprises a styrene-based elastomer, polyurethane, latex, copolymers thereof, blends thereof, or co-extrudates thereof. In one aspect, the elastomeric polymer exhibits a Shore A durometer of less than 90. In certain aspects, the braid or coil can be at least partially embedded within at least a portion of the layer of the elastomeric polymer. And yet, in other aspects, a hydrophilic coating layer can be disposed on the outer surface of the outer layer.

Also disclosed herein one aspect comprising methods of making a sheath having a proximal and a distal end. In certain aspect, the method of making such a sheath comprises forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid or coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

In some exemplary aspects, the step of forming the variable inner liner comprises rolling the sheet over a mandrel having a predetermined diameter to form the spiral configuration, wherein the predetermined diameter of the mandrel is substantially identical to the predetermined diameter *dᵣ* of the inner liner. In still further aspects, the rest diameter *dᵣ* is substantially uniform along the longitudinal axis of the lumen. While in other aspects, the rest diameter *dᵣ* varies along the longitudinal axis of the lumen and wherein the rest diameter *dᵣ* at the proximal end that is larger than the rest diameter *dᵣ* at the distal end. In still further aspects, the expanded diameter *dₑ* of the sheath is configured to accommodate the medical device passing through the lumen. While in other aspects, the sheath formed by the methods disclosed herein can contract to the predetermined rest diameter *dᵣ* after passage of the medical device through the lumen.

In still further aspects, the step of forming the outer layer comprises mounting the braid or coil on the inner liner. In still further aspects, the step of forming the outer layer further comprises mounting the elastomeric polymer on the braid or coil. In some exemplary aspects, the methods disclosed herein can further comprise at least partially embedding the braid or coil within at least a portion of the layer of the elastomeric polymer. In yet other exemplary aspects, the step of forming the outer layer can comprise mounting the layer of the elastic polymer on the braid or coil and then mounting the layer of the elastic polymer and the braid or coil on the inner liner positioned on the mandrel. In such exemplary aspects, the method can further comprise at least partially embedding the braid or coil within at least a portion of the layer of the elastomeric polymer before mounting on the inner liner. While in other aspects, the method can further comprise at least partially embedding the braid or coil within at least a portion of the layer of the elastomeric polymer after mounting on the inner liner. In still further aspects, the sheath is removed from the mandrel after the outer layer is mounted on the inner liner and the binding is complete.

In other aspects, in the methods disclosed herein, the outer surface of the layer of the elastomeric polymer can define at least a portion of the outer surface of the outer layer. Yet, in other aspects, in the methods disclosed herein, at least a portion of the inner surface of the layer of the elastomeric polymer can define at least a portion of the inner surface of the outer layer. Still further disclosed herein are the aspects wherein at least a portion of the braid or coil can define at least a portion of the inner surface of the outer layer.

In certain aspects, the methods disclosed herein further comprise bonding at least a portion of the inner surface of the layer of the elastomeric polymer to at least a portion of the outer surface of the sheet of the inner liner. In one aspect, the bonding is performed by heating at a temperature from about 350 °F to about 550 °F for a time period effective to form a bond between at least a portion of the outer layer and at least a portion of the inner liner.

One aspect, as described herein methods, comprises a first strip of the elastomeric polymer be applied along at least a portion of the longitudinal axis of the lumen to at least a portion of the outer surface of the sheet that does not comprise the overlaying portion prior to or during the step of bonding the at least a portion of the inner surface of the layer of the elastomeric polymer to at least a portion of the outer surface of the sheet of the inner liner. In yet other aspects, a second strip of the elastomeric polymer can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. In still further aspects, a third strip of the elastomeric polymer can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner.

In still further aspects and as described herein, an amount of a first lubricant can be applied to at least a portion of the inner liner prior to the step of forming the outer layer such that the amount of the first lubricant is disposed between at least a portion of the inner liner and at least a portion of the outer liner in the sheath. While in other aspects, an amount of a second lubricant is applied to at least a portion of the overlying and sliding portions of the sheet prior to the step of forming the outer layer.

In other aspects, in the methods described herein, the sheet can comprise a high-density polyethylene, polypropylene, polyamide, fluoropolymer, copolymers thereof, or blends thereof. In yet other aspects, the sheet can have a multilayer structure. In still further aspects, the inner surface of the sheet can be at least partially ribbed. In yet other aspects, the sheet can be lubricious and have a coefficient of friction less than about 0.5.

In yet further aspects, the methods disclosed herein comprise the braid or coil, wherein the braid or coil is an expandable braid or coil. In still further aspects, the braid or coil of the disclosed methods can comprise at least one filament comprising a stainless steel, nitinol, a polymer material, or a composite material. In certain aspects, the at least one of the filaments can be a round filament or a flat filament. In certain aspects, the polymeric material present in the braid can be a polyester or nylon. In the aspects where the at least one filament is the round filament, such a filament can have a diameter of less than about 0.015". In yet other aspects, where the at least one filament is the flat filament, such a filament can have a height of less than about 0.006" and a width greater than about 0.003" to about 0.015". In the aspects of the methods disclosed herein, the braid can have a per inch crosses (PIC) count of less than 50. In yet further exemplary aspects, the PIC can vary along the longitudinal axis of the lumen.

In the aspects where the at least one filament comprises nitinol, the nitinol is heat set at *dₑ*. In the aspects where the at least one filament comprises stainless steel or nitinol, the filament is configured to be atraumatic at least at the distal end of the sheath.

In yet further aspects, the methods disclosed herein comprise the elastomeric polymer comprising a styrene-based elastomer, polyurethane, latex, copolymers thereof, blends thereof, or co-extrudates thereof. In yet further aspects, the elastomeric polymer can exhibit a Shore A durometer of less than 90. In still further aspects, the methods can further comprise disposing a hydrophilic coating layer on the outer surface of the layer of the elastomeric polymer.

In some methods, a soft tip portion can be coupled to a distal end of the expandable sheath to facilitate passing the expandable sheath through a patient's vasculature.

Disclosed aspects of an expandable sheath can minimize trauma to the vessel by allowing for temporary expansion of a portion of the introducer sheath to accommodate the delivery system, followed by a return to the original diameter once the device passes through. Some aspects can comprise a sheath with a smaller profile (*e.g*., a smaller diameter in the rest configuration) than that of prior art introducer sheaths. Furthermore, present aspects can reduce the length of time a procedure takes, as well as reduce the risk of a longitudinal or radial vessel tear or plaque dislodgement because only one sheath is required, rather than several different sizes of sheaths. Aspects of the present expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices (*e.g*., stents, prosthetic heart valves, stented grafts, etc.) into many types of vascular and non-vascular body lumens (*e.g*., veins, arteries, esophagus, ducts of the biliary tree, intestine, urethra, fallopian tube, other endocrine or exocrine ducts, etc.).

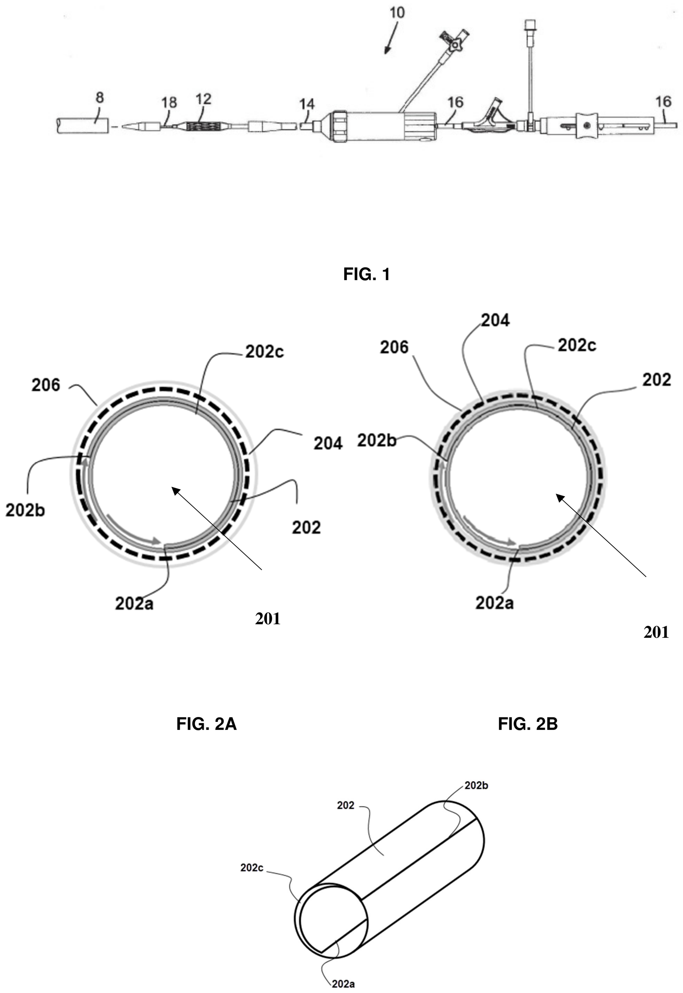

**FIG. 1** illustrates a sheath **8** according to the present disclosure, in use with a representative delivery apparatus **10,** for delivering a prosthetic device **12,** such as a tissue heart valve to a patient. The apparatus **10** can include a steerable guide catheter **14** (also referred to as a flex catheter), a balloon catheter **16** extending through the guide catheter **14,** and a nose catheter **18** extending through the balloon catheter **16.** The guide catheter **14,** the balloon catheter **16,** and the nose catheter **18** in the illustrated aspect are adapted to slide longitudinally relative to each other to facilitate delivery and positioning of the valve **12** at an implantation site in a patient's body, as described in detail below. Generally, sheath **8** is inserted into a vessel, such as the transfemoral vessel, passing through the skin of the patient, such that the distal end of the sheath **8** is inserted into the vessel. Sheath **8** can include a hemostasis valve at the opposite, proximal end of the sheath. The delivery apparatus **10** can be inserted into the sheath **8,** and the prosthetic device **12** can then be delivered and implanted within the patient.

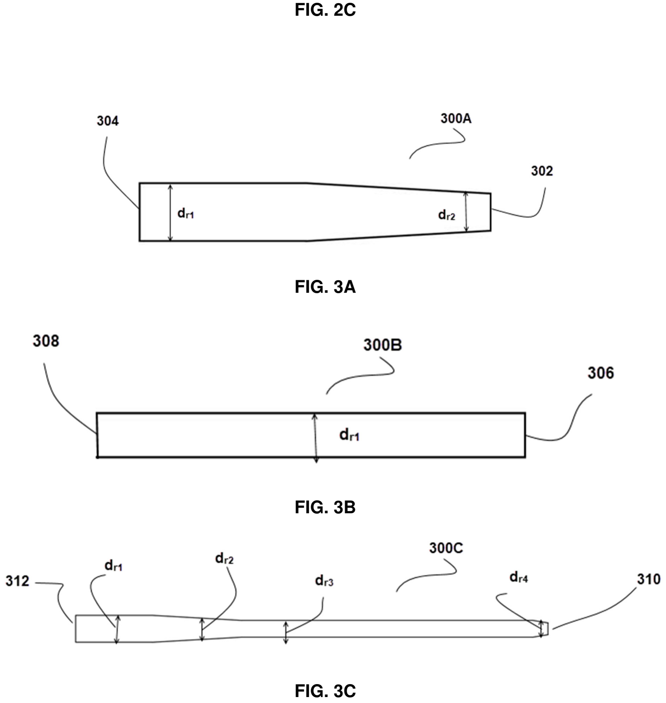

**FIGS. 2A** and **2B** show section views of aspects of two exemplary sheaths disclosed herein for use with a delivery apparatus such as that shown in **FIG. 1****.** **FIG. 2C** shows a perspective view of one aspect of an inner liner **202** for use with the disclosed sheath. As shown in **FIGS. 2A-2C****,** in some aspects, the disclosed sheath comprises an inner liner **202** rolled into a spiral configuration such that at least a portion of the inner surface of the sheet overlays at least a portion of the outer surface of the sheet, forming an overlaying portion **202c,** and wherein the first edge **202a** of the sheet is slidable along at least a portion the inner surface of the sheet and the second edge **202b** is slidable along at least a portion of the outer surface of the sheet. Sheath, as shown in **FIG. 2A** and **FIG. 2B** can further include an outer layer comprising a braid (or coil) **204** and a layer of elastomeric polymer **206.** In one aspect, and as shown in **FIG. 2A****,** the outer layer can comprise the braid (or coil) **204** that is not embedded in the layer of the elastomeric polymer **206.** While in the other aspect, and as shown in **FIG. 2B****,** the outer layer can comprise the braid (or coil) **204** that is embedded in the layer of the elastomeric polymer **206.**

The inner liner **202** defines a lumen **201** through which a delivery apparatus can travel into a patient's vessel in order to deliver, remove, repair, and/or replace a prosthetic device. The disclosed sheath can also be useful for other types of minimally invasive surgery, such as any surgery requiring introduction of an apparatus into a subject's vessel. For example, the disclosed sheath also can be used to introduce other types of delivery apparatus for placing various types of intraluminal devices (*e.g.,* stents, stented grafts, etc.) into many types of vascular and non-vascular body lumens (*e.g.,* veins, arteries, esophagus, ducts of the biliary tree, intestine, urethra, fallopian tube, other endocrine or exocrine ducts, etc.).

In still further aspects, the sheet used to make the inner liner **202** can comprise a high-density polyethylene, polypropylene, polyamide, fluoropolymer, copolymers thereof, or blends thereof. In still further aspects, the sheet can comprise one or more layers. In some aspects, if one or more layers are present, each layer can comprise the same or different polymer. In still further aspects, the sheet can have a predetermined thickness, wherein the predetermined thickness can be defined by one of ordinary skill in the art depending on the specific application. In certain aspects, the predetermined thickness of the inner liner can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner **202** can be varied depending on the desired amount of radial expansion, as well as the strength required.

In still further aspects, the inner surface of the sheet can be at least partially ribbed. In yet further aspects, the sheet can also be lubricious. In some exemplary aspects, the sheet that forms the inner liner can have a coefficient of friction less than about 0.5, less than about 0.4, less than about 0.3, less than about 0.2, less than about 0.1, or less than about 0.05, or even less than about 0.01. It is further understood that the sheet can have a coefficient of friction having any value between any two foregoing values. Such a liner can facilitate passage of a delivery apparatus through the lumen **201** of the disclosed sheath. In some further exemplary aspects, materials that can be used to form suitable lubricious inner liners include materials that can reduce the coefficient of friction of the inner liner **202,** such as PTFE, polyethylene, polyvinylidene fluoride, and combinations thereof. Suitable materials for a lubricious liner also include other materials desirably having a coefficient of friction of about 0.1 or less, of about 0.09 or less, about 0.08 or less, about 0.07 or less, about 0.05 or less, about 0.04 or less, about 0.03 or less, about 0.02 or less, or about 0.01 or less.

In yet further aspects, the outer layer comprising the braid or coil and the layer of the elastomeric polymer can have any predetermined thickness. It is understood that the predetermined thickness of the outer layer can be dependent on the specific application of the sheath. For example, and without limitation, the thicknesses of the inner liner **202** and the outer layer comprising the braid (or coil) **204** and the layer of the elastomeric material **206** can also be varied depending on the particular application of the disclosed sheath. In some aspects, the thickness of the inner liner **202** ranges from about 0.0005 inches to about 0.010 inches, including exemplary values of about 0.0006, about 0.0007, about 0.0008, about 0.0009, about 0.001, about 0.002, about 0.003, about 0.004, about 0.005, about 0.006, about 0.007, about 0.008, about 0.009 inches, and in one particular aspect, the thickness can be about 0.002 inches. The outer layer comprising the braid (or coil) **204** and the layer of the elastomeric material **206** can have a thickness of from about 0.002 inches to about 0.015 inches, including exemplary values of about 0.003, about 0.004, about 0.005, about 0.006, about 0.007, about 0.008, about 0.009, and about 0.01 inches.

It is understood that the inner liner can have any shape or configuration depending on the desired application and the size of the delivery apparatus and prosthetic device. It is further understood that the inner liner is not limited to a specific shape or configuration. In yet further aspects, the outer layer comprising the braid or coil and the layer of the elastomeric polymer can conform to the shape or configuration of the inner liner. In certain aspects, the sheath disclosed herein is defined by the rest diameter *dᵣ* and the outer diameter *dₒ*. As disclosed herein, the rest diameter *dᵣ* is defined by the inner liner, while the outer diameter can be defined by the inner liner and the outer layer, wherein the outer layer comprises the braid or coil and the layer of the elastomeric polymer.

The rest diameter *dᵣ* of the inner liner **202** can vary depending on the application and size of the delivery apparatus and prosthetic device. **FIGS. 3A-C** show various configurations and shapes of the inner liner. It is understood that in some aspects, and as shown in **FIG. 3B****,** the rest diameter *dᵣ* is substantially uniform along the longitudinal axis of the lumen without changing from the proximal end **308** to the distal end **306.** In yet other aspects, and as shown in **FIGS. 3A** and **3C****,** the rest diameter *dᵣ* can vary along the longitudinal axis (for example, *dᵣ₁* and *dᵣ₂* in **FIG. 3A****,** or *dᵣ₁, dᵣ₂, dᵣ₃,* and *dᵣ₄,* as shown in **FIG. 3C**) of the lumen. In certain aspects, rest diameter *dᵣ₁* at the proximal end **304** or **312** is larger than the rest diameter *dᵣ₂,* as shown in **FIG. 3A** or *dᵣ₄,* as shown in **FIG. 3C** at the distal end **302** or **310** *dᵣ.* In yet further aspects, where the outer layer conforms to the shape of the inner liner, the outer diameter *dₒ* (not shown) comprises the overall diameter of the inner liner and the outer layer. In such aspects, the outer diameter *dₒ* is defined by the specific application of the sheath. Similar to the rest diameter *dᵣ,* the outer diameter *dₒ* of the unexpended sheath disclosed herein can be substantially uniform (constant) along the longitudinal axis of the lumen without changing from the proximal end to the distal end (not shown). In alternative aspects, the original unexpanded outer diameter *dₒ* of the disclosed sheath, similarly to the rest diameter *dᵣ,* can decrease from the proximal end to the distal end. In some aspects, and similarly to the rest diameter *dᵣ,* the original unexpanded outer diameter can decrease along a gradient, from the proximal end to the distal end; or it can incrementally step down along the length of the sheath having the largest original unexpanded outer diameter is near *dₒ* the proximal end, and the smallest original unexpanded outer diameter *dₒ* is near the distal end.

In some aspects, the rest diameter *dᵣ* can range from about 0.005 inches to about 0.400 inches, including exemplary values of about 0.01 about 0.02, about 0.03, about 0.04, about 0.05, about 0.06, about 0.07, about 0.08, about 0.09, about 0.1, about 0.2, and about 0.3 inches. As described above, in certain aspects, the sheath can comprise the inner liner having various *dᵣ.* In such aspects, the *dᵣ* can have any value between any two foregoing values and can depend on the specific application and the size and shape of the delivery apparatus and prosthetic device. Different sheaths can be provided with different expanded, and unexpanded rest diameter *dᵣ* and outer diameter *dₒ*, depending on the size requirements of the delivery apparatus for various applications. Additionally, some aspects can provide more or less expansion depending on the particular design parameters, the materials, and/or configurations used.

As disclosed herein, the outer layer of the sheath has an inner surface and an outer surface. The outer layer of the disclosed sheath extends about at least a portion of the variable diameter inner liner such that the inner surface of the outer layer is positioned adjacent to the outer surface of the inner liner. As disclosed herein, the outer layer comprises a braid (or coil) **204** and a layer of an elastomeric polymer **206** having a predetermined thickness and having an inner surface and outer surface (as shown in **FIGS. 2A** and **2B**). In certain aspects, the braid or coil can be an expandable braid or coil. In yet further aspects, the braid or coil can comprise at least one filament comprising stainless steel, nitinol, a polymer material, or a composite material. In certain unlimiting aspects, the braid or coil comprises filaments comprising Nitinol and/or other shape memory alloys. In yet other unlimiting aspects, the braid can have filaments comprising polyester or nylon. In yet some other exemplary aspects, the braid can comprise filaments comprising spectra fiber, polyethylene fiber, aramid fiber, or combinations thereof.

It is understood that the braid or coil can have any configurations known in the art. In certain aspects, the braid (or coil) **204** is generally a thin, hollow, substantially cylindrical tube comprising an arrangement, pattern, structure, or configuration of filaments or struts, however other geometries can also be used. Suitable filaments can be round, having a diameter less than about 0.015", less than about 0.01", less than about 0.008", less than about 0.005", less than about 0.002", less than about 0.001", less than about 0.0008", or less than about 0.0005". In yet other aspects, suitable filaments can be round and having a diameter ranging from about 0.0005" inches thick to about 0.015" thick, including exemplary values of about 0.0006", about 0.0007", about 0.0008", about 0.0009", about 0.001", about 0.002", about 0.003", about 0.004", about 0.005", about 0.006", about 0.007", about 0.008", about 0.009", about 0.01", about 0.012", about 0.013", and about 0.014". In yet other aspects, the suitable filaments can be flat filaments having a height of less than about 0.006", less than about 0.005", less than about 0.004", less than about 0.003", less than about 0.001", less than about 0.0009", less than about 0.0008", less than about 0.0007", less than about 0.0006",and about 0.0005". In yet other aspects, the flat filaments can have a width from greater than about 0.003" to about 0.015", including exemplary values of about 0.004", about 0.005", about 0.006", about 0.007", about 0.008", about 0.009", about 0.01", about 0.012", about 0.013", and about 0.014". However, other geometries and sizes are also suitable for certain aspects.

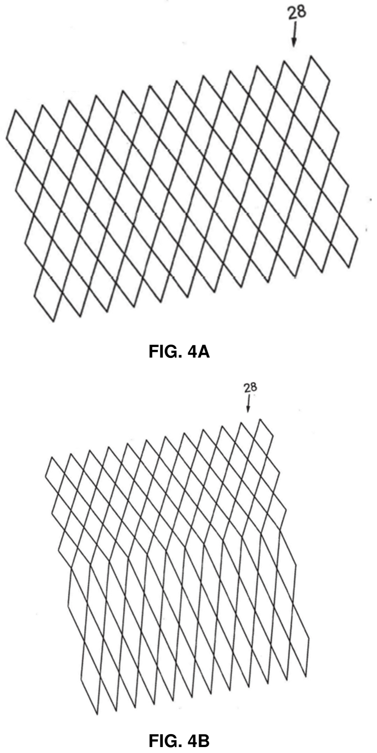

In yet further aspects, the braid can have a per inch crosses (PIC) count of less than 50, less than 40, less than 30, less than 20, or less than 10. In yet other aspects, the braid can have the PIC count from 10 to 2, including exemplary values of 9, 8, 7, 6, 5, 4, and 3. In still further aspects, the PIC can vary along the longitudinal axis of the lumen. In yet other aspects, the braid pattern can vary along the longitudinal axis of the lumen. In the aspects where the braid or coil comprises filament that is nitinol, the nitinol is a heat-set at the expanded diameter *dₑ* In yet further aspects, where the filament comprises stainless steel or nitinol, the filament is configured to be atraumatic at least at the distal end of the sheath. **FIGS. 4A-D** illustrate partial elevation views of various structures for the braid **28.** It is understood that the structure of the braid **28** can vary from section to section, changing along the length of the sheath. It is further understood that the structures shown in **FIGS. 4A-D**are not necessarily drawn to scale and show just exemplary and unlimiting aspects. It is further understood that the braid is configured to provide the torquability of the sheath during the insertion of the prosthetic device.

In still further aspects, the outer layer comprises the layer of the elastomeric polymer **206,** as shown in **FIGS. 2A,** and **2B****.** In certain aspects, the elastomeric polymer can comprise a styrene-based elastomer, polyurethane, latex, copolymers thereof, blends thereof, or co-extrudates thereof. In certain and unlimiting aspects, the elastomeric polymer can comprise polyether block ester copolymer, polyesters, polyvinyl chloride, thermoset silicone, poly-isoprene rubbers, polyolefin, other medical grade polymers, or combinations thereof. In yet further aspects, the elastomeric polymer described herein can have any useful additives. In certain aspects, the elastomeric polymers can comprise at least one friction reduction additive. In some exemplary aspects, the friction reduction additives can comprise, for example, BaSO₄, ProPell^{™}, PTFE, any combination thereof, and the like. It is understood that this list of the friction reduction additives is not limiting, and any known in the art friction reduction additives can be utilized.

It is understood that the hardness of each layer of the disclosed sheath can also be varied depending on the particular application and desired properties of the sheath. In some aspects, the layer of the elastomeric polymer **206** has a Shore hardness of less than 90 Durometer, less than 80 Durometer, less than 70 Durometer, less than 60 Durometer, less than 50 Durometer, less than 40 Durometer, less than 30 Durometer, or less than 20 Durometer. In yet further exemplary aspects, the layer of the elastomeric polymer **206** has a Shore hardness from about 25 Durometer to about 75 Durometer, including exemplary values of about 30 Durometer, about 35 Durometer, about 40 Durometer, about 45 Durometer, about 50 Durometer, about 55 Durometer, about 60 Durometer, about 65 Durometer, and about 70 Durometer.

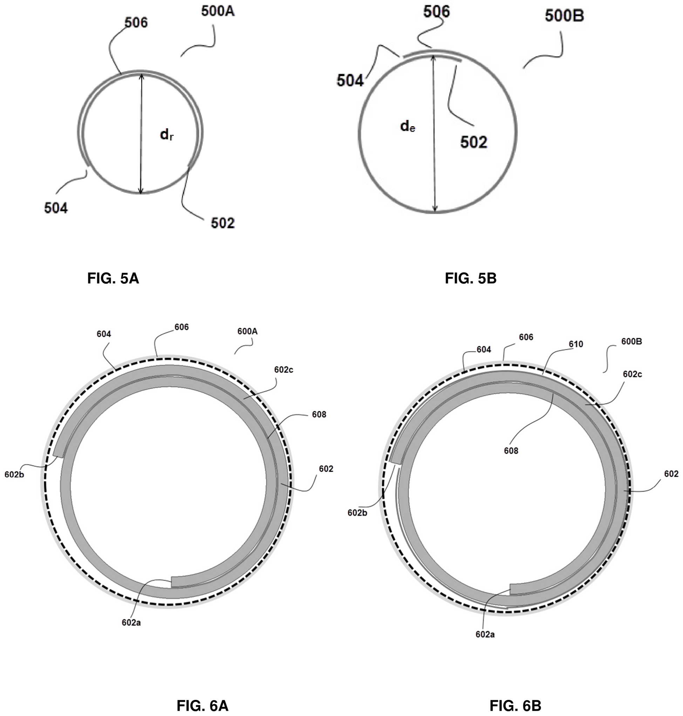

Alternative aspects of a sheath for introducing a prosthetic device are also described. For example, **FIGS. 5A-5B** illustrate a section view of the inner liners **500A** and **500B** of the disclosed sheath in unexpended and expended configurations (**FIGS. 5A** and **5B****,** respectively). Upon introduction of the prosthetic device into the inner liner, the first edge **502** and the second edge **504** slid along and expand the inner liner from the rest diameter *dᵣ* to the expanded diameter *dₑ*, thereby shortening the overlaying portion **506** of the inner liner. It is understood that the expanded diameter *dₑ* is configured to accommodate the medical device passing through the lumen. In yet further aspects, the sheath contracts to the predetermined rest diameter *dᵣ* after passage of the medical device through the lumen.

In certain aspects, an amount of a first lubricant is disposed between at least a portion of the inner liner and at least a portion of the outer layer. In yet other aspects, an amount of a second lubricant is disposed between at least a portion of the overlying portion of the sheet and at least a portion of the sliding portions of the sheet. It is understood that the first lubricant and the second lubricant can be the same or different. In certain and unlimiting aspects, the first and/or second lubricants can comprise Christo Lube supplied by ECL or MED10/6670 supplied by Nusil. In still further aspects, it is understood that the amount of the first and/or second lubricant can be easily determined by one of ordinary skill in the art.

In still further aspects, the outer surface of the layer of the elastomeric polymer defines at least a portion of the outer surface of the outer layer. In yet other aspects, at least a portion of the inner surface of the layer of the elastomeric polymer is at least partially bonded to at least a portion of the outer surface of the sheet of the inner liner. In one aspect, wherein at least a portion of the inner surface of the layer of the elastomeric polymer defines at least a portion of the inner surface of the outer layer. While in the other aspect, at least a portion of the braid defines at least a portion of the inner surface of the outer layer. It is understood that the outer layer of the disclosed sheath is configured to provide hemostasis and prevent bleeding of the patient during the procedure.

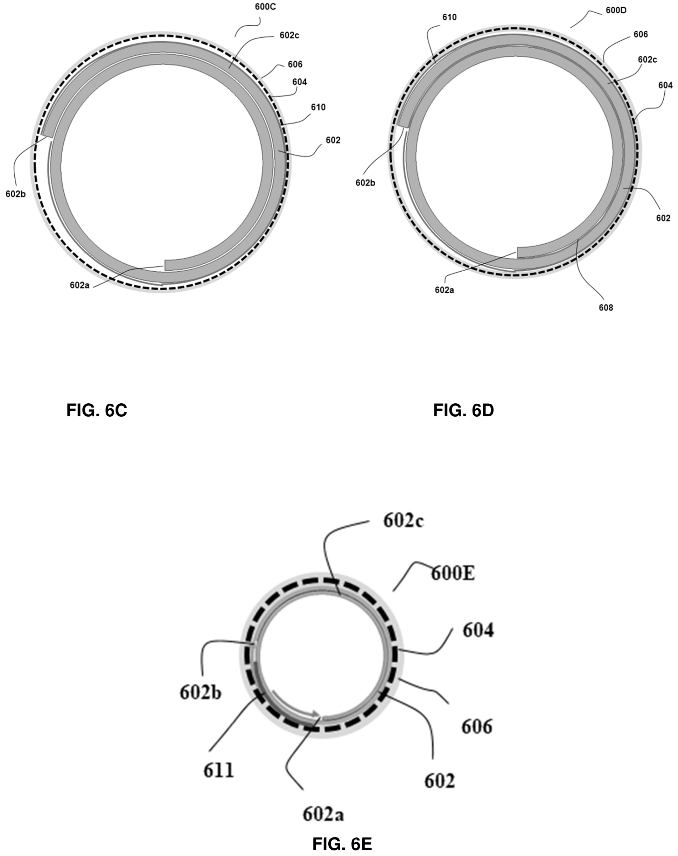

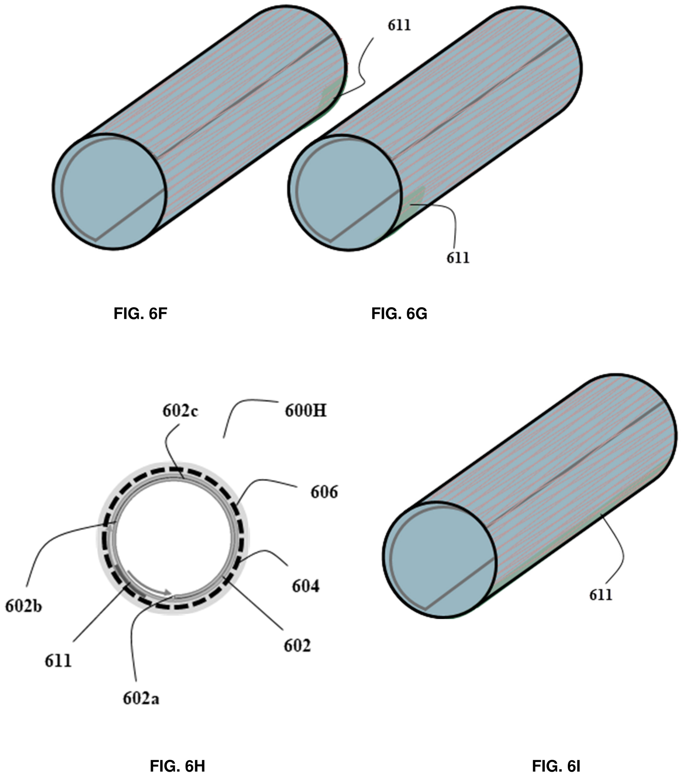

**FIGS. 6A-6D** show other alternative aspects of a sheath for introducing a prosthetic device. **FIG. 6A** shows the sheath **600A** comprising the inner liner **602** having the first edge **602a** and the second edge **602b,** and the overlaying portion **602c,** where the inner and outer surfaces of the inner liner overlay each other. The sheath **600A** further comprises an amount of the second lubricant **608** as disclosed herein that is disposed between the sliding and overlaying portions of the inner sheath. The sheath further comprises the braid **604** and the layer of the elastomeric polymer **606.** In this exemplary aspect, the braid **604** is not embedded in the layer of the elastomeric polymer **606.** **FIG. 6B** depicts an alternative aspect of the sheath **600B** where an amount of the first lubricant **610** is applied between the inner liner and the outer layer comprising the braid **604** and the layer of the elastomeric polymer **606.** An additional aspect of the sheath **600C** is shown in **FIG. 6C****.** In this aspect, the sheath **600C** comprises the inner liner **602,** having the first edge **602a** and the second edge **602b,** and the overlaying portion **602c,** where the inner and outer surfaces of the inner liner overlay each other. The sheath further comprises the braid **604** and the layer of the elastomeric polymer **606** that together form the outer layer of the sheath. The sheath **600C** further comprises an amount of the first lubricant **610,** as disclosed herein, that is disposed between the outer layer and the inner liner of the inner sheath. In this exemplary aspect, the braid **604** is not embedded in the layer of the elastomeric polymer **606.** In the exemplary aspect shown in **FIG. 6D****,** the exemplary sheath **600D** comprises the braid **604** embedded within the layer of the elastomeric polymer **606.**

In still further aspects, the sheath of the instant disclosure can comprise a hemostasis valve inside the lumen of the sheath, at or near the proximal end of the sheath (not shown). Additionally, the exemplary sheaths disclosed herein can comprise a soft tip at the distal end of the sheath (not shown). Such a soft tip can be provided with a lower hardness than the other portions of the sheath. In some aspects, the soft tip can have a Shore hardness from about 25 D to about 40 D, including exemplary values of about 26 D, about 27 D, about 28 D, about 29 D, about 30 D, about 31 D, about 32 D, about 33 D, about 34 D, about 35 D, about 36 D, about 37 D, about 38 D, and about 39 D. In yet other aspects, the soft tip can have a Shore hardness from about 25 A to about 40 A, including exemplary values of about 26 A, about 27 A, about 28 A, about 29 A, about 30 A, about 31 A, about 32 A, about 33 A, about 34 A, about 35 A, about 36 A, about 37 A, about 38 A, and about 39 A.

In certain aspects, the outer layer and the inner liner can be bonded together or otherwise physically associated with one another. It is understood that the amount of adhesion between the inner liner **602** and the outer polymer layer that comprises braid **604** and the layer of the elastomeric polymer **606** can be variable over the surfaces of the layers. The bonding between the layers can be created by, for example, thermal bonding. In certain aspects, the bonding can be facilitated by the presence of an additional portion of the elastomeric polymer. For example, in certain aspects, the sheath as described herein and as shown in **FIGS. 6H-6I** can further comprise a first strip **611** of the elastomeric polymer disposed along at least a portion of the longitudinal axis of the lumen between at least a portion of the outer surface of the sheet that does not comprise the overlaying portion **602c** of the sheet and the inner surface of the outer layer. In such aspects, the bonding between the outer layer and the inner liner can be facilitated by the first strip of the elastomeric polymer. In yet other aspects, the sheath can further comprise a second strip **611** (**FIGS. 6E-6F**) of the elastomeric polymer disposed between at least a portion of the outer surface of the sheet at the proximal end of the sheath and the inner surface of the outer layer. In still further aspects, the sheath can further comprise a third strip **611** of the elastomeric polymer disposed between at least a portion of the outer surface of the sheet at the distal end of the sheath and the inner surface of the outer layer (**FIGS. 6E** and **6G**). Again, in such aspects, the bonding between the outer layer and the inner liner can be facilitated by the second and/or third strips of the elastomeric polymer.

Applications can utilize a sheath of the present disclosure with the rest diameter *dᵣ* of the lumen formed by the inner liner **602** that is expandable to an expanded diameter *dₑ* of from about 3 Fr to about 26 Fr, including exemplary values of about 5 Fr, about 8 Fr, about 10 Fr, about 12 Fr, about 15 Fr, about 18 Fr, about 20 Fr, about 22 Fr, about 25 Fr. The expanded diameter can vary slightly along the length of the disclosed sheath. For example, the expanded outer diameter at the proximal end of the sheath can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, about 25 Fr, while the expanded outer diameter at the distal end of the sheath can range from about 3 Fr to about 25 Fr, including exemplary values of about 8 Fr, about 10 Fr, about 12 Fr, about 15 Fr, about 18 Fr, about 20 Fr, and about 22 Fr. Aspects of the disclosed sheath can expand to an expanded outer diameter that is from about 10% greater than the original unexpanded outer diameter to about 100% greater than the original unexpanded outer diameter, including exemplary values of about 15 % greater, about 20 % greater, about 25 % greater, about 30 % greater, about 35 % greater, about 40 % greater, about 45 % greater, about 50 % greater, about 55 % greater, about 60 % greater, about 65 % greater, about 70 % greater, about 75 % greater, about 80 % greater, about 85 % greater, about 90 % greater, and about 95 % greater than the original unexpanded outer diameter.

It is understood, and as described above, the disclosed sheath can expand from its rest position. The expansion of the disclosed sheath can result in an expansion of the rest diameter *dᵣ* of from about 10% or less to about 430% or more. In certain aspects, expansion of the sheath can result in expansion of the rest diameter *dᵣ* to about 10 % or less, to about 9 % or less, to about 8 % or less, to about 7 % or less, to about 6 % or less, to about 5 % or less, to about 4 % or less, to about 3 % or less, to about 2 % or less, to about 1 % or less. In yet other aspects, expansion of the disclosed sheath can result in expansion of the rest diameter *dᵣ* to about 10 % or more, about 20 % or more, about 30 % or more, about 40 % or more, about 50 % or more, about 60 % or more, about 70 % or more, about 80 % or more, about 90 % or more, about 100 % or more, about 125 % or more, about 150 % or more, about 175 % or more, about 200 % or more, about 225 % or more, or about 250 % or more.

As with previously disclosed aspects, the aspects illustrated in **FIGS. 6A-6D** can be applied to sheaths having a wide variety of rest diameters *dᵣ* and outer diameter *dₒ*. In some aspects, the outer diameter *dₒ* of the sheath gradually decreases from the proximal end of the sheath to the distal end of the sheath. For example, in one aspect, the outer diameter *dₒ* can gradually decrease from about 26 Fr at the proximal end to about 18 Fr at the distal end. The diameter *dₒ* of the sheath can transition gradually across substantially the entire length of the sheath. In other aspects, the transition or reduction of the diameter of the sheath can occur only along a portion of the length of the sheath. For example, the transition can occur along a length from the proximal end to the distal end, where the length can range from about 0.5 inches to about the entire length of the sheath, including any values between any two foregoing values. In yet further aspects, the *dₒ* is minimal and constant along the section of the sheath that passes through the vasculature. In such aspects, the tapered section is about 4" or less at the proximal side of the sheath.

In some aspects, the outer layer comprising the braid and the layer of the elastomeric polymer can comprise the same material or combination of materials along the entire length. In alternative aspects, the material composition of the outer layer can change along the length of the sheath. For example, the outer layer can be provided with one or more segments, where the composition changes from segment to segment. For example, in one segment, the braid can comprise nitinol having a different PIC count than another segment. In yet another exemplary aspect, the layer of the elastomeric material in one segment can be different from the layer of the elastomeric material in another segment. In still further exemplary aspects, one segment of the sheath can comprise the braid or coil embedded within the layer of the elastomeric polymer material, while another segment can comprise the braid or coil that is not embedded within the layer of the elastomeric polymer material. It is understood that the exemplary sheath disclosed herein is not limiting. In certain exemplary aspects, the sheath can comprise an *n* number of segments, wherein each segment can be the same or different. In still further exemplary aspects, the Durometer rating of the composition of the outer layer can also change along the length of the sheath such that segments near the proximal end comprise a stiffer material or combination of materials, while segments near the distal end comprise a softer material or combination of materials. This can allow for a sheath having a relatively stiff proximal end at the point of introducing a delivery apparatus while still having a relatively soft distal tip at the point of entry into the patient's vessel.

**FIGS. 10** and **11** illustrate an expandable sheath **100** according to the present disclosure, which can be used with a delivery apparatus for delivering a prosthetic device, such as a tissue heart valve, into a patient. In general, the delivery apparatus can include a steerable guide catheter (also referred to as a flex catheter) a balloon catheter extending through the guide catheter, and a nose catheter extending through the balloon catheter (*e.g*., as depicted in **FIG. 1**). The guide catheter, the balloon catheter, and the nose catheter can be adapted to slide longitudinally relative to each other to facilitate delivery and positioning of the valve at an implantation site in a patient's body. However, it should be noted that the sheath **100** can be used with any type of elongated delivery apparatus used for implanting balloon-expandable prosthetic valves, self-expanding prosthetic valves, and other prosthetic devices. Generally, sheath **100** can be inserted into a vessel (*e.g.,* the femoral or iliac arteries) by passing through the skin of a patient, such that a soft tip portion **102** at the distal end **104** of the sheath **100** is inserted into the vessel. The sheath **100** can also include a proximal flared end portion **114** to facilitate mating with an introducer housing **101** and catheters mentioned above (*e.g*., the proximal flared end portion **114** can provide a compression fit over the housing tip and/or the proximal flared end portion **114** can be secured to the housing **101** via a nut or other fastening device or by bonding the proximal end of the sheath to the housing). The introducer housing **101** can house one or more valves that form a seal around the outer surface of the delivery apparatus once inserted through the housing, as known in the art. The delivery apparatus can be inserted into and through the sheath **100,** allowing the prosthetic device to be advanced through the patient's vasculature and implanted within the patient.

In exemplary aspects, the sheath **100** comprises an inner liner **108** and an outer layer **110** disposed around the inner liner **108.** The outer layer **110** comprises the braid (or coil) **111** and the layer of the elastomeric polymer **113.** **FIG. 11** depicts one and unlimiting aspect where the braid (or coil) **111** is embedded in the layer of the elastomeric polymer **113.** The inner liner **108** defines a lumen having the rest diameter *dᵣ* through which a delivery apparatus can travel into a patient's vessel in order to deliver, remove, repair, and/or replace a prosthetic device, moving in a direction along the longitudinal axis X. As the prosthetic device passes through the sheath **100,** the sheath locally expands from the resting diameter *dᵣ* to the expanded diameter *dₑ* to accommodate the prosthetic device. After the prosthetic device passes through a particular location of the sheath **100,** each successive expanded portion or segment of the sheath **100** at least partially returns to the resting diameter *dᵣ*. In this manner, the sheath **100** can be considered self-expanding in that it does not require the use of a balloon, dilator, and/or obturator to expand.

The inner and outer layers **108, 110,** as shown herein, can comprise any materials disclosed above.

Additionally, some aspects of a sheath **100** can include an exterior hydrophilic coating on the outer surface of the outer layer **110.** Such a hydrophilic coating can facilitate insertion of the sheath **100** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g*., PTFE, polyethylene, polyvinylidene fluoride), are also suitable for use with the sheath **100.**

Best seen in **FIG. 11****,** the soft tip portion **102** can comprise, in some aspects, low density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 A to about 40 A, including exemplary values of about 28 A, about 30 A, about 32 A, about 35 A, and about 38 A. It is further understood that Shore hardness can have any value between any two foregoing values. In yet other aspects, the soft tip **102** can have a Shore hardness from about 25 D to about 40 D, including exemplary values of about 28 D, about 30 D, about 32 D, about 35 D, and about 38 D. The tip portion **102** is configured to be radially expandable to allow a prosthetic device to pass through the distal opening of the sheath **100.**

**As** shown in **FIG. 11****,** the sheath **100** can optionally include at least one radiopaque filler or marker, such as a discontinuous or C-shaped, band **112** positioned near the distal end **104** of the sheath **100.** The marker **112** can be associated with the inner liner and/or outer layer **108, 110** of the sheath **100.** Such a radiopaque tip marker can comprise materials such as those suitable for the radiopaque filler, platinum, iridium, platinum/iridium alloys, stainless steel, other biocompatible metals, or combinations thereof. Suitable materials for use as a radiopaque filler or marker include, for example, barium sulfite, bismuth trioxide, titanium dioxide, bismuth subcarbonate, or combinations thereof. The radiopaque filler can be mixed with or embedded in the layer of the elastomeric polymer used to form the outer layer and can comprise from about 5% to about 45% by weight of the outer layer, including exemplary values of about 10%, about 15%, about 20%, about 25%, about 30%, about 35%, and about 40% by weight of the outer polymeric tubular layer. The more or less radiopaque material can be used in some aspects, depending on the particular application.

**FIGS. 12A-12B** shows a cross-section view of the sheath **100** taken near the distal end **104** of the sheath **100.** **FIG. 12A** shows the sheath **1200A** comprising the inner liner **1202** having the first edge **1202a** and the second edge **1202b,** and the overlaying portion **1202c,** where the inner and outer surfaces of the inner liner overlay each other. The sheath **1200A** further comprises an amount of the second lubricant **1208** as disclosed herein that is disposed between the sliding and overlaying portions of the inner sheath. The sheath further comprises the braid (or coil) **1204** and the layer of the elastomeric polymer **1206.** In this exemplary aspect, the braid (or coil) **1204** is not embedded in the layer of the elastomeric polymer **1206.** **FIG. 12B** depicts an alternative aspect of the sheath **1200B** where an amount of the first lubricant **1210** is applied between the inner liner and the outer layer comprising the braid (or coil) **1204** and the layer of the elastomeric polymer **1206.** An additional aspect of the sheath **1200C** is shown in **FIG. 12C****.** In this aspect, the sheath **1200C** comprises the inner liner **1202,** having the first edge **1202a** and the second edge **1202b,** and the overlaying portion **1202c,** where the inner and outer surfaces of the inner liner overlay each other. The sheath further comprises the braid (or coil) **1204** and the layer of the elastomeric polymer **1206** that together form the outer layer of the sheath. The sheath **1200C** further comprises an amount of the first lubricant **1210,** as disclosed herein, that is disposed between the outer layer and the inner liner of the inner sheath. In this exemplary aspect, the braid (or coil) **1204** is not embedded in the layer of the elastomeric polymer **1206.** In the exemplary aspect shown in **FIG. 12D****,** the exemplary sheath **1200D** comprises the braid (or coil) **1204** embedded within the layer of the elastomeric polymer **1206.**

**FIGS. 13A-D** show a section view of a proximal section of the sheath of **FIGURE 10****,** taken along line 38-38. **FIG. 13A** shows the sheath **1300A** comprising the inner liner **1302** having the first edge **1302a** and the second edge **1302b,** and the overlaying portion **1302c,** where the inner and outer surfaces of the inner liner overlay each other. The sheath **1300A** further comprises an amount of the second lubricant **1308** as disclosed herein that is disposed between the sliding and overlaying portions of the inner sheath. The sheath further comprises the braid (or coil) **1304** and the layer of the elastomeric polymer **1306.** In this exemplary aspect, the braid (or coil) **1304** is not embedded in the layer of the elastomeric polymer **1306.** **FIG. 13B** depicts an alternative aspect of the sheath **1300B** where an amount of the first lubricant **1310** is applied between the inner liner and the outer layer comprising the braid (or coil) **1304** and the layer of the elastomeric polymer **1306.** An additional aspect of the sheath **1300C** is shown in **FIG. 13C****.** In this aspect, the sheath **1300C** comprises the inner liner **1302,** having the first edge **1302a** and the second edge **1302b,** and the overlaying portion **1302c,** where the inner and outer surfaces of the inner liner overlay each other. The sheath further comprises the braid (or coil) **1304** and the layer of the elastomeric polymer **1306** that together form the outer layer of the sheath. The sheath **1300C** further comprises an amount of the first lubricant **1310,** as disclosed herein, that is disposed between the outer layer and the inner liner of the inner sheath. In this exemplary aspect, the braid (or coil) **1304** is not embedded in the layer of the elastomeric polymer **1306.** In the exemplary aspect shown in **FIG. 13D****,** the exemplary sheath **1300D** comprises the braid (or coil) **1304** embedded within the layer of the elastomeric polymer **1306.**

In yet further aspects, as shown in **FIG. 14****,** the sheath **1400,** whether with the braid or coil embedded within the layer of the elastomeric polymer (as shown in **FIG. 14****)** or with the braid or coil that is not embedded within the layer of the elastomeric polymer (not shown) is configured to expand from a resting configuration to an expanded configuration shown in **FIG. 15****.** In such aspects, the first and the second edges **(1502a** and **1502b)** of the inner liner slide such that a length of the overlaying portion shortens. In some exemplary aspects, this movement can be facilitated by the presence of the first and/or second lubricant, as disclosed above.

The disclosed herein sheath can be configured such that it locally expands at a particular location corresponding to the location of the medical device along the length of the lumen and then locally contracts once the medical device has passed that particular location. Thus, a bulge may be visible, traveling longitudinally along the length of the sheath as a medical device is introduced through the sheath, representing continuous local expansion and contraction as the device travels the length of the sheath. In some aspects, each segment of the sheath can locally contract after removal of any radial outward (insertion) force such that it regains the original resting diameter of lumen *dᵣ*.

In some aspects, each segment of the sheath can locally contract after removal of any radial outward force such that it at least partially returns to the original resting diameter of lumen *dᵣ*.

Additional sheath **8** configurations that can be utilized with the delivery apparatus **10,** as shown in **FIG. 1** for delivery of a prosthetic device **12** are further disclosed.

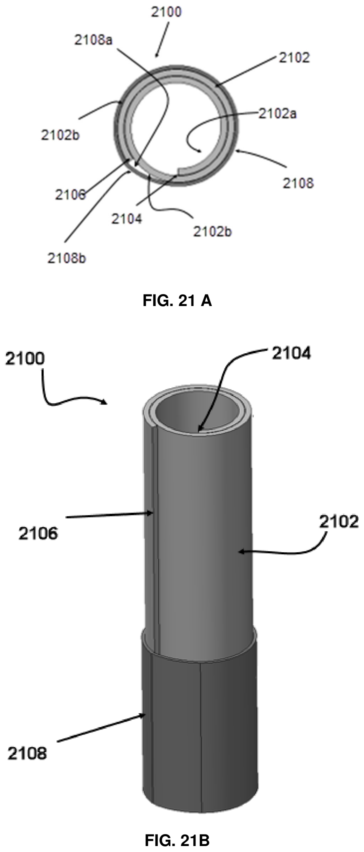

For example, and without limitations, **FIGS. 21A-B** how one exemplary aspect of the disclosed herein sheath. In such aspects, the sheath comprises a proximal end and a distal end. The sheath **2100** can comprise a variable diameter inner liner **2102** comprising a sheet having a first edge **2104** and a second edge **2106** and is defined by an inner surface **2102a** and an outer surface **2102b.** When the sheet is wound in a spiral configuration least a portion of the inner surface **2102a** of the sheet overlays at least a portion of the outer surface **2102b** of the sheet. As can be seen in **FIGS. 21A-B** the first edge **2104** of the sheet is slidable along at least a portion of the inner surface **2102a** of the sheet, and the second **2106** edge is slidable along at least a portion of the outer surface **2102b** of the sheet. The sheath further comprises an outer layer **2108** having an inner surface **2108a** and an outer surface **2108b.**

The inner surface **2102a** of the sheet further defines a lumen of the sheath through which a delivery apparatus can travel into a patient's vessel in order to deliver, remove, repair, and/or replace a prosthetic device. The disclosed sheath can also be useful for other types of minimally invasive surgery, such as any surgery requiring introduction of an apparatus into a subject's vessel. For example, the disclosed sheath also can be used to introduce other types of delivery apparatus for placing various types of intraluminal devices (e.g., stents, stented grafts, etc.) into many types of vascular and non-vascular body lumens (e.g., veins, arteries, esophagus, ducts of the biliary tree, intestine, urethra, fallopian tube, other endocrine or exocrine ducts, etc.).

It is further understood that the sheath can also comprise additional layers. Some of these additional layers are disclosed in detail below or above. For example, as disclosed in some exemplary aspects above, the sheath can also comprise a braid or coil disposed between the inner liner and the outer layer and/or a braid or coil that is embedded in the outer layer.

In exemplary aspects shown in **FIGS. 21A-B** the sheath does not comprise a braid or coil disposed along a length of the sheath between the inner liner and outer layer or being embedded in the outer layer.

Similar to other aspects of the sheath, an exemplary sheath of **FIGS. 21A-B** can have an inner liner having various depending on the desired application and the size of the delivery apparatus and prosthetic device. It is further understood that the inner liner is not limited to a specific shape or configuration. In certain aspects, the sheath disclosed herein is defined by the rest diameter *dᵣ* and the outer diameter *dₒ*. As disclosed herein, the rest diameter *dᵣ* is defined by the inner liner, while the outer diameter can be defined by the inner liner and the outer layer.

The rest diameter *dᵣ* of the inner liner **2102** can vary depending on the application and size of the delivery apparatus and prosthetic device. The sheath disclosed herein can have configurations similar to the configurations depicted in **FIG. 3A-C** and described above. It is understood that in some aspects, and as shown in **FIG. 3B****,** the rest diameter *dᵣ₁* is substantially uniform along the longitudinal axis of the lumen without changing from the proximal end **308** to the distal end **306.** In yet other aspects, and as shown in **FIGS. 3A** and **3C****,** the rest diameter *dᵣ* can vary along the longitudinal axis (for example, *dᵣ₁* and *dᵣ₂* in **FIG. 3A****,** or *dᵣ₁, dᵣ₂, dᵣ₃,* and *dᵣ₄,* as shown in **FIG. 3C****)** of the lumen. In certain aspects, rest diameter *dᵣ₁* at the proximal end **304** or **312** is larger than the rest diameter *d_{r2,}* as shown in **FIG. 3A** or *d_{r4,}* as shown in **FIG. 3C** at the distal end **302** or **310** *dᵣ*. In yet further aspects, where the outer layer conforms to the shape of the inner liner, the outer diameter *dₒ* (not shown) comprises the overall diameter of the inner liner and the outer layer. In such aspects, the outer diameter *dₒ* is defined by the specific application of the sheath. Similar to the rest diameter *dᵣ,* the outer diameter *dₒ* of the unexpended sheath disclosed herein can be substantially uniform (constant) along the longitudinal axis of the lumen without changing from the proximal end to the distal end (not shown). In alternative aspects, the original unexpanded outer diameter *dₒ* of the disclosed sheath, similarly to the rest diameter *dᵣ,* can decrease from the proximal end to the distal end. In some aspects, and similarly to the rest diameter *dᵣ,* the original unexpanded outer diameter can decrease along a gradient, from the proximal end to the distal end; or it can incrementally step down along the length of the sheath having the largest original unexpanded outer diameter is near *dₒ* the proximal end, and the smallest original unexpanded outer diameter *dₒ* is near the distal end.

In some aspects, and similarly to other sheath configurations disclosed herein, the rest diameter *dᵣ* of the sheath as shown in **FIGS. 21A-21B** can also range from about 0.005 inches to about 0.400 inches, including exemplary values of about 0.01 about 0.02, about 0.03, about 0.04, about 0.05, about 0.06, about 0.07, about 0.08, about 0.09, about 0.1, about 0.2, and about 0.3 inches. As described above, in certain aspects, the sheath can comprise the inner liner having various *dᵣ*. In such aspects, the *dᵣ* can have any value between any two foregoing values and can depend on the specific application and the size and shape of the delivery apparatus and prosthetic device. Different sheaths can be provided with different expanded, and unexpanded rest diameter *dᵣ* and outer diameter *dₒ*, depending on the size requirements of the delivery apparatus for various applications. Additionally, some aspects can provide more or less expansion depending on the particular design parameters, the materials, and/or configurations used.

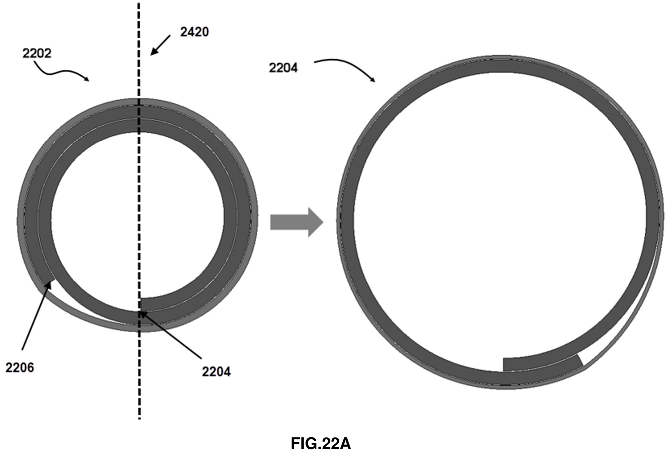

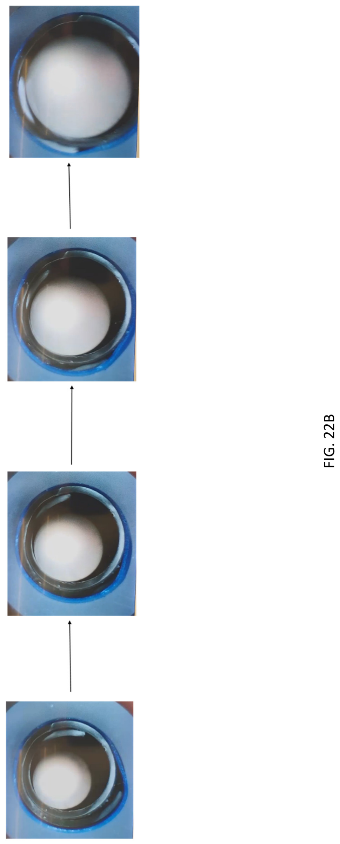

**FIGS. 22A-22B** depict expansion process of an exemplary sheath, as shown, for example, in **FIGS. 21-21****B.** The sheath **2202,** as shown in **FIG. 22A** can expand from the collapsed configuration to expanded configuration **2204** during the passage of the medical device by sliding the first and the second longitudinal edges along each other and decreasing the overlapping portion of the spiral configuration. Again, the expanded diameter *dₑ* can be dependent on a diameter of a medical device passing through. In still further aspects and as discussed in detail below, the outer layer is configured to impart an inward radial force on the inner liner to contract the sheath to a diameter that is substantially identical to *dᵣ* after the medical device passed through the lumen. It is further understood that any of the sheath configurations described herein can locally expand and collapse upon passage of the medical device. **FIG. 22B** shows a snapshot of various moments of the sheath expansion during the passage of the exemplary medical device.

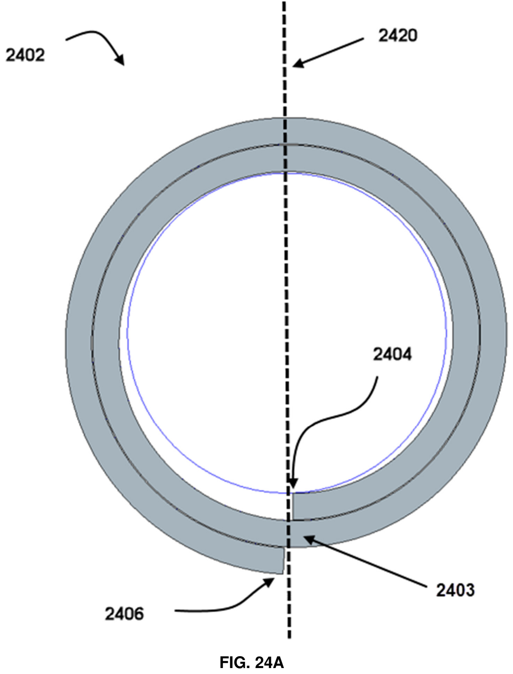

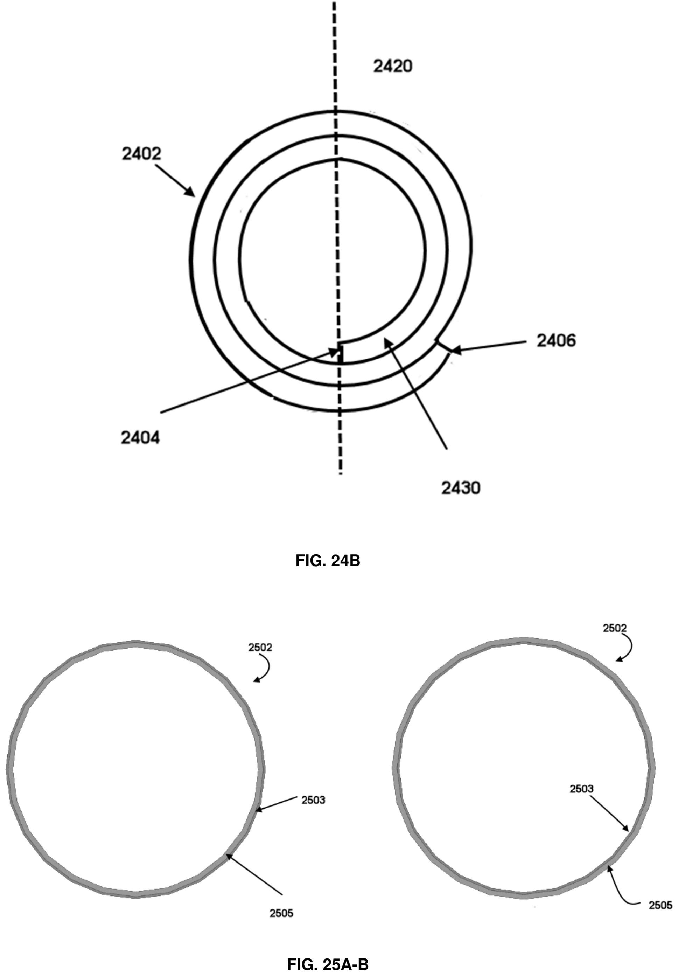

Additional aspects of the sheath are disclosed in **FIGS. 24A-24B****.** For example, **FIG. 24** **A** shows the inner liner of the sheath having a configuration similar to the sheath disclosed in **FIG.22****,** where the first end **2404** and the second **2406** are substantially aligned in a spaced relationship along a vertical axis **2420** passing through a thickness of the sheath. In such a configuration, a portion of the sheet **2403** is positioned between the first edge and the second edge along the vertical axis. As it can be seen in **FIG. 24A****,** when the inner liner **2402** is in an unexpanded rest state, the inner liner comprises at least two layers of the sheet overlaying each other at at least a portion of a circumference of the sheath. Yet, and as seen in **FIG.24****,** the inner liner can comprise at least two layers of the sheet overlaying each other along a whole circumference of the sheath.

**FIG. 24B** shows a different configuration of the liner in an expanded state. For example, when liner **2402** is in an unexpanded rest state, the inner liner can comprise a portion along a circumference of the sheath that can have three layers of the sheet **2430.**

Referring to **FIG. 22A****,** for example, there are also configurations of the inner liner, where the first edge **2204** is substantially aligned with a vertical axis **2420** passing through a thickness of the sheath and the second edge **2406** circumferentially offset from the vertical axis. In such an aspect, at at least a portion of a circumference of the sheath, the inner liner comprises one layer of the sheet without any overlaying portion.

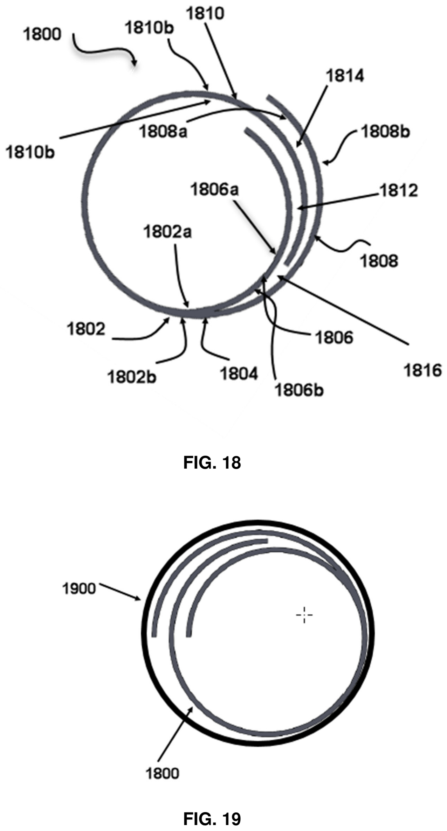

An additional configuration of the inner liner is also depicted in **FIG. 18. FIG. 18** shows an inner liner **1800** comprising a sheet rolled into a spiral configuration wherein the sheet comprises a first portion **1802** having a first surface **1802a** and an opposite second surface **1802,** wherein a first end **1804** of the first portion **1802** splits into a first segment **1806** having a first surface **1806a** and an opposite second surface **1806b** and a third segment **1808** having a first surface **1808a** and an opposite second surface **1808b,** and wherein a second end of the first portion extends into a second segment **1810** having a first surface **1810a** and an opposite second surface **1810b.**

As it can be seen in **FIG. 18****,** in a spiral configuration, at least a portion of the first surface **1810a** of the second segment **1810** overlaps at least a portion of the second surface **1806b** of the first segment **1806,** wherein at least a portion of the first surface **1808a** of the third segment **1808** overlaps at least a portion of the second surface **1810b** of the second segment **1810,** and wherein at least a portion of the first surface **1808a** of the third segment overlaps at least a portion of the second surface **1806b** of the first segment **1806.** It can also be seen that that wherein the first surface **1804a** of the first portion **1804** extends into the first surface **1806a** of the first segment **1806,** the first surface **1810a** of the second segment **1810** and the first surface **1808a** of the third segment **1808.** It can also be seen that the second surface **1804b** of the first portion **1804** extends into the second surface **1810b** of the second segment **1810** and into the second surface **1808b** of the third segment **1808.** Each of these segments can slide along each other during the passage of the medical device and expand the sheath.

**FIG. 19** shows an exemplary sheath having an inner liner **1800,** as depicted in **FIG.18** and an outer layer **1900.**

Referring back to **FIG. 18****,** when the at least a portion of the first surface **1810a** of the second segment **1810** overlaps the at least a portion of the second surface **1806b** of the first segment **1806** a first gap **1812** is formed between the at least a portion of the first surface **1810a** of the second segment **1810** and the at least a portion of the second surface **1804b** of the first segment **1806.**

Further, a second gap **1814** can be formed when the at least a portion of the first surface **1808a** of the third segment **1808** overlaps the at least a portion of the second surface **1810b** of the second segment **1810.** Still, further, a third gap **1816** can be formed when the at least a portion of the first surface **1808a** of the third segment **1808** overlaps the at least a portion of the second surface **1806b** of the first segment **1806.**

It is understood that the first gap can have a substantially uniform width along overlapping portions, or its width can vary. Similarly, the second gap has a substantially uniform width along overlapping portions, or the width of the second gap can vary along overlapping portions. Still, further, the third gap can have a substantially uniform or a variable width along overlapping portions. In still further aspects, the width between the gaps can be any width as desired. It is understood, for example, that width of each of the three gaps can be the same, or it can be different. In certain aspects, the width of some of the gaps is the same, while the width of the others is different.

When the sheath as disclosed in **FIGS. 18** and **19** is utilized, upon passage of the medical device through the lumen, the first segment **1806,** the second segment **1810,** and the third segment **1808** are configured to slidably move along each other such that an overlapping portion between the first segment and the second segment and between the second segment and the third segment decreases, while an overlapping portion between the first segment and the third segment increases.

Similar to any of the disclosed herein sheath configurations, a diameter of the lumen of the sheath shown in **FIG.19** increases from the first rest diameter *dᵣ* to the second expanded diameter *dₑ*. After passage of the medical device through the lumen, the first segment **1806,** the second segment **1808,** and the third segment **1810** are configured to slidably move back along each other such that the overlapping portion between the first segment and the second segment, between the second segment and the third segment, and between the first segment and the third segment increases. After the passage of the device, the diameter of the lumen decreases from the second expanded diameter *dₑ* to a diameter that is substantially identical to the first rest diameter *dᵣ*.

It is further understood that the *d_{r,}* as disclosed above, can be uniform along a length of the sheath or vary from the proximal end of the sheath to the distal end of the sheath, as shown in **FIGS. 3A-3C****.** It is further understood that any values of dᵣ disclosed herein are also applicable to the sheath configurations, as shown in **FIG. 18** and **19****.**

During transcatheter aortic valve replacement (TAVR) procedures, a sheath as disclosed herein is used to provide access to the vasculature with no trauma to the patient, acting to maintain hemostasis and facilitate the delivery of interventional devices, as well as wire and catheter exchanges. In order for the sheath to be as minimally invasive as possible, it must have a low profile, or low outer diameter (OD), upon entry. However, the sheath must expand to a larger diameter once inside the body in order to allow passage of catheters larger than the initial diameter of the sheath. The force to advance these devices through the sheath is commonly referred to as push force. With larger devices, such as a crimped valve on a delivery system (DS), as well as challenging vessel anatomies, such as small, tortuous, or constricted vessels, the push force during the procedure is of great importance. High push force can cause procedural delays, physician dissatisfaction, and even inability to complete the procedure.

An important function of the sheath, then, is to have clinically acceptable push force to advance the delivery system and valve throughout the sheath for all patient anatomies. In some aspects, lubrication is used to aid in this reduction of push force. Lubrication is placed in between layers that slide over one another, reducing the frictional forces that are needed to be overcome to expand the sheath, thus making the DS easier to pass through the sheath. Recent studies have shown that lubrication is needed in order to bring push forces down to acceptable levels.

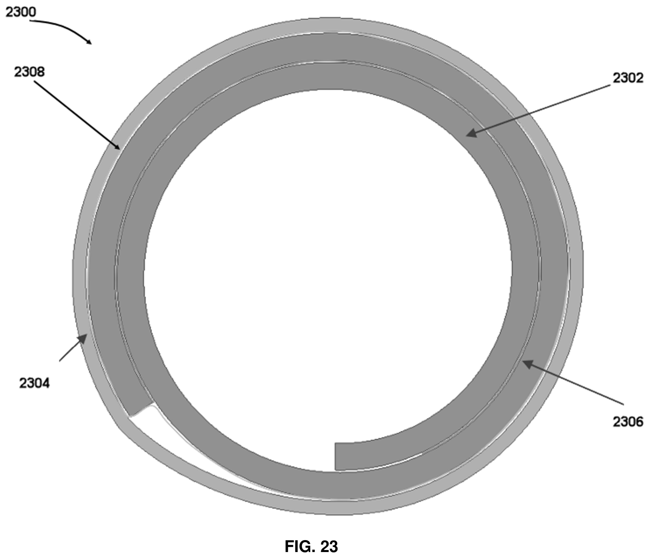

Still, further, the sheath configurations, as disclosed herein, can comprise a lubricant. One exemplary aspect of the sheath **2300** is shown in **FIG. 23****.** In this configuration, for example, a lubricant **2306** can be disposed between an inner liner **2302** and an outer layer **2304.**

For sheath having a configuration as shown in **FIG. 18** and **19****,** a lubricant can be disposed between any portions and segments in any amounts and any combinations. In certain exemplary and unlimiting aspects, the lubricant can be disposed between the first and the second segment, or between the second and the third segment, or between the first and the third segment, or any combination thereof. In yet other aspects, the lubricant can be disposed such that it is positioned on an innermost surface of the sheath, or on an outermost surface of the sheath, or a combination thereof.

In still further aspects, the lubricant is disposed along a whole circumference of the inner liner, or it can be disposed between at least a portion of the overlying portion of the sheet and at least a portion of the sliding portions of the sheet.

Still, further, the lubricant can be disposed along at least a portion of the inner surface of the sheet or at least a portion of the outer surface of the sheet or a combination thereof.

Any known in the art lubricants can be utilized. In yet further aspects, the lubricant can comprise a PTFE-based lubricant or a silicone-based lubricant. In certain and unlimiting aspects, the lubricants can comprise Christo Lube supplied by ECL, or MED10/6670 or PRO-3499 supplied by Nusil, or PRO-3595 also supplied by Nusil. In still further aspects, it is understood that the amount of the first and/or second lubricant can be easily determined by one of ordinary skill in the art.

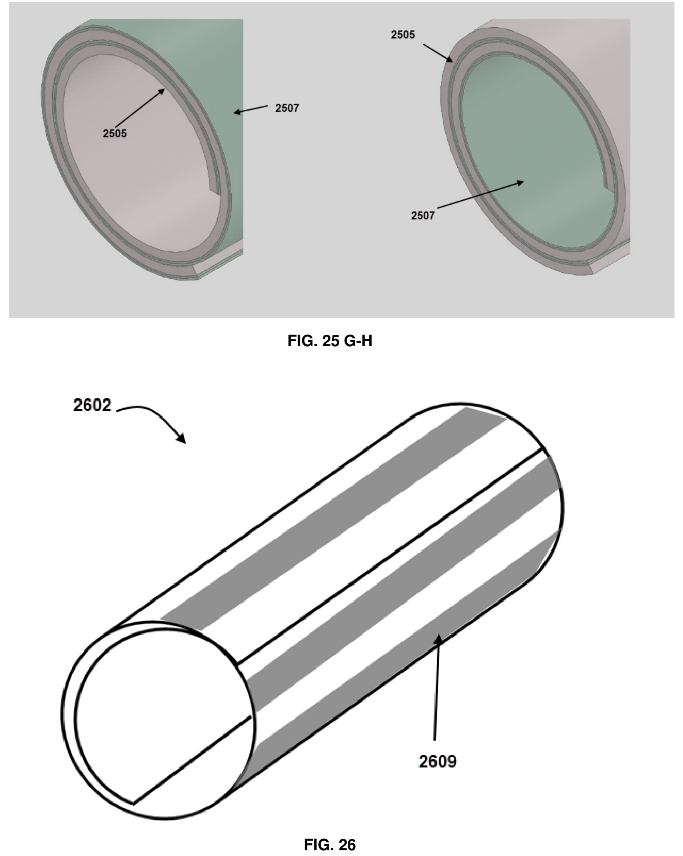

In still certain aspects, the lubricant can be disposed in a predetermined pattern, as shown, for example, in **FIG. 26****.** In such aspects, the lubricant is disposed in a pattern **2609,** for example, on the inner liner **2602.** It is understood that the pattern **2609** is only exemplary, and any desired pattern for any specific application can be applied.

The lubricant can be applied in any manner. For example, it can be applied manually. Lubrication is brushed by hand onto the sheath, making it hard to precisely control how much lubrication is added to the sheath and the exact locations where the lubrication is applied onto the sheath.

Lubricants, as disclosed herein, therefore, can also be applied by pad-printing or spraying that results in the material applied in a precisely controlled and repeatable manner, suitable for large-scale manufacturing. Detailed methods of lubricant application are discussed below.

It is understood, however, that in aspects where the lubricant is applied by pad-printing, the lubricant has a viscosity prior to it is application from about 600 to about 1,200 cP, including exemplary values of about 650 cP, about 700 cP, about 750 cP, about 800 cP, about 850 cP, about 900 cP, about 950 cP, about 1,000 cP, about 1,050 cP, about 1,100 cP, and about 1150 cP.

While in aspects where the lubricant is sprayed, the lubricant can have a viscosity equal to or less than about 600 cP, or about 550 cP, about 500 cP, about 450 cP, about 400 cP, about 350 cP, or equal to or less than about 350 cP.

In still further aspects, when the lubricant is cured prior to positioning the outer layer on the inner liner of the sheath.

The lubricants can also form a film. When a film of the lubricant is formed, such a film can have a thickness of equal to or less than about 20 µm, about 15 µm, about 10 µm, about 5 µm, about 1 µm, or even equal to or less than about 0.5 µm.

In still further aspects, the inner liner of any of the configurations disclosed herein can comprise a polyolefin, polyamide, fluoropolymer, copolymers thereof or blends thereof. In still further aspects, the polyolefin can comprise a high-density polyethylene, polypropylene, or blends thereof.

In still further aspects, the sheet can comprise one or more layers. In yet further aspects, the sheet can have a multilayer structure. In some aspects, if one or more layers are present, each layer can comprise the same or different polymer. In still further aspects, the sheet can have a predetermined thickness, wherein the predetermined thickness can be defined by one of ordinary skill in the art depending on the specific application. In certain aspects, the predetermined thickness of the inner liner can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner of any of the disclosed herein configurations can be varied depending on the desired amount of radial expansion, as well as the strength required.

In certain aspects, the inner liner of any of the sheath configurations described herein can comprise a compound material. For example, the polymer layer of the sheet used to form the inner liner can comprise a compound material comprising a polyolefin and a lubricious filler. It is understood that any of the polyolefins mentioned above can be used. In some exemplary aspects, the polyolefin used in the compound material is high-density polyethylene. In yet other aspects, the lubricious filler can be any filler that can improve the lubricity of the polymer layer and decrease its overall friction coefficient of the liner. In some exemplary and unlimiting aspects, the lubricious filler can comprise any additive that is known to reduce friction and behave as a lubricant. In such exemplary and unlimiting aspects, the lubricious filler can comprise one or more of graphene, reduced graphene oxide, carbon black, boron nitride, silicones, talc, polytetrafluorethylene (PTFE), fluorinated ethylene propylene, and the like. In still further aspects, the lubricious filler comprises a PTFE filler. In yet further aspects, the PTFE filler is a powder.

In yet further aspects, the lubricious filler can be present in any amount. In some exemplary and unlimiting aspects, the lubricious filler can be present in an amount from about 5 wt % to about 20 wt% of a total weight of the compound material used to make the polymer layer of the inner liner. In yet further aspects, the lubricious filler can be present in an exemplary amount of about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, or about 20 wt%.

In still further aspects, the sheet comprising such compound materials is lubricious and can have a coefficient of friction less than about 0.5, less than about 0.4, less than about 0.3, less than about 0.2, less than about 0.1, or less than about 0.05, or even less than about 0.01. It is further understood that the sheet can have a coefficient of friction having any value between any two foregoing values.

It is further understood that when the polymer layer of the sheet used to form the inner liner comprises the disclosed herein compound material, the sheath can be substantially free of a separately disposed lubricant. For example, the lubricant as disclosed above that is applied between the overlapping portions of the inner liner or between an outermost surface of the inner liner and an innermost surface of the outer layer may not be needed if the inner liner itself comprises a lubricious filler. However, disclosed herein are also aspects of the sheath where the inner liner comprises a lubricious compound in its composition, and a separate lubricant as disclosed above is still applied between various portions of the sheath. In such exemplary aspects, this additional lubricant applied manually, pad-printed, or sprayed can be applied between some of the portions of the inner liner and outer layer or all the portions of the inner liner and outer layer as disclosed above. It is also understood that this additional lubricant can be applied in any pattern that is desirable. It can also be applied along all the length of the sheath or only some portions of the sheath. The lubricant can also be applied in different patterns in different portions of the sheath. Yet, in other aspects, the lubricant can be applied in the same pattern along various portions of the sheath.

In still further aspects, the inner surface of the sheet can be at least partially ribbed. In such exemplary aspects, the inner surface of the sheet can be at least partially ribbed prior to rolling the sheet into the spiral configuration to form the inner liner.

In some aspects, the sheath described herein that comprises the lubricious material in the inner liner can exhibit a push force needed to move the prosthetic device through the sheath that is comparable or even smaller than a push force of a substantially identical reference sheath rolled in a spiral configuration, wherein an inner liner of the substantially identical reference sheath comprises a polymer layer substantially free of a lubricious filler and comprises an amount of a lubricant disposed between overlapping portions of the spiral configuration and/or an outermost surface of the inner liner. In other words, in some aspects when the performances of any of the disclosed herein, sheath configurations are compared, in such exemplary and unlimiting aspects, the sheath having the lubricious material in the inner liner and no additional lubricant present can demonstrate similar or even better performance than the similar sheath without lubricious material present in the inner liner but having an additional lubricant dispersed between various portions of the sheath.

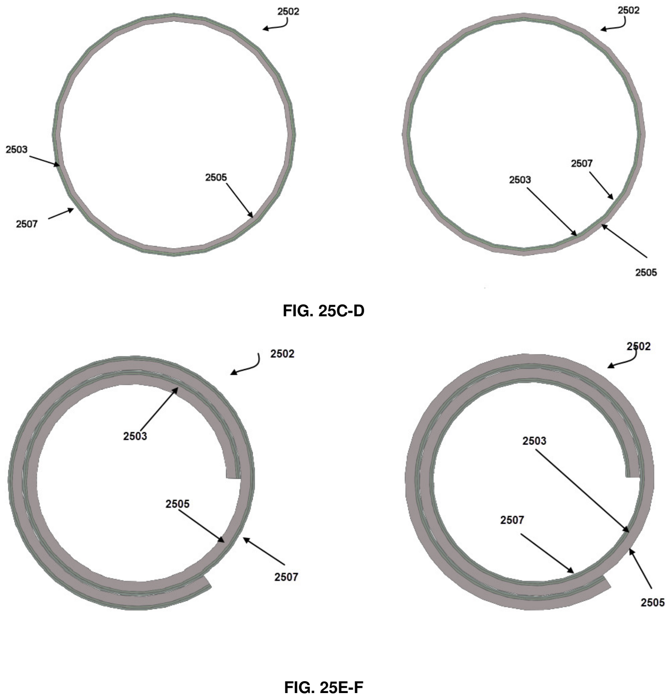

In still further aspects and as described herein, the sheath can also comprise a tie layer. In such aspects, the tie layer can be disposed at the inner surface of the inner liner or the outer surface of the inner liner, referring to **FIGS. 25A-B,** for example. **FIGS. 25A-B,** show an exemplary coextruded tubing that can be used to form the sheet that is then rolled into the spiral configuration. The specifics of the methods of forming the inner liner are discussed in more detail below. Here, **FIGS. 25A-B,** show the coextruded tubing **2502** comprising a polymer layer **2505** and a tie layer **2503.** **FIG. 25A** shows the tie layer **2503** is coextruded with the polymer layer **2505** such that the tie layer is positioned on an outer surface of the polymer layer. It is understood that the outer surface of the polymer layer will define at least a portion of the outer surface of the inner liner when it is in the spiral configuration. **FIG. 25B** shows a tie layer **2503** being coextruded with the polymer layer **2505** such that the tie layer **2503** is positioned on an inner surface of the polymer layer. It is understood that the inner surface of the polymer layer will define at least a portion of the inner surface of the inner liner when it is in the spiral configuration.

It is understood that the polymer layer **2505** can be any polymer layer described above and used to make the sheet. In certain aspects, the polymer layer can be high-density polyethylene.

In yet further aspects, the tie layer **2503** can comprise any material suitable for the desired application. It is understood that the tie layer can have adhesive or bonding properties. In certain aspect, the tie layer can comprise a polyurethane material such as Tecoflex, or polymer, copolymer, or terpolymer such as maleic anhydride modified polyolefin, for example, and without limitation, Orevac^{®} (commercially available from Arkema), ethylene acrylic acid copolymers, such as DOW Chemical Primacor^{®}, ethylene acrylate copolymers such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymers such as Lotader^{®} or Orevac^{®} (commercially available from Arkema).

In certain aspects, a total thickness of the sheet having the polymer layer and the tie layer can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the total thickness of the sheet forming the inner liner of any of the disclosed herein configurations can be varied depending on the desired amount of radial expansion, as well as the strength required.

In yet further aspects, the tie layer can have a thickness from about 0.001" to about 0.003", including exemplary values of about 0.0011", about 0.0012", about 0.0013", about 0.0014", about 0.0015", about 0.0016", about 0.0017", about 0.0018", 0.0019", about 0.0020", about 0.0021", about 0.0022", about 0.0023", about 0.0024", about 0.0025", about 0.0026", about 0.0027", about 0.0028", and about 0.0029".

In still further aspects, any of the disclosed herein sheath configurations can have at least one lubricious liner, as for example shown in **FIGS. 25C-D**

**FIGS. 25C-D** show an exemplary coextruded tubing comprising the tie layer **2503** and the polymer layer **2505,** and the disclosed above lubricious liner **2507.** This tubing can be used to form the sheet that is then rolled into the spiral configuration. The specifics of the methods of forming the inner liner are discussed in more detail below. As can be seen herein, the lubricous liner **2507** is disposed on the tie layer **2503.** It is understood that in one configuration, the tie layer and the lubricious liner are disposed on the outer surface of the polymer layer **(****FIG. 25C****)** or on the inner surface of the polymer layer **(****FIG. 25D****).** In yet further aspects, the lubricious liner is bonded to the polymer layer of the sheet with the tie layer.

**FIGS. 25E-H** show various configurations of sheet **2502** in the spiral configuration when the sheet comprises the polymer layer **2505,** the tie layer **2503,** and the lubricious liner **2507.**

The lubricious liner can comprise any material that can reduce the coefficient of friction of the sheath. In some exemplary and unlimiting aspects, the lubricious liner can comprise PTFE, polyether block amide, silicone-based liners, perfluoro alkoxy alkane-based liner, e-PTFE, ethylene tetrafluoroethylene, and the like. In yet further aspects, the lubricious liner comprises PTFE.

In yet further aspect, the total thickness of the sheath can be any thickness, as disclosed above.

Yet in other aspects, the at least one lubricious liner has a thickness from about 0.001" to about 0.005", including exemplary values of about 0.0011", about 0.0012", about 0.0013", about 0.0014", about 0.0015", about 0.0016", about 0.0017", about 0.0018", 0.0019", about 0.0020", about 0.0021", about 0.0022", about 0.0023", about 0.0024", about 0.0025", about 0.0026", about 0.0027", about 0.0028", about 0.0029", about 0.0030" about 0.0031", about 0.0032", about 0.0033", about 0.0034", about 0.0035", about 0.0036", about 0.0037", about 0.0038", 0.0039", about 0.0040", about 0.0041", about 0.0042", about 0.0043", about 0.0044", about 0.0045", about 0.0046", about 0.0047", about 0.0048", and about 0.0049".

In still further aspects, the lubricious liner can be further ribbed. It is also understood that aspects comprising an additional lubricant added separately from the lubricious liner are also disclosed. In such aspects, an additional lubricant can be disposed by any of the disclosed herein methods. It can be manually disposed, pad-printed, or sprayed. It is further understood that when this additional lubricant is present, it can be disposed in any of the disclosed herein predetermined patterns, along a portion of the sheath length or along a whole length of the sheath. In yet other aspects, the additional lubricant is not present when the lubricant layer, as described herein, is present.

In still further aspects, the outer layer of any one of the sheath configurations can comprise a styrene-based elastomer, polyurethane, latex, copolymers thereof, blends thereof, or co-extrudates thereof. In certain and unlimiting aspects, the polymer can comprise polyether block ester copolymer, polyesters, polyvinyl chloride, thermoset silicone, poly-isoprene rubbers, polyolefin, other medical grade polymers, or combinations thereof.

In still further aspects, the outer layer can comprise one or more layers. In some aspects, at least one layer comprises the styrene-based elastomer. In yet other aspects, at least one layer can comprise polyurethane. While in other aspects, the at least one layer comprises a blend of the styrene-based elastomer and polyurethane.

It is understood that the hardness of each layer of the disclosed sheath can also be varied depending on the particular application and desired properties of the sheath. In some aspects, the layer of the outer layer has a Shore A durometer between 20A to 50A, including exemplary values of about 25A, about 30A, about 35A, about 40A, and about 45 A.

In yet further aspects, the polymers layer of the outer layer can have a Shore hardness of less than 90 Durometer, less than 80 Durometer, less than 70 Durometer, less than 60 Durometer, less than 50 Durometer, less than 40 Durometer, less than 30 Durometer, or less than 20 Durometer. In yet further exemplary aspects, the polymers layer of the outer layer can have a Shore hardness from about 25 Durometer to about 75 Durometer, including exemplary values of about 30 Durometer, about 35 Durometer, about 40 Durometer, about 45 Durometer, about 50 Durometer, about 55 Durometer, about 60 Durometer, about 65 Durometer, and about 70 Durometer.

The sheath as shown in any of the preceding configurations can also comprise an outer layer comprising a first polymer layer, wherein the first polymer layer comprises a first compound composition comprising from greater than 0 wt% to less than 100 wt% of a polymer comprising a polyether block amide, a polyurethane, or a combination thereof; less than about 65 % of an inorganic filler based on a total weight of the first compound composition; and up to about 20 % of a solid lubricant filler based on a total weight of the first compound composition. It is understood, however, that there are also aspects where the disclosed sheaths can comprise additional components. These exemplary aspects are disclosed herein, as shown below in detail.

In certain aspects, the outer layer comprises a first polymer layer. In such exemplary aspects, the first polymer layer can comprise a first compound composition comprising from greater than 0 wt% to less than 100 wt%, including exemplary values of about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof.

In still further aspects, the first compound composition can comprise from greater than about 35 wt% to less than about 80 wt%, including exemplary values of about 40 wt%, about 45 wt%, about 50 wt%, about 55 wt%, about 60 wt%, about 65 wt%, about 70 wt%, and about 75 wt% of a polymer comprising a polyether block amide, a polyurethane, or any combination thereof.

In certain aspects, the polymer in the first compound composition comprises a polyether block amide. In such exemplary aspects, the polyether block amide can comprise PEBAX^{®} from Arkema. In yet further aspects, the polymer can comprise polyurethane, for example, NEUSoft^{®}. While in still further aspects, the polymer can compromise a combination of the polyether block amide, such as, for example, PEBAX^{®} and polyurethane. It is further understood that if the mixture of the polymers is present, such a mixture can comprise each component in any amount relative to another component to provide the desired polymer falling within the disclosed above range.

In still further aspects, the first compound composition can comprise less than about 65 wt% of an inorganic filler based on a total weight of the first compound composition, including exemplary values of less than about 60 wt%, less than about 55 wt%, less than about 50 wt%, less than about 45 wt%, less than about 40 wt%, less than about 35 wt%, less than about 30 wt%, less than about 25 wt%, less than about 20 wt%, less than about 15 wt%, less than about 10 wt%, less than about 5 wt%, and less than about 1 wt% of the inorganic filler.

In yet further aspects, the inorganic filler can be present in an amount of at least about 1 wt%, at least about 2 wt%, at least about 5 wt%, at least about 10 wt%, at least about 15 wt%, at least about 20 wt%, at least about 25 wt%, at least about 30 wt%, at least about 35 wt%, at least about 40 wt%, at least about 45 wt%, at least about 50 wt%, or at least about 55 wt%.

In still further aspects, the inorganic filler can comprise any inorganic materials that can be used as a filler and are acceptable for the desired application. In certain exemplary and unlimiting aspects, the inorganic filler can comprise bismuth oxychloride, barium sulfate, bismuth subcarbonate, calcium carbonate, aluminum trihydrate, barite, kaolin clay, limestone, or any combination thereof. Again, it is understood that the inorganic filler can comprise a combination of the various fillers. In such exemplary aspects, an amount of each filler in the combination can be in any range to provide a final combination that falls within the disclosed above range.

In still further aspects, the first compound composition can comprise up to about 20 wt% of a solid lubricant filler based on a total weight of the first compound composition, including exemplary values of about 0.01 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, and about 19.9 wt%. In yet further aspects, the solid lubricant filler can be present up to about 20 wt%, up to about 15 wt%, or up to about 10 wt% based on a total weight of the first compound composition.

In still further aspects, the solid lubricant filler can comprise any additive that is known to reduce friction and behave as a lubricant. In such exemplary and unlimiting aspects, the solid lubricant filler can comprise one or more of graphene, reduced graphene oxide, carbon black, boron nitride, silicones, talc, polytetrafluorethylene (PTFE), fluorinated ethylene propylene, and the like. In still further aspects, the solid lubricant comprises a PTFE filler. In yet further aspects, the PTFE filler is a powder.

In still further aspects, the first compound composition can further comprise at least one tackiness reducing compound. Any compounds known in the art as capable of reducing the tackiness of the polymer composition can be considered and used for the purpose of this disclosure. In yet further exemplary and unlimiting aspects, the at least one tackiness reducing compound comprises ProPell^{™} from Foster Corporation
In certain aspects, the at least one tackiness reducing compound is present in an amount from 0 wt% to about 20 wt%, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the first compound composition. In still further aspects, the at least one tackiness reducing compound is present in any amount having a value between any two foregoing values. For example, and without limitation, the at least one tackiness reducing compound can be present in an amount from about 1 wt% to about 5 wt%, or from about 5 wt% to about 10 wt% based on a total weight of the first compound composition.

In still further aspects and as disclosed herein, the polymer in the first polymer layer composition has a substantially same durometer along a total length of the outer layer. It is understood, however, the durometer of the polymer in the first polymer layer composition of the outer layer can also be varied along the length of the outer layer. For example, and without limitation, disclosed herein are aspects where a durometer of the polymer in the first polymer layer composition at a proximal end of the outer layer is different from a durometer of the polymer in the first polymer layer composition at a distal end of the outer layer.

In still further aspects, the polymer in the first polymer layer composition has a Shore D from about 20D to about 72D, including exemplary values of about 25D, about 30D, about 35D, about 40D, about 45D, about 50D, about 55D, about 60D, about 65D, and about 70D. In still further aspects, the polymer in the first polymer layer composition has a Shore D from about 20D to about 35D. In still further aspects, the polymer in the first polymer layer composition has a Shore D of about 30D. Yet, in still further aspects, the polymer in the first polymer layer composition has a Shore D of about 25D.

It is understood that the outer layer, as disclosed herein, can comprise aspects where only one polymer layer is present. Yet, in other aspects, two or more polymer layers can be present in the outer layer. In such exemplary aspects, the outer layer comprises at least a second polymer layer comprising a second compound composition comprising from greater than 0 wt% to 100 wt% of a second polymer comprising polyether block amide, a polyurethane, or a composition thereof. Similar to the first compound composition, the second polymer can be present in any amount that falls within the disclosed range. For example, the second polymer can be present in the second compound composition from greater than 0 wt%, about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof. In yet further aspects, the second polymer can be present in the second compound composition from greater than about 95 wt% to less than about 99wt%, including exemplary values of about 95.5 wt %, about 96 wt%, 96.5 wt %, about 97 wt%, about 97.5 wt %, about 98 wt%, and about 98.5 wt%.

In yet further aspects, the second compound composition can further comprise up to 20 wt% of a tackiness reducing additive, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the second compound composition. In still further aspects, the at least one tackiness reducing compound is present in any amount having a value between any two foregoing values. For example, and without limitation, the at least one tackiness reducing compound can be present in an amount from about 1 wt% to about 5 wt%, or from about 5 wt% to about 10 wt% based on a total weight of the second compound composition. In still further aspects and as disclosed herein, the second compound composition can be substantially free of a solid lubricant filler.

It is further understood that in certain aspects, the first polymer in the first compound composition can be the same as the second polymer in the second compound composition. Yet, in other aspects, the first polymer in the first compound composition is different from the second polymer in the second compound composition. In yet further aspects, the second polymer layer composition comprises PEBAX^{®}. While in further aspects, the second polymer layer composition can comprise polyurethane, for example, NEUSoft^{®} from PolyOne.

In still further aspects, the second polymer has a Shore D from about 20D to about 35D. Yet, in further aspects, the second polymer has a Shore D of about 25D or about 35D.

In still further aspects, the second compound composition can be substantially free of an inorganic filler. While in certain aspects, the inorganic filler can be present in the second compound composition in any amount from greater than 0 wt% to less than 100 wt%, including exemplary values of about 0.01 wt%, about 0.05 wt%, about 0.1 wt%, about 0.5 wt%, about 1 wt%, about 5 wt%, about 10 wt%, about 20 wt%, about 30 wt%, about 40 wt%, about 50 wt%, about 60 wt%, about 70 wt%, about 80 wt%, about 90 wt%, and about 95 wt%. In the aspects where the inorganic filler is present in the second compound composition, such inorganic filler can comprise any filler disclosed above.

In still further aspects, and as disclosed herein, the outer layer has a predetermined thickness, and wherein at least about 10%, at least about 20%, at least about 30%, at least about 40%, at least about 50%, at least about 60%, at least about 70%, at least about 80%, at least about 90%, or 100% of the predetermined thickness comprises the first and/or the second compound composition comprising the first and/or the second polymer having a Shore D equal to or lower than about 30D.

In still further aspects, the predetermined thickness of the outer layer can vary along a length of the sheath. While in other aspects, the predetermined thickness of the outer layer is the same along a length of the sheath. Yet, in further aspects, the predetermined thickness of the outer layer is greater at the proximal end. In still further aspects, the predetermined thickness of the outer layer is up to 0.006", for example, and without limitation from about 0.001" to about 0.006", including exemplary values of about 0.0015", about 0.002", about 0.0025", about 0.003", about 0.0035", about 0.004", about 0.0045", about 0.005", about 0.0055", and about 0.006".

In still further aspects, the first polymer layer and the second polymer layer can have the same thickness. While in other aspects, the first polymer layer and the second polymer layer have different thicknesses. For example, in some aspects, the first polymer layer has a thickness of about .001" to about 0.003", including exemplary values of about 0.0011", about 0.0012", about 0.0013", about 0.0014", about 0.0015", about 0.0016", about 0.0017", about 0.0018", about 0.0019", about 0.0020", about 0.0021", about 0.0022", about 0.0023", about 0.0024", about 0.0025", about 0.0026", about 0.0027", about 0.0028", and about 0.0029". Yet still, in further aspects, the second polymer layer can have a thickness of about 0.002" to about 0.004", including exemplary values of about 0.0011", about 0.0012", about 0.0013", about 0.0014", about 0.0015", about 0.0016", about 0.0017", about 0.0018", about 0.0019", about 0.0020", about 0.0021", about 0.0022", about 0.0023", about 0.0024", about 0.0025", about 0.0026", about 0.0027", about 0.0028", about 0.0029", 0.0030", about 0.0031", about 0.0032", about 0.0033", about 0.0034", about 0.0035", about 0.0036", about 0.0037", about 0.0038", and 0.0039".

In still further aspects, the predetermined thickness of the outer layer is greater at the proximal end. While in other aspects, the predetermined thickness of the outer layer smaller at the distal end as compared to the predetermined thickness of the outer layer at the proximal end.

In still further aspects where two or more layers are present in the outer layer, the first polymer layer can define the inner surface of the outer layer, while the second polymer layer can define the outer surface of the outer layer. However, there are also aspects, where the first polymer layer defines the outer surface of the outer layer, while the second polymer layer defines the inner surface of the outer layer. It is also understood that other aspects are also enclosed, where one or more additional polymer layers are disposed between the first polymer layer and the second polymer layer.

In still further aspects, the outer layer is extruded as a tube that can then be slide on the inner liner of the sheath. In the aspects where the first and the second polymer layers are present, such polymer layers can be co-extruded. In still further aspects, the first polymer layer can be substantially bonded to the second polymer layer. In such exemplary aspects, the first polymer layer substantially does not delaminate from the second polymer layer. It is understood that in some aspects, the bonding can be physical or chemical or any other type known in the art.

In still further aspects, any sheath that comprises the disclosed herein outer layer can exhibit an insertion force of less than about 55 N, less than about 50 N, less than about 45 N, less than about 40 N, less than about 35 N, or less than about 35 N when a medical device is pushed through the sheath.

In still further aspects, the outer layer can also exhibit a friction force of less than about 10 N, or less than about 9 N, or less than about 8 N, or less than about 7 N, or less than about 6 N, or even less than about 5 N, in the dry state against a substrate surface comprising one or more of polytetrafluoroethylene, fluorinated ethylene propylene, or high density polyethylene having a diameter of about 0.300".

In still further aspects, the outer layer extruded as a tube can exhibit a hoop force at 10 mm extension (about 85% strain) of less than about 10 N, or less than about 9 N., or less than about 8 N, or less than about 7 N, or less than about 6 N, or even less than about 5 N. In such exemplary aspects, the extruded tube that will form the outer layer of the sheath can have a diameter of about 0.290" (7.4 mm) and wall thickness as disclosed herein. In aspects where the outer layer has a diameter of about 0.290" (7.4 mm) and a total wall thickness of about 0.0045", with a sample length of about 0.25" (6.4 mm), a hoop direction forces at 10 mm extension can be less than about 8 N. It is understood that in some exemplary and unlimiting aspects, a low force at 10 mm extension is desired for low sheath expansion force.

In still further aspects, the outer layer can exhibit an elongation at break of ranging between about 650 % and about 800 %, including exemplary values of about 680 %, about 700 %, about 710 %, about 750 %, and about 780 %. It is understood that in some exemplary and unlimiting aspects, a high elongation is preferable for expansion to a larger diameter before the outer layer breaks.

In certain aspects, the outer layer extends along a portion of the length of the sheath. In such exemplary aspects, the outer layer can be positioned at the proximal end of the sheath, or in the middle of the sheath, or at the distal portion of the sheath. While in other aspects, the outer layer extends along the whole length of the sheath. In such exemplary aspects, the outer layer can be positioned at the proximal end of the sheath and extend to the distal end of the sheath.

In still further aspects, the outer layer of any one of the disclosed herein sheath configurations can comprise one or more polymer layers. In some aspects, a first polymer layer can be the first polymer layer disclosed above. Yet, in other aspects, the outer layer can also comprise a second polymer layer, wherein the second polymer layer can be any second polymer layer disclosed above. In some exemplary and unlimiting aspects, the second polymer layer can comprise polyurethane. In some exemplary and unlimiting aspects, the first polymer layer can comprise PEBAX alone or in combination with the inorganic filler and the solid lubricant filler, as disclosed above. In yet other exemplary and unlimiting aspects, the second polymer layer can comprise polyurethane, such as Neusoft.

In certain aspects, the first polymer layer, as disclosed above, and the second polymer can be coextruded to form a bump tubing. It is understood that a bump tubing, or a tapered tubing, are commonly used in various applications.

It is understood that in some aspects, the bump tubing or the tapered tubing can be especially useful for certain catheter applications. Neurovascular and microcatheters usually depend on a larger proximal diameter to increase the pushability of the device, while a smaller distal end provides improved performance and deliverability.

In some aspects disclosed herein, the bump tubing that forms the outer layer of the sheath can have a predetermined length that is substantially similar to a length of the sheath. Yet, in other aspects, the bump tubing that forms the outer layer of the sheath can have a predetermined length that is shorter than a length of the sheath.

In certain aspects, the first polymer layer can define an inner surface of the outer layer (bump tubing). In such aspects, the second polymer layer will define an outer surface of the outer layer.

Yet, in other aspects, it can define an outer surface of the outer layer (bump tubing). In such aspects, the second polymer layer will define an inner surface of the outer layer.

In still further aspects, where the outer layer has the described above configuration, the first polymer layer can have a thickness from about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095". It is understood that the thickness of the first polymer layer in the outer layer can be uniform along the length of the sheath. Yet, in other aspects, the thickness of the first polymer layer in the outer layer can vary along the length of the sheath. In some aspects, the thickness of the first polymer layer is greater at the proximal end of the sheath as compared to a thickness of the first polymer layer along other portions of the sheath. Yet, in other aspects, the thickness of the first polymer layer can be smaller at the distal end of the sheath as compared to a thickness of the first polymer layer at the proximal end of the sheath.

In some aspects, the second polymer layer, if present in the outer layer, can have a thickness from about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095". It is understood that the thickness of the second polymer layer in the outer layer can be uniform along the length of the sheath. Yet, in other aspects, the thickness of the outer polymer layer in the outer layer can vary along the length of the sheath. In some aspects, the thickness of the second polymer layer is greater at the proximal end of the sheath as compared to a thickness of the second polymer layer along other portions of the sheath. Yet, in other aspects, the thickness of the second polymer layer can be smaller at the distal end of the sheath as compared to a thickness of the second polymer layer at the proximal end of the sheath.

In still further aspects, the first polymer layer can have a Shore D from about 20D to about 72D, including exemplary values of about 25D, about 30D, about 35D, about 40D, about 45D, about 50D, about 55D, about 60D, about 65D, and about 70D. In still further aspects, the polymer in the first polymer layer composition has a Shore D from about 20D to about 35D. In still further aspects, the polymer in the first polymer layer composition has a Shore D of about 30D. Yet, in still further aspects, the polymer in the first polymer layer composition has a Shore D of about 25D.

Yet, in other aspects, the second polymer layer can have a Shore A from about 30A to about 80A, including exemplary values of about 40A, about 45A, about 50A, about 55A, about 60A, about 65A, about 70A, and about 75A.

In still further aspects, and as disclosed herein, the outer layer has a total predetermined thickness, and wherein at least about 10%, at least about 20%, at least about 30%, at least about 40%, at least about 50%, at least about 60%, at least about 70%, at least about 80%, at least about 90%, or 100% of the total predetermined thickness comprises the first compound composition comprising the first polymer having a Shore D from about 20D to about 35D. In still further aspects, the total predetermined thickness of the outer layer is up to about 0.02", or up to about 0.015", or up to about 0.01", or up to about 0.009", or up to about 0.008", or up to about 0.007", or up to about 0.006'.

In yet further aspects, the total predetermined thickness of the outer layer can be uniform along the length of the sheath, or it can vary along the length of the sheath. In some exemplary and unlimiting aspects, the total predetermined thickness of the outer layer is greater at the proximal end of the sheath. Yet, in other aspects, the total predetermined thickness of the outer layer is smaller at the distal end of the sheath as compared to the total predetermined thickness of the outer layer at the proximal end of the sheath.

Also disclosed herein are sheath aspects, where the outer layer has one or more layers and when these layers are formed separately. For example, the first and the second polymer layer, as described above, instead of being coextruded, are formed separately. In such aspects, the outer layer is formed by disposing one of the polymer layers on another.

In some exemplary and unlimiting aspects, the second polymer layer can be at least partially disposed over the first polymer layer. Also disclosed are aspects where the first polymer layer at least partially overlies the second polymer layer.

In certain aspects, when the two polymers are formed separately and disposed over each other, each of the polymer layers can have a different length.

In some exemplary and unlimiting aspects, the first polymer layer can have a length that is shorter than the length of the second polymer layer. In some aspects, the first polymer layer can be disposed on the inner liner at the proximal end of the sheath and have a length from about 5 cm to about 15 cm, including exemplary values of about 6 cm, about 7 cm, about 8 cm, about 9 cm, about 10cm, about 11 cm, about 12 cm, about 13 cm, and about 14 cm. In a still further aspect, the second polymer layer is then disposed on the first polymer layer. In such aspects, the second polymer layer can have any length that suits the desired application. In certain aspects, the second polymer layer can have a length that is substantially identical to the length of the sheath.

It is understood, however, that an opposite configuration of the outer layer is also disclosed. In such an aspect, the second polymer layer can be first disposed on the inner liner and have a length that is shorter than the length of the sheath. Further, the second polymer layer can be disposed on the second polymer layer. In such an exemplary aspect, the first polymer layer can have any length. In some aspects, the length of the first polymer layer can be substantially identical to the length of the sheath.

Still further, the first polymer layer, as disclosed in these aspects, can have a thickness that is uniform along the length of the first polymer layer, or it can vary along the length of the first polymer layer. The thickness of the first polymer layer can be any thickness, as disclosed above. In some aspects, the thickness can be anywhere between about 0.001" to about 0.006", including exemplary values of about 0.002", about 0.0025", about 0.003", about 0.0035", about 0.004", about 0.0045", about 0.005", and about 0.0055".

Still further, the second polymer layer, as disclosed in these aspects, can have a thickness that is uniform along the length of the second polymer layer, or it can vary along the length of the second polymer layer. The thickness of the first polymer layer can be any thickness, as disclosed above. In some aspects, the thickness can be anywhere between about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095".

In some exemplary and unlimiting aspects, if the two-polymer layer of the outer layer is formed separately, a tie layer can be disposed between the two. It is further understood that any of the disclosed above tie layers can be utilized.

As discussed in detail above, an important function of the sheath is to have clinically acceptable push force for all patient anatomies.

To decrease the push force, various types of lubrication can be used. Some of the various lubrication types and methods are disclosed above. As disclosed, any of the disclosed lubricants (lubricious materials, lubricious fillers, lubricious liners) can reduce the frictional force between various layers of the sheath that slide over one another, making it easier for the delivery system to open the sheath. However, lubrication between the inner liner and the outer layer can cause the outer layer to easily slide over the sheath shaft. When large portions of the outer layer slide together, the outer layer can bunch up in one spot, thereby raising the outer diameter (OD), called bunching. This can lead to trouble inserting or retrieving the sheath and a more traumatic interaction with the vessel.

In certain aspects, to avoid this problem, the outer layer can be bonded to the inner liner of an expandable sheath to prevent movement of the outer layer along the sheath shaft.

In certain aspects, the bonding of the inner liner and the outer layer can occur anywhere. In yet further aspects, such bonding can occur on an area of the sheath cross section where minimal stretching of the outer layer during expansion is set to occur. The bonding is also done in a location where no lubrication is applied.

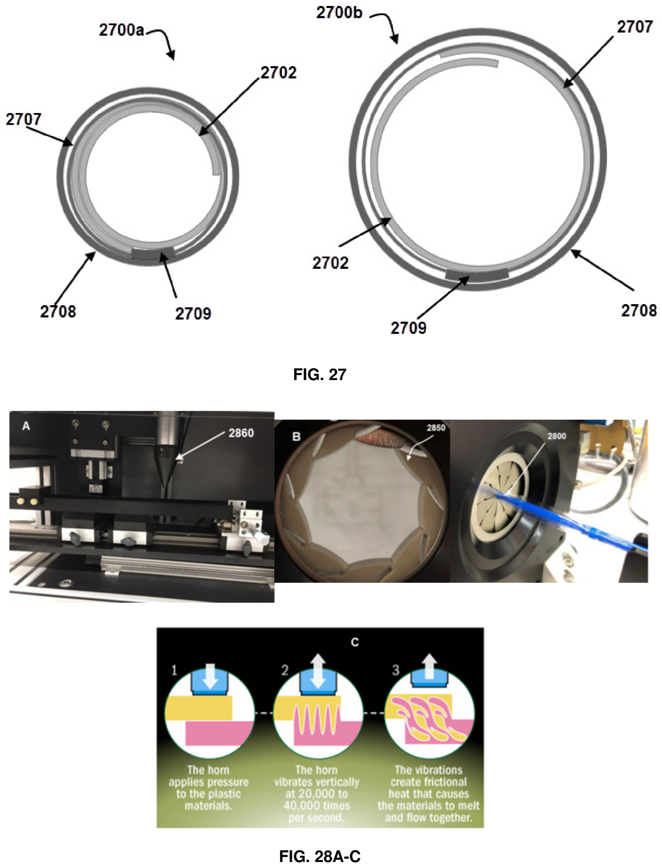

Thus, disclosed herein are aspects where at least a portion of an innermost surface of the outer layer is bonded to at least a portion of an outermost surface of the inner liner. Such exemplary aspects are also depicted in **FIG. 27****.** It is understood that such a bond can be optionally present in any one of the disclosed above sheath configurations. The bonding can be done by any methods known in the art. In some aspects, the bonding is done by laser welding, compression bonding, and/or selective ultrasonic welding.

In certain aspects, the bonding of the outer layer to the inner liner can occur on a section of the sheath cross section where the outer layer is not expected to stretch much or move relative to the inner liner during expansion. In yet further exemplary and unlimiting aspects, the portion of the inner member directly next to the end of its' outside layer meets this criterion. As shown in **FIG. 27****,** the bonding **2790** is done between the inner liner **2702** and the outer layer **2708** at a portion that is not expected to stretch much or move relative to the inner liner. In such aspects, lubrication **2707** is not applied in this location on the inner liner to ensure a good bond between the two components. At this location, the outer layer can be bonded in place along the length of the sheath shaft to prevent longitudinal movement over the inner member. In yet other aspects, the outer layer can be bonded to the inner liner at a portion of the sheath. Since the bond as disclosed herein does not hinder the inner liner movement relative to the outer layer or the stretching of the outer layer itself, the force required to expand the sheath is not adversely affected by the bond.

In still further aspects, it is understood that the bond location can be important to minimize push forces. Yet, in other aspects, the bond covers a relatively small portion of the sheath circumference. In yet other aspects, the methods of making the bond need to be precisely controlled, and repeatability of the process needs to be ensured. The specific methods of making the bond are described in detail below.

In still further aspects, two or more portions of the inner liner and the outer layer can be bonded together. In such exemplary aspects, the bond can be formed in a predetermined pattern. In yet further aspects, the bonding pattern can be aligned to the lubricant pattern. In such aspects, the bonding can be done at any portion of the sheath where the lubricant is not present.

It is understood that when the sheath is used to deliver a prosthetic device into a patient's vessel, hemostasis can be compromised if the blood from the patient's arteriotomy penetrates in between the inner liner and the outer layer. In such a scenario, the outer layer is the only sheath element that resists blood pressure and maintains hemostasis.

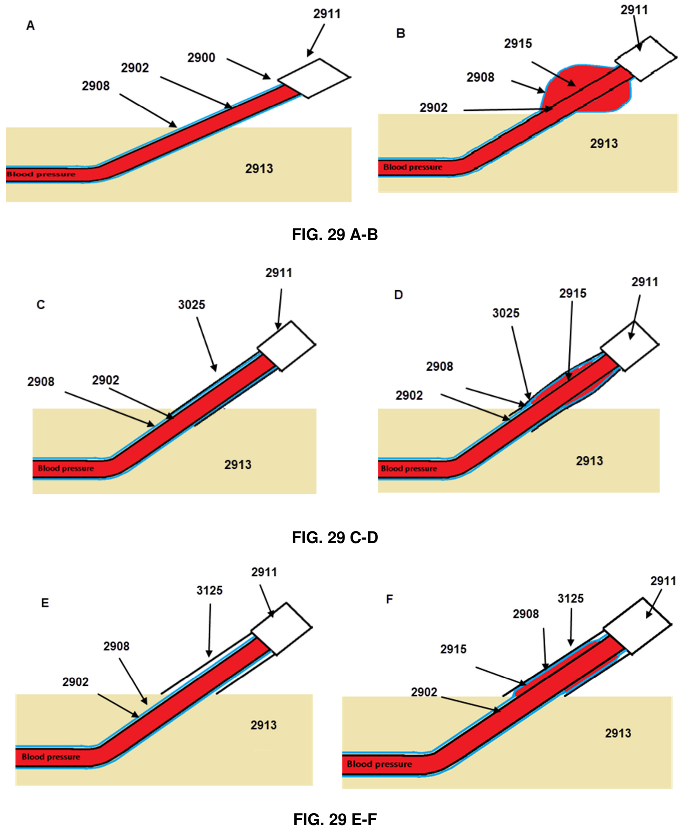

The outer layer is usually formed from the materials that one hand allows easy expansion of the inner liner and, on the other hand, can apply inward force on the inner liner to contract it to the original unexpanded configuration. However, high enough blood pressure may cause the outer layer to "balloon" and, at some point, even burst and compromise hemostasis. The schematic representation of such phenomena is shown in **FIGS. 29A-B.** An exemplary sheath **2900** having an inner liner **2902** and outer layer **2908** and connected to a hub **2911** is inserted into the patient anatomy **2913.** When hemostasis is not maintained, there is a possibility that the outer layer will "balloon" **2915** and undesirably affect the patient.

For the portion of the sheath that is under the skin level of the patient, the potential ballooning can be contained by the tissue surrounding the sheath and resists the blood pressure, but for the portion of the sheath that is outside the patient, this undesired phenomenon can still occur. The portion of the sheath that stays outside the patient's body varies and depends on the patient's size and anatomy, as well as on the physician's preferences.

Making the outer layer material stiff enough such that it will resist the blood pressure and will not balloon may have an undesired tradeoff of increasing the force required to expand the inner liner and thus increasing the force required to advance a delivery system through the sheath.

The aspects described herein address this issue and help prevent excessive outer layer ballooning on the portion of the sheath that stays outside the patient's body while having a minimal effect on the force to expand the sheath.

The aspects described herein are aimed to reinforce the outer layer of the sheath along its proximal section without having a significant impact on its ability to expand while the delivery system is being pushed through the sheath. It is understood that in some aspects described herein, the reinforced portion of the outer layer can fully remain outside of the patient arteriotomy. Yet, in other aspects, at least a portion of the reinforced portion can be inserted in the patient's vessel. Some exemplary schematic of the disclosed aspects is shown in **FIGS. 29C-D,** wherein the reinforcing layer **3025** is disposed on the outer layer 2908 and substantially prevents the "ballooning" effect. As shown in **FIG. 29D****,** the reinforcing portion of the sheath can be long enough that at least a portion of this reinforcing portion is inserted into the patient's anatomy **2913.**

In such aspects, the reinforcing of the outer layer at the proximal portion of the sheath can be achieved by disposing a reinforcing jacket having a proximal end and a distal end at at least a portion of the outer layer. The reinforcing jacket, as disclosed herein, can comprise an elastomeric material and a reinforcing element. This reinforcing jacket is then positioned on the proximal portion of the outer layer. In such aspects, the end of the reinforcing jacket is substantially seamlessly bonded to at least a portion of the outer surface of the outer layer. This smooth transition between the outer layer and the reinforcing jacket allows the sheath to be inserted into the patient's body.

In still further aspects, the proximal end of the reinforcing jacket can be bonded to the proximal end of the outer layer. However, also disclosed are the aspect where the proximal end of the reinforcing jacket is not bonded to the proximal end of the outer layer.

In certain aspect, the reinforcing jacket can have a length from about 5 to about 15 cm, including exemplary values of about 6 cm, about 7 cm, about 8 cm, about 9 cm, about 10 cm, about 11 cm, about 12 cm, about 13 cm, and about 14 cm.

In still further aspects, the elastomeric material present in the reinforcing jacket can be any elastomer known in the art. In some aspects, the elastomeric material comprises a polyether block amide, styrene-based elastomer, polyurethane, latex, copolymers thereof, blends thereof or co-extrudates thereof. In still further aspects, the elastomer can include silicon-based elastomers.

In still further aspects, the elastomeric material can have a Shore hardness of about 10A to about 80A, including exemplary values of about 20A, about 25A, about 30A, about 35A, about 40A, about 45A, about 50A, about 55A, about 60A, about 65A, about 70A, and about 75A.

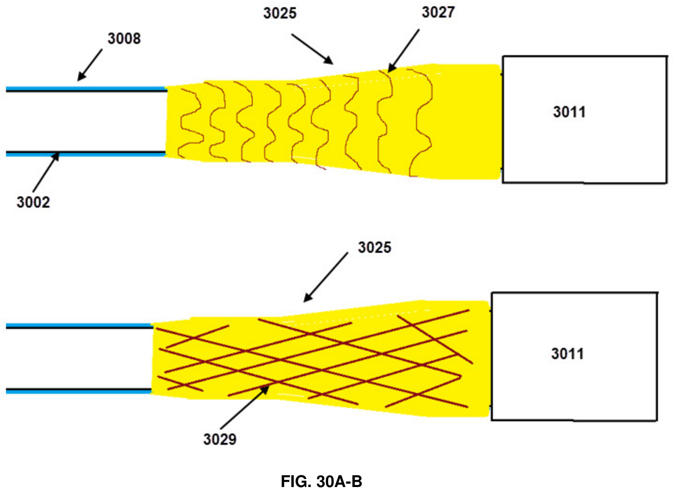

In still further aspects, the reinforcing jacket comprises a reinforcing element. Some exemplary schematic of various reinforcing jackets is shown in **FIGS. 30A-B.** The reinforcing jacket **3025** is seamlessly bonded at the distal end of the reinforcing jacket to the outer layer **3008** that is disposed on the inner liner **3002.** The proximal end of the reinforcing jacket may or may not bonded to the inner liner and/or hub **3011.** The reinforcing filament **3027** is disposed within the elastomer. The reinforcing element that has a minimal impact on the resistance of the outer layer to expand up to a certain diameter in which its resistance increases dramatically.

When a delivery system is pushed through the sheath, it requires only a limited amount of expansion to accommodate the OD of the crimped valve. This limited expansion will not engage the reinforcing element, so the impact on the force to expand the sheath up to this point will minimal and only be derived from the low durometer elastomer. In case a ballooning phenomenon starts, the diameter of the outer layer and reinforcing layer will increase only until the reinforcing element come into effect and prevents excessive ballooning.

In certain aspects, the reinforcing element can comprise a plurality of filaments arranged in a braid configuration. In such aspects, the plurality of filaments can be disposed in a plurality of circumferential rows within the reinforcing jacket, wherein each of plurality has a sinusoidal form or any irregular form, or any combination thereof. In certain aspects, the braid or coil can be an expandable braid or coil.

In yet further aspects, the plurality of filaments can comprise stainless steel, nitinol, a polymer material, or a composite material. In certain unlimiting aspects, the filaments can comprise Nitinol and/or other shape memory alloys. In yet other unlimiting aspects, the filaments can comprise polyester or nylon. In yet some other exemplary aspects, the filaments can comprise spectra fiber, polyethylene fiber, aramid fiber, or combinations thereof.

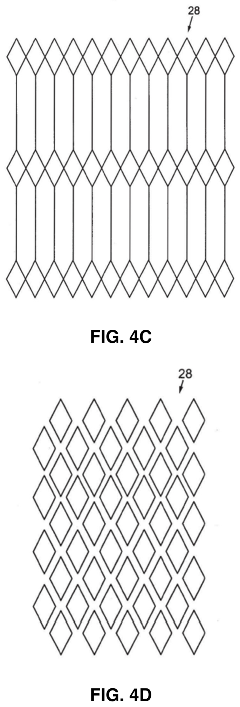

Some exemplary aspects of the braid or coil configurations are shown in **FIG. 4****.** In certain aspects, the braid or coil can be a generally thin, hollow, substantially cylindrical tube comprising an arrangement, pattern, structure, or configuration of filaments or struts, however other geometries can also be used. Suitable filaments can be round, having a diameter less than about 0.015", less than about 0.01", less than about 0.008", less than about 0.005", less than about 0.002", less than about 0.001", less than about 0.0008", or less than about 0.0005". In yet other aspects, suitable filaments can be round and having a diameter ranging from about 0.0005" inches thick to about 0.015" thick, including exemplary values of about 0.0006", about 0.0007", about 0.0008", about 0.0009", about 0.001", about 0.002", about 0.003", about 0.004", about 0.005", about 0.006", about 0.007", about 0.008", about 0.009", about 0.01", about 0.012", about 0.013", and about 0.014". In yet other aspects, the suitable filaments can be flat filaments having a height of less than about 0.006", less than about 0.005", less than about 0.004", less than about 0.003", less than about 0.001", less than about 0.0009", less than about 0.0008", less than about 0.0007", less than about 0.0006",and about 0.0005". In yet other aspects, the flat filaments can have a width from greater than about 0.003" to about 0.015", including exemplary values of about 0.004", about 0.005", about 0.006", about 0.007", about 0.008", about 0.009", about 0.01", about 0.012", about 0.013", and about 0.014". However, other geometries and sizes are also suitable for certain aspects.

In yet further aspects, the braid can have a per inch crosses (PIC) count of less than 50, less than 40, less than 30, less than 20, or less than 10. In yet other aspects, the braid can have the PIC count from 10 to 2, including exemplary values of 9, 8, 7, 6, 5, 4, and 3. In still further aspects, the PIC can vary along the longitudinal axis of the lumen. In yet other aspects, the braid pattern can vary along the longitudinal axis of the lumen. In the aspects where the braid or coil comprises filament that is nitinol, the nitinol is a heat-set at the expanded diameter *d_{e.}* In yet further aspects, where the filament comprises stainless steel or nitinol, the filament is configured to be atraumatic at least at the distal end of the sheath. **FIGS. 4A-D** illustrate partial elevation views of various structures for the braid or coil 28. It is understood that the structure of the braid or coil **28** can vary from section to section, changing along the length of the sheath. It is further understood that the structures shown in **FIGS. 4A-D**are not necessarily drawn to scale and show just exemplary and unlimiting aspects. It is further understood that the braid or coil is configured to provide the torquability of the sheath during the insertion of the prosthetic device.

Yet, in other aspects, the reinforcing element can comprise wires disposed in a plurality of circumferential rows embedded within the elastomeric material. In such aspects, the wires can have any shape that is configured to expand or contract. For example, the wires can have a sinusoidal or waveform. The phase and amplitude of the wire can vary depending on the desired application. Also, it is understood that the frequency and the total number of the circumferential rows present in the elastomeric material can also vary depending on the desired application. The expansion of the wires will allow the outer layer of the sheath to continue to expand until the reinforcing element present in the elastomeric material is tighten. In this configuration, the reinforcing jacket stops further expansion and thus maintains hemostasis.

In still further aspects, a tie layer can be disposed between the reinforcing jacket and the outer layer of the sheath.

An additional configuration of the reinforcing jacket is also disclosed. In this aspect, the outer layer does not extend to the proximal end of the sheath and wherein at least a portion of the inner liner at the proximal end of the sheath is substantially free of the outer layer. This proximal portion of the sheath that is not covered by the outer layer can be any length. In some aspects, this proximal portion is from about 5 to about 15 cm, including exemplary values of about 6 cm, about 7 cm, about 8 cm, about 9 cm, about 10 cm, about 11 cm, about 12 cm, about 13 cm, and about 14 cm.

A proximal portion of such an outer layer can be bonded to the inner liner to ensure that no spacing between the inner liner and the outer layer is present when the sheath is inserted into the patient's body. This bond can also prevent the outer layer from sliding off when the sheath is inserted into the patient's body. In such aspect, the reinforcing jacket having a proximal end and a distal end is positioned then over the at least a portion of the outer surface of the inner liner at the proximal end of the sheath that is substantially free of the outer layer. It is understood that in such configuration, the proximal end of the reinforcing jacket is abutting the proximal end of the sheath and is at least partially bonded to at least a portion of a proximal end of the outer surface of the inner liner.

Again, the reinforcing jacket can have a length substantially similar to a length of the proximal portion of the sheath that does not have an outer layer. In some exemplary and unlimiting aspects, the length of the reinforcing jacket can be from about 5 to about 15 cm, including exemplary values of about 6 cm, about 7 cm, about 8 cm, about 9 cm, about 10 cm, about 11 cm, about 12 cm, about 13 cm, and about 14 cm.

In still further aspects, the distal end of the reinforcing jacket is abutting or at least partially overlays a proximal portion of the outer layer. It is further understood that the distal end of the reinforcing jacket is seamlessly bonded to the at least a portion of the proximal portion of the outer surface of the outer layer. It is understood that the reinforcing jacket, as described in this aspect, can comprise all components of the reinforcing jacket disclosed above.

In still further aspects also disclosed the sheath having any one of the disclosed above configurations and comprising a ballooning guard. The balloon guard, similarly to the reinforcing jacket disclosed above, is configured to prevent excessive outer layer ballooning on the portion of the sheath that stays outside the patient's body while accommodating for the different insertion depths of the sheath and not affecting the force to expand the sheath. The ballooning guard is configured to stay outside of the patient's body and not to be inserted into the patient's anatomy.

In the aspects disclosed herein, the ballooning guard has a proximal end and a distal end and is disposed over at least a portion of the outer layer and wherein the ballooning guard is configured to remain outside of a subject's blood vessel and to substantially maintain hemostasis.

The ballooning guard, as described in the current disclosure, can be collapsible. In yet other aspects, the ballooning guard can be configured such that its length can be adjusted based on the insertion depth of the sheath.

In some aspects, the inner diameter of the ballooning guard can be big enough or have minimal resistance to expansion up to a certain diameter, such that it is not affecting the force to expand the sheath nor the force to advance the delivery system through the sheath. Once the ballooning guard reaches a certain diameter, the force to expand the ballooning guard increases significantly, such that it can resist the blood pressure and stop the ballooning. It is understood that while the ballooning guard may not prevent the initiation of the ballooning, it can contain it such that the outer layer will not over expand, burst and compromise hemostasis. Some exemplary schematic of the balloon guard is shown in **FIGS. 29E-F.** The ballooning guard **3125** is disposed over the outer layer **2908** such that while the blood **2915** maybe can enter into the portion between the outer layer **2908** and the inner layer **2902,** it does not cause a burst of the sheath and helps maintain hemostasis.

In still further aspects, the proximal end of the ballooning guard is connected to a most proximal portion of the outer layer and/or a hub of the sheath. While the distal end of the ballooning guard radially circumscribes at least a portion of the outer layer and wherein the distal end is not bonded to the outer layer. It is understood that the ballooning guards, as described herein, seal against a subject's skin and are not inserted into the subject's anatomy **2913.**

In still further aspects, the ballooning guard can comprise a braided or coiled sleeve comprising a plurality of filaments. Any of the disclosed above plurality of filaments can be utilized. In some aspects, where the braided or coiled sleeve is present, at least a portion of the plurality of the filaments at the distal end of the ballooning guard are coupled to each other to allow shortening of the braided or coiled sleeve and sealing against the outer layer and against the skin of the patient.

In still further aspects, the braided or coiled sleeve can also comprise a polymer. Any of the disclosed above elastomeric polymers can be used. In certain aspects, the braid or coil can be embedded with the polymer. It is understood that the braid or coil material can be any material known in the art. In certain aspects, the braid or coil material can comprise metal or metal alloys. In certain aspects, any known in the art metals or metal alloys used in medical devices can be utilized to make a braid or coil. In some aspects, the braid or coil can be made of a memory shape material. In still further aspects, the braid or coil can be any braid or coil as disclosed herein.

In yet other aspects, the braid material can comprise a polymer. It is understood that any known in the art polymers can be used to form a braid. In such exemplary and unlimiting aspects, the polymer can comprise any known polyolefin, any known polyamide, or any known polyester. In still further aspects, the braid material can comprise a fabric. In still further aspects, the braided sleeve can comprise a fabric. Yet, still, in further aspects, the ballooning guard can comprise the e-PTFE tubing, the ballooning guard comprises the e-PTFE tubing, corrugated tubing, or any polymeric tubing having a shape that is configured to be compressed. In certain aspects, the ballooning guard can comprise the e-PTFE tubing. In such exemplary and unlimiting aspects, the e-PTFE tubing can be compressed without it losing its shape. While in still further aspects, the ballooning guard can comprise a corrugated tubing. The corrugated tubing allows the guard to be compressed and to adjust its length.

In such exemplary and unlimiting aspects, the ballooning guard can be made of any polymeric material that can have a shape that allows this material to be compressed. For example, a tubing can be formed from any polymer known in the art. The wall of this tubing can be cut in a pattern that allows it to be compressed without losing the initial shape.

Additionally, some aspects of any of the sheath configurations disclosed herein can include an exterior hydrophilic coating on the outer surface of the outer layer. Such a hydrophilic coating can facilitate insertion of any of the sheaths disclosed herein into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other coatings (*e.g.*, PTFE, polyethylene, polyvinylidene fluoride), are also suitable for use with the sheath.

Also, soft tip **102,** as shown in **FIG. 11****,** can be utilized with any of the disclosed herein sheath configurations. In certain aspects, the tip can comprise low density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 A to about 40 A, including exemplary values of about 28 A, about 30 A, about 32 A, about 35 A, and about 38 A. It is further understood that Shore hardness can have any value between any two foregoing values. In yet other aspects, the soft tip **102** can have a Shore hardness from about 25 D to about 40 D, including exemplary values of about 28 D, about 30 D, about 32 D, about 35 D, and about 38 D. The tip portion **102** is configured to be radially expandable to allow a prosthetic device to pass through the distal opening of the sheath **100.**

As shown in **FIG. 11****,** the sheath **100** can optionally include at least one radiopaque filler or marker, such as a discontinuous or C-shaped, band **112** positioned near the distal end **104** of the sheath **100.** The marker **112** can be associated with the inner liner and/or outer layer **108, 110** of the sheath **100.** Such a radiopaque tip marker can comprise materials such as those suitable for the radiopaque filler, platinum, iridium, platinum/iridium alloys, stainless steel, other biocompatible metals, or combinations thereof. Suitable materials for use as a radiopaque filler or marker include, for example, barium sulfite, bismuth trioxide, titanium dioxide, bismuth subcarbonate, or combinations thereof. The radiopaque filler can be mixed with or embedded in the layer of the elastomeric polymer used to form the outer layer and can comprise from about 5% to about 45% by weight of the outer layer, including exemplary values of about 10%, about 15%, about 20%, about 25%, about 30%, about 35%, and about 40% by weight of the outer polymeric tubular layer. The more or less radiopaque material can be used in some aspects, depending on the particular application.

The disclosed herein sheath can be configured such that it locally expands at a particular location corresponding to the location of the medical device along the length of the lumen and then locally contracts once the medical device has passed that particular location. Thus, a bulge may be visible, traveling longitudinally along the length of the sheath as a medical device is introduced through the sheath, representing continuous local expansion and contraction as the device travels the length of the sheath. In some aspects, each segment of the sheath can locally contract after removal of any radial outward (insertion) force such that it regains the original resting diameter of lumen *dᵣ*.

In some aspects, each segment of the sheath can locally contract after removal of any radial outward force such that it at least partially returns to the original resting diameter of lumen *dᵣ*.

### METHODS

The aspects of the present disclosure also relate to a method of making a sheath having a proximal and a distal end and comprising: forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

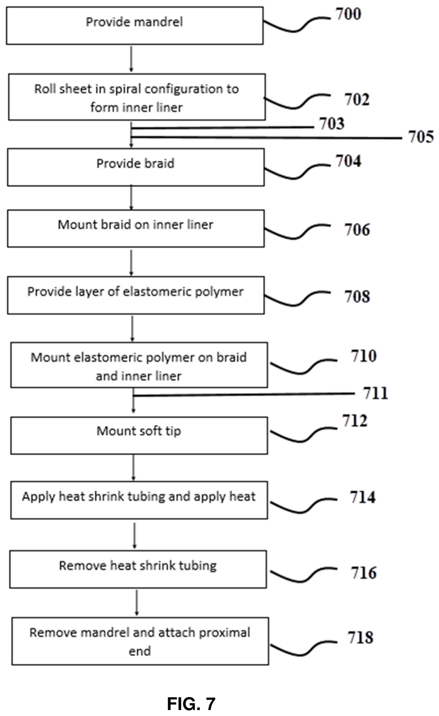

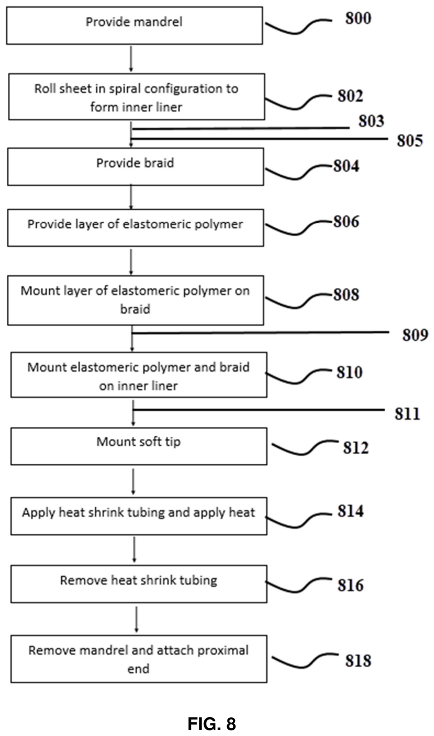

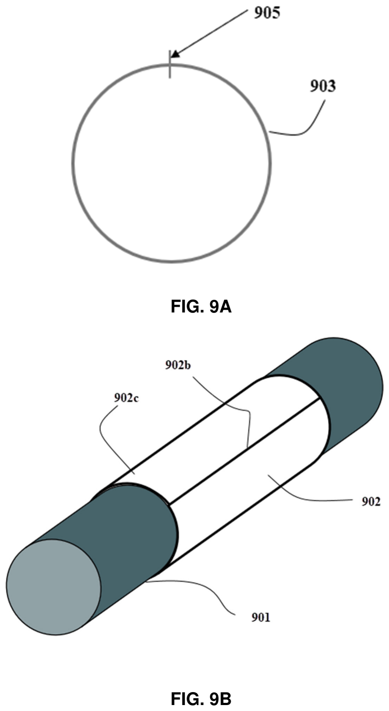

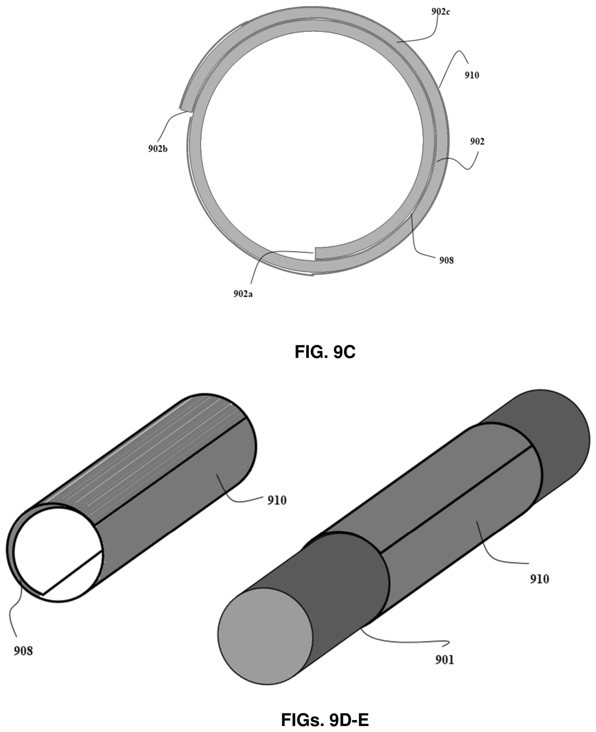

Various methods can be used to produce the sheaths discussed above and below throughout the present disclosure. For example, **FIGS. 7** and **8** exemplify block diagrams of exemplary methods of producing the sheath in various aspects. The various methods steps are also depicted in **FIG. 9A-9K****.** In certain aspects, and as shown in **FIG. 9A****,** the inner liner can be formed from an extruded tube **903** having an inner surface and outer surface and having any thickness that is described above. This extruded tube can be cut **905** along the length to form a sheet. In certain aspects, the inner surface and/or outer surface of the tube can be surface treated, such as, for example, by plasma etching, chemical etching, or other suitable methods of surface treatment. In some exemplary aspects, where the outer surface of the inner liner is treated, the treatment can provide for better bonding with the outer layer when formed. In yet other aspects, the inner surface of the inner liner can be ribbed. In such exemplary aspects, the ribbed surface facilitates a reduction of contact points with the prosthetic device and can reduce friction. In still further aspects, the initial extruded tube **903** can be produced by co-extrusion with multiple layers of the same or different polymers as described herein. It is understood that one of ordinary skills in the art can choose the composition of the inner liner depending on the desired application. In certain aspects, the decision to use a specific material for the inner liner can be dependent on the desired stiffness, wall-thickness, and lubricious optimization.

In still further aspects, one or more mandrels can be provided (step **700** or **800 in** **FIG. 7** and **8****,** respectively). The mandrel can be provided with an exterior coating, such as a Teflon^{®} coating, and the mandrel's diameter can be predetermined based on the desired rest diameter *dᵣ* of the resulting sheath. As shown in **FIG. 9B****,** the sheet formed by cutting **905** the extruded tube **903** can be rolled in a spiral configuration (step **702** and **802 in** **FIGS.** 7 and **8** respectively) around the mandrel **901** to form the inner liner **902** such that at least a portion of the inner surface of the sheet overlays at least a portion of the outer surface of the sheet thereby forming an overlying portion **902c** and wherein the first edge (not shown) of the sheet is slidable along at least a portion the inner surface of the sheet and the second edge **902b** is slidable along at least a portion of the outer surface of the sheet.

In still further exemplary aspects, in steps **705** and **805 (****FIGS. 7** and **8** respectively), an amount of a first lubricant **910 (****FIGS. 9C-9E****)** can be optionally applied on the outer surface of the inner liner. The presence of this lubricant material can reduce the friction between the inner liner and the outer layer of the final sheath. In yet other aspects, in steps **703** and **803,** an amount of a second lubricant **908** can be applied between the overlaying and sliding portions of the inner liner to further improve slidability and decrease friction. **(****FIG. 9D** depicts the inner liner with the two optional lubricants present with the mandrel hidden from the view). In still further aspects, it is understood that the inner liner formed with the use of mandrel can have any rest diameter, as described above. In certain aspects, the rest diameter dᵣ is substantially uniform along the longitudinal axis of the lumen. While in the other aspects, the rest diameter *dᵣ* varies along the longitudinal axis of the lumen and wherein the rest diameter *dᵣ* at the proximal end that is larger than the rest diameter *dᵣ* at the distal end.

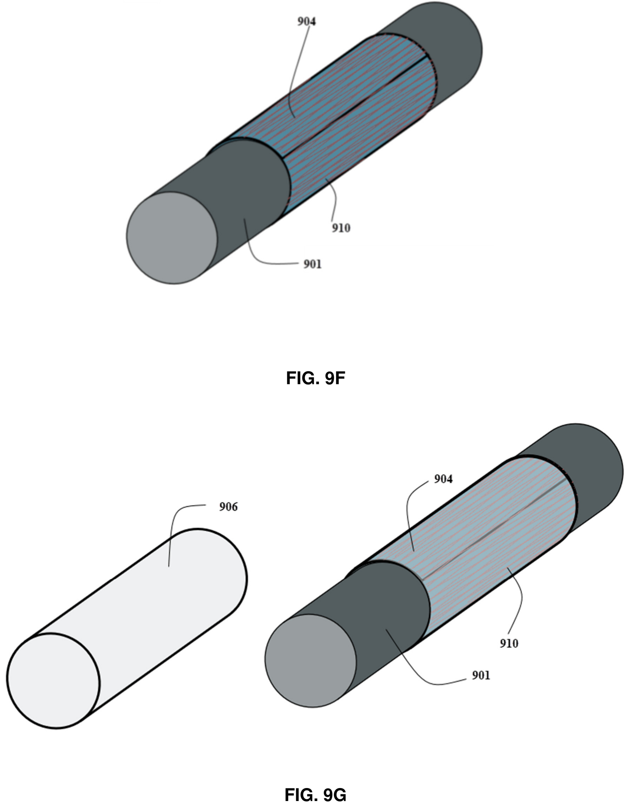

In still further aspects, the methods can further comprise a step of providing a braid or coil (steps **704** and **804).** It is understood that any of the described above braid or coil can be used in this step. In still further aspects, and as shown in step **706** of **FIG. 7****,** the braid or coil is mounted on the inner liner. In some exemplary aspects, and as shown in **FIG. 9F** the braid or coil **904** can be mounted on the first lubricant **910** that can be present on the outer surface of the inner liner. It is understood that in some aspects, the second lubricant can be present only at a portion of the outer surface of the inner liner. In yet other aspects, the disclosed sheath can have segments where the first lubricant is present and the braid or coil is mounted over it, while it can have other segments where the second lubricant is not present and the braid or coil is mounted directly on the outer surface of the inner liner. It is understood that the location of these specific segments can be determined by one of ordinary skill in the art depending on the desired application. It is understood that the mounting of the braid or coil can be done by any known in the art methods. In some unlimiting aspects, the braid or coil can be provided as a cylindrical tube, and it can be slid on top of the inner liner or the first lubricant if it is present.

In yet further aspects and as shown in step **708,** the method can further comprise a step of providing a layer of the elastomeric polymer. It is understood that any of the disclosed above elastomeric polymers can be used. The specific polymer can be chosen based on the desired properties of the disclosed sheath, such as, for example, level of stiffness, hemostasis, and the like. The layer of the elastomeric polymer can be provided in any form known in the art. In certain and unlimiting aspects, the elastomeric polymer can be provided as a cylindrical tube **906 (****FIG. 9G****).** In a still further aspect, the elastomeric polymer can be mounter on the inner liner and the braid or coil (step **710**). **FIG. 9G****,** for example, depicts an aspect where the cylindrical tube of the elastomeric polymer **906** is used to slide on the inner liner having a first lubricant **910** overlaying the inner liner's outer surface and the braid or coil **904.**

In yet further aspects, the disclosed method can comprise a step of embedding (step **711,** **FIG. 7****)** the braid or coil into the layer of the elastomeric polymer. It is understood that the sheath can comprise various segments. In some aspects, some of the segments can comprise the braid or coil embedded within the layer of the elastomeric polymer, while in other segments, the braid or coil and the layer of the elastomeric polymer are separate. It is further understood that in some aspects, the sheath can have a braid or coil embedded within the elastomeric polymer over the whole length of the sheath, while in other aspects, the braid or coil is not embedded within the elastomeric polymer over the whole length of the sheath. It is further understood that any methods known in the art can be used to embed the braid or coil within the elastomeric polymer. In some aspects, the application of heat can be utilized. In certain aspects, the use of heat shrink tubing can be utilized to embed the braid or coil within the elastomeric polymer. It is understood that after the step of embedment is complete, the heat shrink tubing is removed. In yet other aspects, the braid or coil can be embedded within the layer of the elastomeric polymer by placing the assembly in an oven or otherwise heating it.

In still further aspects, a soft, atraumatic tip can be provided at the distal end of the resulting sheath (step **712).** In yet further aspects, the outer layer comprising the braid or coil and the layer of the elastomeric polymer is at least partially bonded to the inner liner. It is understood that this bonding can also be achieved by any known in the art methods. In certain aspects, and as shown in step **714,** a heat shrink is applied to the portion that is being bound and heated to form a bonding between the inner liner and the outer layer. In yet other aspects, the bonding between the inner liner and the outer layer can be achieved by placing the assembly in an oven or otherwise heating it. In still further aspects, the bonding is performed by heating at a temperature from about 350 °F to about 550 °F for a time period effective to form a bond between at least a portion of the outer layer and at least a portion of the inner liner. In yet further aspects, the heating can be done at a temperature of about 375 °F, about 400 °F, about 425 °F, about 450 °F, about 475 °F, about 500 °F, or about 525 °F. In yet other aspects, the time period effective to form a bond can comprise from about 1 second to about 60 seconds, including exemplary values of about 5 seconds, about 10 seconds, about 15 seconds, about 20 seconds, about 25 seconds, about 30 seconds, about 35 seconds, about 40 seconds, about 45 seconds, about 50 seconds, and about 55 seconds. However, it is further understood that this time period is not limiting, and it can have any value needed to provide for an effective bond, for example, it can have any value from about 1 second to about 5 hours. It is further understood that if the heat shrink tubing is used to obtain the desired bonding, the heat shrink tubing is removed (step **716,** **FIG. 7****).**

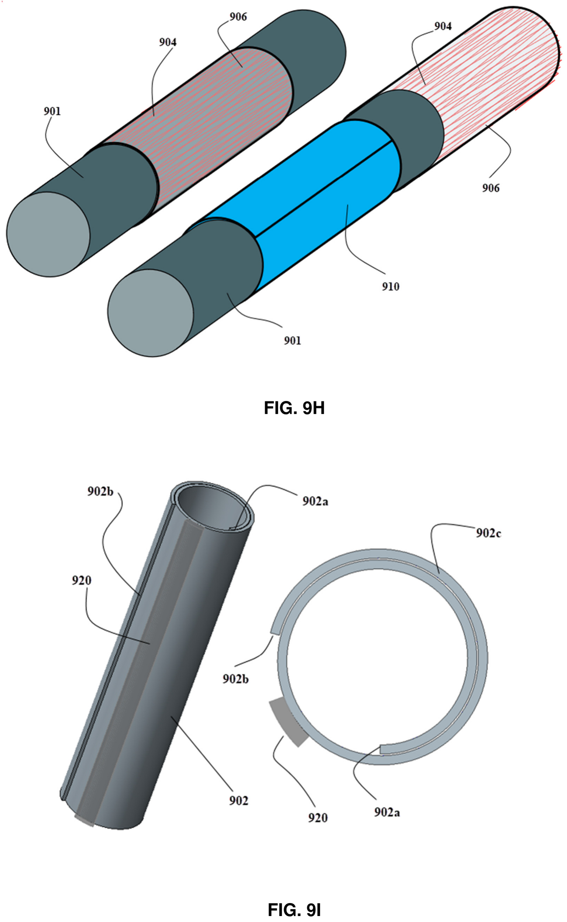

In still further aspects and as shown in **FIG. 9I****,** the bonding step can also comprise a first strip of the elastomeric polymer **920** to be applied along at least a portion of the longitudinal axis of the lumen to at least a portion of the outer surface of the sheet that does not comprise the overlaying portion prior **902c** to or during the step of bonding the at least a portion of the inner surface of the layer of the elastomeric polymer to at least a portion of the outer surface of the sheet of the inner liner. It is understood that in some exemplary aspects and as shown in the **FIG. 9I****,** this first strip can be applied prior to mounting the braid or coil. In still further aspects, the location where the first strip is applied does not comprise a first lubricant. However, it is understood that in such aspects, the first lubricant can be present in other locations.

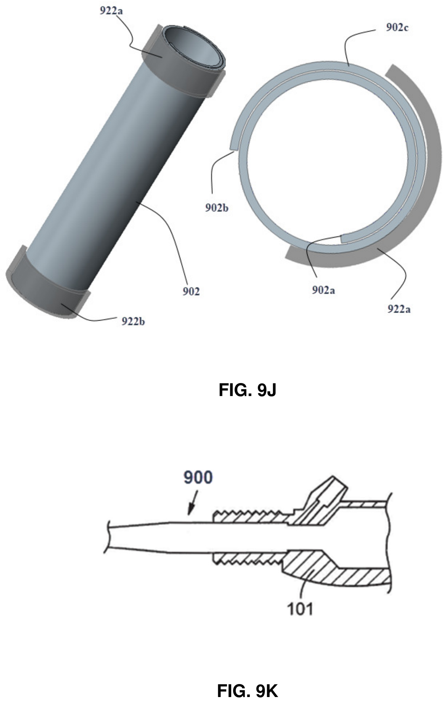

In still further exemplary aspects, the methods can comprise a second strip **922a** of the elastomeric polymer that can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to mounting the braid or coil. In still further aspects, the location where the second strip is applied does not comprise a first lubricant. However, it is understood that in such aspects, the first lubricant can be present in other locations. In yet other aspects, the method can comprise a third strip of the elastomeric polymer **922b** that can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to mounting the braid or coil. In still further aspects, the location where the third strip is applied does not comprise a first lubricant. However, it is understood that in such aspects, the first lubricant can be present in other locations. In still further aspects, both the second and the third elastomeric polymers are present. While in other aspects, only one of the second or the third elastomeric polymers is present. It is further understood that the first, second, and third elastomeric polymers can be the same or different. It is also understood that the first, second, and third elastomeric polymers can be the same as the layer of the elastomeric polymer present in the outer layer and can comprise any of the elastomeric polymers described herein.

**FIG. 9K** illustrates an elevation view of the sheath at step **718 of** **FIG. 7****.** The sheath **900,** made according to described methods and processes, can be attached or bonded to a housing **101,** such as by bonding the proximal end of the sheath **900** to the polycarbonate housing **101.**

Some alternative aspects are shown in **FIG. 8** and **FIG. 9H****.** In such alternative aspects, the outer layer is pre-formed and then mounted on the inner liner positioned on the mandrel. In such aspects, the provided layer of the elastomeric polymer is first mounted on the braid or coil (step **808)** prior to mounting it on the inner liner. In yet other aspects, the method can also comprise a step of partially embedding the braid or coil within the layer of the elastomeric polymer before mounting both of them on the inner liner (step **809).** However, the step of partially embedding the braid or coil with the layer of the elastomeric polymer can be done after the braid or coil, and the layer of the elastomeric polymer is mounted on the inner liner (step **811**). Steps **812-818** can be performed analogously to steps **712-718.**

In still further aspects, and as shown in **FIG. 9I****,** the bonding step can also comprise a first strip of the elastomeric polymer **920** to be applied along at least a portion of the longitudinal axis of the lumen to at least a portion of the outer surface of the sheet that does not comprise the overlaying portion prior **902c** to or during the step of bonding the at least a portion of the inner surface of the layer of the elastomeric polymer to at least a portion of the outer surface of the sheet of the inner liner. It is understood that in some exemplary aspects and as shown in the **FIG. 9I****,** this first strip can be applied prior to the mounting pre-formed outer layer comprising the braid or coil and the elastomeric polymer. In still further aspects, the location where the first strip is applied does not comprise a first lubricant. However, it is understood that in such aspects, the first lubricant can be present in other locations.

It is also understood that these alternative aspects can also include a step where a second strip **922a** of the elastomeric polymer that can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to the mounting pre-formed outer layer comprising the braid or coil and the elastomeric polymer. In still further aspects, the location where the second strip is applied does not comprise a first lubricant. However, it is understood that in such aspects, the first lubricant can be present in other locations. In yet other aspects, these methods can also comprise a third strip of the elastomeric polymer **922b** that can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this third strip can be applied prior to the mounting pre-formed outer layer comprising the braid or coil and the elastomeric polymer. In still further aspects, the location where the third strip is applied does not comprise a first lubricant. However, it is understood that in such aspects, the first lubricant can be present in other locations. In still further aspects, both the second and the third elastomeric polymers are present.

Also disclosed herein are the methods of making additional configurations of the sheath. For example, disclosed herein is a method of making a sheath having a proximal and a distal end. Such methods comprising forming a variable diameter inner liner by providing an elongated single lumen tubing comprising at least one polymer layer; longitudinally cutting at least a portion of a circumference of the elongated single lumen tubing to form a sheet having a first longitudinal edge and an opposite second longitudinal edge and having an inner surface and an outer surface; and then forming a variable diameter inner liner by rolling the sheet in a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis.

It is understood that the elongated single lumen tubing can be extruded or coextruded from any polymers or compounds disclosed above. For example, and without limitation, the elongated single lumen tubing can comprise at least one polymer comprising a polyolefin, a polyamide, a fluoropolymer, copolymers thereof, co-extrudates thereof, or blends thereof. Yet, in other aspects, a compound material comprising a polyolefin and a lubricious filler. In such exemplary aspects, the polyolefin can be high-density polyethylene. Yet, in other aspects, the lubricous filler can comprise a polytetrafluoroethylene (PTFE) filler. In such aspects, the lubricious filler can be present in an amount from about 5 wt % to about 20 wt % of a total weight of the compound material.

For example, and without limitations, a tubular body can be extruded to form an elongated tubing comprising a compound material. This compound material can comprise a polyolefin present in an amount from greater than 0 wt% to less than 100 wt % based on a total weight of the compound and a lubricious filler present in an amount from about 5 wt % to about 20 wt% of a total weight to the compound material.

The extruded or coextruded elongated single lumen tubing can a coefficient of friction less than about 0.5.

In still further aspects, the single lumen tubing can be produced by co-extrusion with multiple layers of the same or different polymers as described herein. In yet other aspects and as disclosed above, this tubing can be coextruded with any of the disclosed above tie layers. In such exemplary aspects, where for example, the tie layer is present, the elongated tubing can comprise any of the disclosed above polymers, and the tie layer disposed on the inner surface of the tubing or/and an outer surface of the tubing.

It is understood that this elongated tubing is used to form the inner liner, and any of the disclosed above materials can be used. In still further aspects prior to cutting the tubing to form the spiral configuration of the inner liner, a lubricious liner can be disposed on the inner surface of the tubing and/or the outer surface of the tubing. It is understood that this lubricious liner can be disposed on the tie layer if it is present. In certain aspects, the tie layer is used to bond the lubricious liner with the polymer layer forming the elongated tubing. In yet further aspects, at least a portion of the inner surface of the elongated tubing can be ribbed. In such exemplary aspects, the ribbed surface facilitates a reduction of contact points with the prosthetic device and can reduce friction.

Yet, in other aspects, where the tie layer and/or lubricious liners are present, at least a portion of the lubricious liner can also be ribbed. Also, in such aspects, the tubing comprising any of the disclosed above polymers, the tie layer and the lubricious liner has a coefficient of friction less than about 0.5.

The elongated tubing is then positioned on a mandrel and cut at at least a portion of the circumference of the tubing, as disclosed above. In still further aspects, the methods of forming the inner liner are similar to those shown in **FIG. 9A****.** The inner liner can be formed from a single lumen extruded tube **903,** having an inner surface and outer surface and having any thickness that is described above. This extruded tube can be cut **905** along the length to form a sheet.

Additionally, in certain aspects, the inner surface and/or outer surface of any of the disclosed above single lumen tubing can further be surface treated, such as, for example, by plasma etching, chemical etching, or other suitable methods of surface treatment. In some exemplary aspects, where the outer surface of the inner liner is treated, the treatment can provide for better bonding with the outer layer when formed. It is understood that one of ordinary skills in the art can choose the composition of the inner liner depending on the desired application. In certain aspects, the decision to use a specific material for the inner liner can be dependent on the desired stiffness, wall-thickness, and lubricious optimization.

The formed sheet can then be positioned on an additional mandrel. Such a mandrel can be provided with an exterior coating, such as a Teflon^{®} coating, and the mandrel's diameter can be predetermined based on the desired rest diameter *dᵣ* of the resulting sheath. As shown in **FIG. 9B****,** the sheet formed by cutting **905** the extruded tube **903** can be rolled in a spiral configuration around the mandrel **901** to form the inner liner **902** such that at least a portion of the inner surface of the sheet overlays at least a portion of the outer surface of the sheet thereby forming an overlying portion **902c** and wherein the first edge (not shown) of the sheet is slidable along at least a portion the inner surface of the sheet and the second edge **902b** is slidable along at least a portion of the outer surface of the sheet. It is understood that any of the disclosed above spiral configurations can be obtained.

For example, the sheet is rolled such that the first and the second edges of the sheath are substantially aligned in a spaced relationship along a vertical axis, passing through a thickness of the sheath. In such aspects, the spaced relationship can comprise a portion of the sheet positioned between the first edge and the second edge along the vertical axis. In certain aspects, the sheet is rolled such that when the sheath is in an unexpanded rest state, the inner liner comprises at least two layers of the sheet overlaying each other along at least a portion of a sheath's circumference. While in other aspects, when the sheath is in an unexpanded rest state the at least a portion of the sheath's circumference comprises three layers of the sheet overlaying each other. Also disclosed are aspects that when the sheath is rolled into the spiral configuration, the first edge of the sheet can be substantially aligned with a vertical axis passing through a thickness of the sheath and the second edge circumferentially offset from the vertical axis. In such configuration, in some aspects, at at least a portion of a circumference of the sheath, the inner liner comprises one layer of the sheet without any overlaying portion.

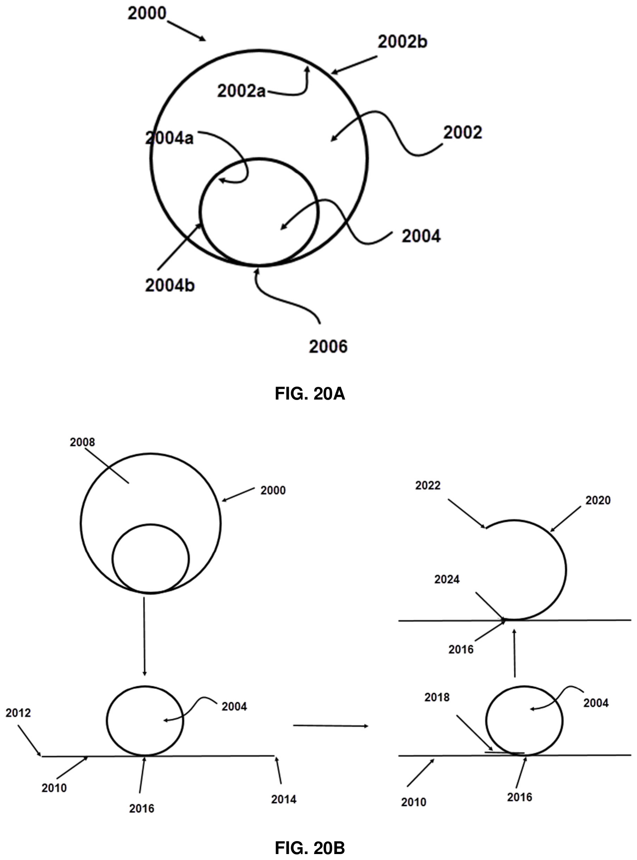

In certain aspects, however, the inner liner can be formed from an extruded double lumen tubing. The extruded double lumen tubing can comprise any of the disclosed above polymers or compounds. Exemplary schematic of the methods of making the inner liner from such double lumen tubing is shown in **FIGS. 20A-C.** The double lumen **2000** can comprise a first channel **2002** having an inner surface **2002a** and an outer surface **2002b** and a second channel **2004** having an inner surface **2004a** and an outer surface **2004b.** The second channel **2004** is positioned within the first channel **2002** such that at least a portion of a circumference of the first channel and at least a portion of a circumference of the second channel have at least one shared inner surface and at least one shared outer surface **2006.** In such configuration, the outer surface **2002b** of the first channel **2002** defines an outer surface of the double-lumen tubing.

It is understood this double lumen tubing can comprise any of the disclosed above layers.

For example, the double-lumen tubing can be coextruded from the compound material described above. In such exemplary aspects, the first channel can comprise the compound material, or the second channel, or both can comprise the described above compound material.

In yet further aspects, the double-lumen tubing can be coextruded with any of the disclosed above tie layers, where the tie layer can be coextruded with the first channel or the second channel or both. In still further aspects, any of the disclosed above lubricious liners can be disposed at the tie layer. It can be disposed, for example, within the first channel, or the second channel, or both.

In still further aspects, and as shown in **FIG. 20B****,** the first channel can be cut longitudinally along at least a portion of the first channel circumference 2008 that is not shared with the circumference of the second channel to form a first sheet. The first sheet **2010** has a first edge **2012** and a second edge **2014,** and the second channel disposed longitudinally along at least a portion of the first sheet **2004,** such that at least a portion of the circumference of the second channel has at least a portion that has a shared surface **2016** with the first sheet along a length of the sheet and along a length of the second channel.

The second channel is then longitudinally cut at a portion of the circumference of the second channel **2018** that abuts the shared surface **2016** with the first sheet to form a second sheet **2020** having a first edge **2022** and a second edge **2024,** wherein the second edge is defined by a portion of the shared surface with the first sheet **2016.**

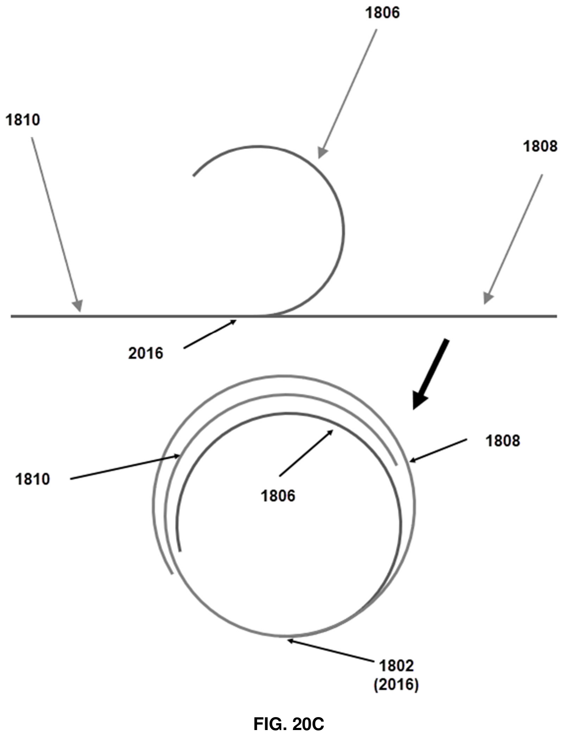

In still further aspects, the first and the second sheets are rolled into a spiral configuration, as shown in **FIG. 20C****,** such that: the shared surface **2016** between the second and the first sheets form a first portion **1802** (**2016**) of an inner liner having a first surface and an opposite second surface. At least a portion of the second sheet **1806** forms a first segment of an inner liner having a first surface and an opposite second surface. A portion **1810** of the first sheet adjacent to the second end of the second sheet and extending to the first end of the first sheet forms a second segment **1810** of an inner liner having a first surface and an opposite second surface. While a portion of the first sheet adjacent the second end **1808** of the second sheet and extending to the second end of the first sheet forms a third segment 1808 of an inner liner having a first surface and an opposite second surface. In such a spiral configuration and as shown in **FIG. 20C** and **FIG 18****,** at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment; wherein the first surface of the first portion extends into the first surface of the first segment, the second segment and the third segment, and wherein the second surface of the first portion extends into the second surface of the second and the third segments.

In certain aspects, whether the inner liner is formed from the single lumen tubing or from the double-lumen tubing, prior to the step of cutting, the methods can comprise the step of disposing an amount of lubricant. It is understood that the lubricant can be disposed at any surface of any channel.

In still further aspects, the lubricant can be disposed manually. While in other aspects, the lubricant can be disposed by pad printing. Yet still, in other aspects, the lubricant can be disposed by spraying.

It is understood that any of the disclosed above lubricants can be used.

When the lubricant is disposed manually, any known in the art techniques can be utilized. For example, and without limitations, the lubricant can be disposed with a brush, cloth, pad, and the like.

In some aspects, the lubricant is disposed by pad-printing. During pad printing, an amount of lubricant is placed in a recessed channel on a plate. The length and width of the channel can be predetermined as desired. For example, the length and width of the channel can correspond to the desired area on a surface of the inner liner where the lubricant is placed. The volume of the channel can also be defined, partially determining the total amount of lubricant to be transferred. In such aspects, a soft pad can be dipped in the channel, picking up the lubricant, and moved over the desired portion of the inner liner. The pad is then can be stamped onto the predetermined portion of the liner, transferring the lubricant onto the surface of the inner liner.

It is understood that in the aspects disclosed herein, the predetermined amount of lubricant is applied to a set location (predetermined portion) of the inner liner's surface. In such aspects, the methods disclosed herein allow a substantial control of both the application area and the volume of lubricant used. As disclosed above, since the depth of the channel can be controlled, an amount of the lubricant disposed on the surface of the inner liner can also be substantially controlled.

In aspects where the pad-printing is used to transfer the lubricant on the surface of the inner liner, any of the disclosed above lubricants can be utilized. In certain aspects, the lubricant used for the pad-printing methods is substantially viscous. In certain exemplary and unlimiting aspects, the lubricant can have a viscosity from about 600 cP to about 1,200 cP. In yet further aspects, it is understood that the viscosity of the lubricant can be adjusted by adding more solids or more solvents to the lubricant composition. For example, and without limitation, the lubricant such asMED10-6670 (or similar) can be thickened beyond its' standard viscosity.

It is understood that the use of pad-printing allows the lubricant to be applied to a very specific portion of the inner liner with tight tolerance. In certain aspects, the lubricant can comprise a fluorescent material or any other material that would allow it to determine its specific location on the device. In such aspects, for example, a specific lubricant location can be determined under UV light. It can allow easy quality control to determine whether the lubricant has been applied in the desired portion of the sheath.

Yet, in other aspects, the substantially precise pad printing can help reduce push forces by decreasing frictional force in the most relevant area of the sheath while at the same time preventing undesired migration of the lubricant.

In certain aspects, the lubricant can be applied around a portion of the circumference of the inner liner. Yet, in other aspects, the lubricant can be applied around a whole circumference of the outer surface of the inner liner

In yet other aspects, the lubricant can be transferred in a predetermined pattern. For example, it can be transferred in the "stripe" pattern. However, it is understood that such a pattern is only exemplary. The pattern can have any regular or irregular shape. For example, if the pattern is the stripe pattern, lubricant can be disposed in a plurality of stripes along a whole length of the inner liner or along a portion of the inner liner. In still further aspects, the pattern can have a triangular shape, tapered shape, oval shape, circular shape, rectangular shape, or any irregular shape. The pattern can be determined based on the desired application and/or the desired location on the outer surface of the inner liner.

In still further aspects, the inner liner can be rotated by the pad printing machine in between runs so that the printer can make lines (or any other desired shapes) of the lubricant in a new location along the surface of the inner liner. In addition to precisely controlling the amount of lubrication added to the inner liner and the lubricant location, this method allows the manufacturing of the sheath to be more cost-effective by reducing the amount of lubricant and reducing the number of a sheath that could be discarded due to inaccurate application of the lubricant that can affect the overall sheath performance.

In still further aspects, the lubricant can be applied by spray coating. In such methods, the lubricant is loaded into an atomizing machine. The sheath or, more specifically, the inner liner is mounted on a mandrel that able to rotate about the long axis of the sheath. The lubricant is then sprayed onto the rotating sheath and translated down of a desired portion of the inner liner length, ensuring substantially uniform coverage. The amount of the lubricant to be sprayed onto the inner liner, the speed at which the nozzle moves along the sheath, and the sheath rotation speed can all be specified to optimize this application process.

In certain aspects, any of the disclosed above lubricants can be used. In aspects where the spray coating is utilized, the viscosity of the lubricant is equal to or less than 600 cP. Spray coating uses equipment that atomizes a liquid solution input to the spray coating machine. The resulting spray can be used to coat various devices. In the case of the expandable sheath device, the lubricant is mixed and fully prepared, then loaded into the machine.

To apply the lubricant in a substantially uniform manner onto the inner liner, the inner liner is mounted on a mandrel that is configured to rotate along the long axis of the sheath at a specified speed. The nozzle of the coating machine then sprays out atomized droplets of the lubricant solution onto the inner liner. The nozzle translates, moving horizontally along the length of the sheath as it rotates. Speeds can be adjusted to optimize the amount of lubricant sprayed onto the inner liner. It is understood that if more lubricant is needed, the speeds can be slowed, so the nozzle stays at a given location along the surface of the inner liner for a longer period and vice versa. As with pad printing, the lubrication can be comprised of a fluorescing component, so under blacklight, it is clearly visible to see the areas of the sheath coated with lubrication.

In still further aspects, after application of the lubricant on the surface of the inner liner, the lubricant is cured. It is understood that the curing can be done at any condition effective to provide the desired result. In certain exemplary and unlimiting aspects, the curing is performed in an oven. Curing temperature and timing can be defined by the specific lubricant used in the methods disclosed above.

In still further aspects, the methods can further comprise disposing an outer layer over at least a portion of the outer layer of the inner liner to form the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

In certain aspects, the outer layer can be made by extruding a tubular body to form an elongated tube comprising a first polymer layer, wherein the first polymer layer comprises a first compound composition comprising from greater than 0 wt% to less than 100 wt% of a polymer comprising a polyether block amide, a polyurethane, or a combination thereof; less than about 65 wt% of an inorganic filler based on a total weight of the first compound composition; and up to about 20 wt% of a solid lubricant filler based on a total weight of the first compound composition. This elongated tube can then be disposed on any of the disclosed above inner liners to form the outer layer of the sheath. It is understood that any of the disclosed above additional layers can also be present between the inner liner and the outer layer.

In yet other aspects, the outer layer as presented herein can be made by coextruding an elongated bump tubing comprising a first polymer layer and a second polymer layer; wherein the first polymer layer comprises: a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; the second polymer layer comprises a polyurethane wherein the first polymer layer defines an inner surface of the tubing and the second polymer layer defines an outer surface of the tubing; and then disposing such a tubing on the surface of any one of the disclosed herein inner liners. It is understood that any of the disclosed above additional layers can also be present between the inner liner and the outer layer.

In still further aspects, methods also comprise a) forming an inner liner by any of the disclosed above methods and then b) disposing a first polymer layer comprising a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; wherein the first polymer layer is disposed over a proximal portion of the inner liner and has a length from about 5 cm to about 15 cm; c) disposing a second polymer layer comprising polyurethane over the first polymer layer, wherein the second polymer layer extends along a length of the sheath; wherein the first polymer layer and the second polymer layer together form an outer layer of the sheath.

It is understood that any known in the art methods can be utilized to form any of the disclosed herein compositions. In certain aspects, the components that are present in any of the disclosed herein elongated tubes are provided to form a compound. The compound is then mixed to form a substantially homogeneous mixture. Yet, in other aspects, the mixture is homogeneous. In still further aspects, the mixture is extruded to form an elongated tube having a first polymer layer. The formed first polymer layer can comprise any (and in any combination) of the compositions and characteristics disclosed above.

In yet further aspects, the methods also comprise steps of forming the elongated tube comprising two or more layers, as disclosed above. In such aspects, for example, when the elongated tube comprises any of the disclosed above first polymer and the second polymer layers, such layers can be coextruded to form the elongated tube as disclosed. Any of the known in the art extrusion devices can be used to obtain any of the desired elongated tubes.

Also disclosed herein are methods of bonding the outer layer and inner liner if desired. It is understood that any known in the art methods can be used to form a bond. In certain aspects, heat treatment can be utilized. For example, the sheath can be inserted into a heat-shrink tubing and heated together to a temperature that would allow at least a partial bonding between the inner liner and the outer layer.

Some additional exemplary aspects can include laser welding, compression head welding or ultrasonic welding, as shown in **FIG. 28A-C.**

In yet other aspects, laser welding can be utilized. In such aspects, an inner liner can be formed by any of the methods disclosed above. The outer layer comprising any of the disclosed above compositions and formed by any of the disclosed above methods is then disposed over the inner liner to form the sheath. It is also understood that any of the disclosed above layers can also be presented between the inner liner and the outer surface. The sheath is then positioned on a mandrel configured to rotate. Laser welding uses a focused laser to heat a part in a selected location. The mandrel is aligned with a laser beam configured to move along a longitudinal axis of the sheath to a predetermined distance at conditions effective to form the bond (**FIG. 28A**). In such aspects, the head of the laser bonder is positioned directly above the center of the sheath, and the sheath can be rotated to align the laser with any desired point on the diameter of the sheath. The laser bonder then moves horizontally across the sheath.

A bond can be formed at a predetermined portion of the sheath between at least a portion of an outermost surface of the inner liner and at least a portion of an innermost surface of the outer layer. It is understood, however, while other additional layers such as lubricants or tie layers can be present, the predetermined location where the bond is formed is substantially free of a lubricant and/or a tie layer.

It is understood that settings on the laser welder can be changed to optimize both the level of heating and bonding area on the device. Laser power in terms of wattage changes the heat that is imparted onto the sheath, while the feed rate adjusts the length of time the laser is focused on a given portion of the sheath. Focus position, weld start angle, and welding distance can be used to control the area of the laser, are fine-tuned to create a bond of the correct size.

Also disclosed herein are additional methods of forming a bond. For example, the use of a compression head bonder (**FIG. 28B**). In such methods, the inner and outer layers are formed by any methods disclosed above and to assemble to form a sheath. The sheath is then positioned onto a mandrel into a radial compression head bonder. Any known in the art compression head bonders can be used. In certain aspects, the compression head bonder can comprise a collapsible aperture configured to compress the sheath to a predetermined diameter. The radial compression head bonder comprises a plurality of dies, wherein at least one of a plurality of dies can be heated to form the bond at the preterminal portion of the sheath.

An exemplary and unlimiting bonder, as shown in **FIG. 28B** can, for example, comprise a total of nine separate dies, eight made from PEEK plastic, for example, and one metallic die. The metallic die can be heated before use. The machine brings the jaws of the aperture together to a smaller diameter, then compresses around a device inserted through the block. The heated die then melts the portion of the inserted component that comes in contact with it. To use with an expandable sheath, a mandrel is inserted into the lumen of the device to prevent compression of the sheath shaft while under the load of the bonder. In certain aspects, fluorinated ethylene propylene can be placed over the desired bonding area of the sheath to transfer the heat evenly, preventing the metal from directly burning the outer layer. The sheath can be supported in the machine using a separate channel to the side. In certain aspects, the width of the bonding dies can be less than the length of the sheath, so to lengthen the bonding area, the sheath can be moved horizontally after each run, and the bonding can be repeated for the desired length along the sheath.

It is understood that the specific bond and its location can be controlled as desired. For example, the temperature of the metal die can be determined to ensure that it is set to a temperature that effective to at least partially melt both the outer layer and inner liner components. In yet further aspects, the compressive force exerted onto the part can also be adjusted such that at least partial melting is obtained without any substantial damage to the remaining components of the sheath. A final diameter the aperture reaches while bonding can also be predetermined. It is understood that the larger the diameter, the more exposure the metal die has to the part, and the larger the bonding area can be obtained. Still further, a length of time the dies are compressed onto the sheath can also impact the degree of melting of the components and the strength of the bond.

In still further aspects, the methods can comprise ultrasonic welding. In such aspects, the sheath components to be bonded are placed on the welding machine, and then a load is applied from a moveable horn. The horn moves at an extremely fast rate, exerting a high amount of vibrational energy. This energy is absorbed by the materials, which can melt in predetermined locations. The melted materials can flow together, achieving a bond when cooled. In some aspects, ultrasonic welding can be used prior to forming a fully assembled sheath. In such aspects, for example, ultrasonic welding can be used to bond any desired parts at any step of the manufacturing.

Also disclosed are methods of making a sheath comprising a reinforcing jacket. In such methods, the sheath components, such as the inner liner and the outer layer, are formed by any of the disclosed above methods and assembled together to form a sheath. Then a reinforcing jacket having a proximal end and a distal end is disposed such that it overlayer at least a portion of the outer layer. The methods further comprise substantially seamlessly bonding the distal end of the reinforcing jacket to at least a portion of the outer layer. It is understood that any of the disclosed above or generally known in the art bonding methods can be used.

**The** reinforcing jacket can comprise any of the disclosed above components, wherein the reinforcing jacket comprises any of the disclosed above elastomers and any of the disclosed above reinforcing elements.

In still further aspects, the reinforcing jacket can be formed by any methods known in the art. For example, the reinforcing jacket can be formed by injection molding, extruding or reflow process. In still further aspects, any of the disclosed herein reinforcing members can be embedded in a soft polymer to form the reinforcing jacket. For example, and without limitation, during the reflow process, a reinforcing element can be disposed on top of the polymer layer and is exposed to heat to allow the reinforcing element and the polymer to fuse together. In yet other exemplary aspects, the reinforcing jacket can be formed by injection molding. In such aspects, the reinforcing element can be positioned within a mold, and a polymer is injected over it. Yet, in other exemplary and unlimiting aspects, the reinforcing jacket can be formed by an extrusion process. In such exemplary aspects, the heated polymer can be extruded to a tube while in the parallel feed of the reinforcing element to allow the polymer and reinforcing element to combine.

Also are methods of making a sheath comprising a ballooning guard. In such methods, the sheath components, such as the inner liner and the outer layer, are formed by any of the disclosed above methods and assembled together to form a sheath. Then a ballooning guard having a proximal end and a distal end is disposed such that it overlayer at least a portion of the outer layer, and wherein the ballooning guard is configured to remain outside of a subject's vessel and to maintain hemostasis. The methods further connecting the proximal end of the ballooning guard to a most proximal portion of the outer layer and/or a hub of the sheath, wherein the distal end of the ballooning guard radially circumscribes at least a portion of the outer layer and wherein the distal end is not bonded to the outer layer. The ballooning guard, as described herein, is configured to adjust a length of the guard as a function of an insertion depth of the inner liner and outer layer of the sheath into a subject's vessel

In still further aspects, the disclosed herein methods can comprise a step of disposing a hydrophilic coating layer on the outer surface of the layer of the elastomeric polymer. Any disclosed herein hydrophilic coating can be used.

Sheaths of the present disclosure can be used with various methods of introducing a prosthetic device into a patient's vasculature. One such method comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some methods, the expandable sheath can be sutured to the patient's skin at the insertion site so that once the sheath is inserted the proper distance within the patient's vasculature, it does not move once the implantable device starts to travel through the sheath.

Disclosed aspects of an expandable sheath can be used with other delivery and minimally invasive surgical components, such as an introducer and loader. An introducer can be inserted into the expandable sheath, and the introducer/sheath combination can be fully inserted into vasculature over a guiding device, such as a 0.35" guidewire. Once the sheath and introducer are fully inserted into a patient's vasculature, in some aspects, the expandable sheath can be sutured in place at the insertion site. In this manner, the expandable sheath can be substantially prevented from moving once positioned within the patient.

**The** introducer can then be removed, and a medical device, such as a transcatheter heart valve, can be inserted into the sheath, in some instances, using a loader. Such methods can additionally comprise placing the tissue heart valve in a crimped state on the distal end portion of an elongated delivery apparatus and inserting the elongated delivery device with the crimped valve into and through the expandable sheath. Next, the delivery apparatus can be advanced through the patient's vasculature to the treatment site, where the valve can be implanted.

Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (*e.g*., the expanded configuration) just in the area where the medical device is currently located. Once the medical device passes a particular location of the sheath, the sheath can at least partially contract to the smaller diameter of its original configuration. The expandable sheath can thus be expanded without the use of inflatable balloons or other dilators. Once the medical device is implanted, the sheath and any sutures holding it in place can be removed. In some exemplary aspects, the sheath is removed without rotating it.

### EXAMPLES

The following examples are put forth so as to provide those of ordinary skill in the art with a complete disclosure and description of how the compounds, compositions, articles, devices and/or methods claimed herein are made and evaluated and are intended to be purely exemplary and are not intended to limit the disclosure. Efforts have been made to ensure accuracy with respect to numbers (*e.g*., amounts, temperature, etc.), but some errors and deviations should be accounted for.

Unless indicated otherwise, parts are parts by weight, temperature is degrees C or is at ambient temperature, and pressure is at or near atmospheric or full vacuum.

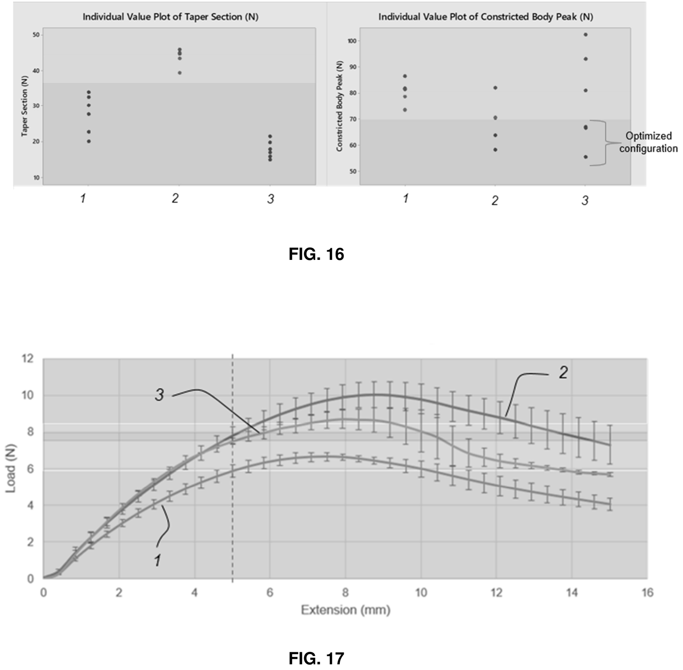

**FIG. 16** depicts experimental data measured for the insertion force required to insert a medical device in the disclosed herein sheath **3** as compared to commercially available sheaths **1** and **2.** The force was measured using a typical delivery system on a Zwick push force test machine. It can be seen that the required insertion force measured in the taper section of the sheath is lower than one needed for the commercially available sheaths **1** and **2.** Similarly, the insertion force measured in the constricted body peak shows better results for the disclosed sheath **3** as compared to the commercially available sheaths **1** and **2.**

**FIG. 17** depicts experimental data of the load measured as a function of the extension of the disclosed herein sheath **3** and the commercially available sheaths **1** and **2.** It can be seen that the disclosed sheath demonstrates comparable results with the clinically acceptable commercial sheath.

### EXEMPLARY ASPECTS:

**EXAMPLE 1:** A sheath for delivering a medical device, wherein the sheath has a proximal and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; an outer layer having an inner surface and an outer surface and extending about at least a portion of the variable diameter inner liner such that the inner surface of the outer layer is positioned adjacent to the outer surface of the inner liner, wherein the outer layer comprises: a braid or a coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 2:** The sheath of any examples herein, particularly example **Error! Reference source not found.,** wherein the rest diameter *dᵣ* is substantially uniform along the longitudinal axis of the lumen.

**EXAMPLE 3:** The sheath of any examples herein, particularly example **Error! Reference source not found.,** wherein the rest diameter *dᵣ* varies along the longitudinal axis of the lumen and wherein the rest diameter *dᵣ* at the proximal end that is larger than the rest diameter *dᵣ* at the distal end.

**EXAMPLE 4:** The sheath of any examples herein, particularly examples **Error! Reference source not found.-Error! Reference source not found.,** wherein the expanded diameter *dₑ* is configured to accommodate the medical device passing through the lumen.

**EXAMPLE 5:** The sheath of any examples herein, particularly examples **Error! Reference source not found.-Error! Reference source not found.,** wherein the sheath contracts to the predetermined rest diameter *dᵣ* after passage of the medical device through the lumen.

**EXAMPLE 6:** The sheath of any examples herein, particularly examples **Error! Reference source not found.-Error! Reference source not found.,** wherein the sheet comprises a high density polyethylene, polypropylene, polyamide, fluoropolymer, copolymers thereof, or blends thereof.

**EXAMPLE 7:** The sheath of any examples herein, particularly example **Error! Reference source not found.,** wherein the sheet has a multilayer structure.

**EXAMPLE 8:** The sheath of any examples herein, particularly examples **Error! Reference source not found.-Error! Reference source not found.,** wherein the inner surface of the sheet is at least partially ribbed.

**EXAMPLE 9:** The sheath of any examples herein, particularly examples **Error! Reference source not found.-Error! Reference source not found.,** wherein the sheet is lubricious and has a coefficient of friction less than about 0.5.

**EXAMPLE 10:** The sheath of any examples herein, particularly examples **Error! Reference source not found.-Error! Reference source not found.,** wherein an amount of a first lubricant is disposed between at least a portion of the inner liner and at least a portion of the outer layer.

**EXAMPLE 11:** The sheath of any examples herein, particularly examples **Error! Reference source not found.-Error! Reference source not found.,** wherein an amount of a second lubricant is disposed between at least a portion of the overlying portion of the sheet and at least a portion of the sliding portions of the sheet.

**EXAMPLE 12:** The sheath of any examples herein, particularly examples **Error! Reference source not found.-Error! Reference source not found.,** wherein the outer surface of the layer of the elastomeric polymer defines at least a portion of the outer surface of the outer layer.

**EXAMPLE 13:** The sheath of any examples herein, particularly examples **Error! Reference source not found.-Error! Reference source not found.,** wherein at least a portion of the inner surface of the layer of the elastomeric polymer is at least partially bonded to at least a portion of the outer surface of the sheet of the inner liner.

**EXAMPLE 14:** The sheath of any examples herein, particularly examples **Error! Reference source not found.-Error! Reference source not found.,** wherein at least a portion of the inner surface of the layer of the elastomeric polymer defines at least a portion of the inner surface of the outer layer.

**EXAMPLE 15:** The sheath of any examples herein, particularly examples **Error! Reference source not found.-Error! Reference source not found.,** wherein at least a portion of the braid or a coil defines at least a portion of the inner surface of the outer layer.

**EXAMPLE 16:** The sheath of any examples herein, particularly examples **Error! Reference source not found.-Error! Reference source not found.,** further comprising a first strip of the elastomeric polymer disposed along at least a portion of the longitudinal axis of the lumen between at least a portion of the outer surface of the sheet that does not comprise the overlaying portion of the sheet and the inner surface of the outer layer.

**EXAMPLE 17:** The sheath of any examples herein, particularly examples **Error! Reference source not found.-Error! Reference source not found.,** further comprising a second strip of the elastomeric polymer disposed between at least a portion of the outer surface of the sheet at the proximal end of the sheath and the inner surface of the outer layer.

**EXAMPLE 18:** The sheath of any examples herein, particularly examples **Error! Reference source not found.-Error! Reference source not found.,** further comprising a third strip of the elastomeric polymer disposed between at least a portion of the outer surface of the sheet at the distal end of the sheath and the inner surface of the outer layer.

**EXAMPLE 19:** The sheath of any examples herein, particularly examples **Error! Reference source not found.-Error! Reference source not found.,** wherein the braid or a coil is an expandable braid or an expandable coil.

**EXAMPLE 20:** The sheath of any examples herein, particularly examples **Error! Reference source not found.-Error! Reference source not found.,** wherein the braid or the coil comprises at least one filament comprising stainless steel, nitinol, a polymer material, or a composite material.

**EXAMPLE 21:** The sheath of any examples herein, particularly example **Error! Reference source not found.,** wherein the filament is a round filament or a flat filament.

**EXAMPLE 22:** The sheath of any examples herein, particularly examples **Error! Reference source not found.** or **Error! Reference source not found.,** wherein the polymer material is polyester or nylon.

**EXAMPLE 23:** The sheath of any examples herein, particularly examples 21-**Error! Reference source not found.,** wherein the round filament has a diameter of less than about 0.015".

**EXAMPLE 24:** The sheath of any examples herein, particularly examples 21-22, wherein the flat filament has a height of less than about 0.006" and a width from greater than about 0.003" to about 0.015".

**EXAMPLE 25:** The sheath of any examples herein, particularly examples **Error! Reference source not found.-Error! Reference source not found.,** wherein the braid has a per inch crosses (PIC) count of less than 50.

**EXAMPLE 26:** The sheath of any examples herein, particularly example **Error! Reference source not found.,** wherein the PIC varies along the longitudinal axis of the lumen.

**EXAMPLE 27:** The sheath of any examples herein, particularly examples **Error! Reference source not found.-Error! Reference source not found.,** wherein the filament is nitinol and wherein the nitinol is heat set at dₑ.

**EXAMPLE 28:** The sheath of any examples herein, particularly examples **Error! Reference source not found.-Error! Reference source not found.,** where the filament comprises stainless steel or nitinol, the filament is configured to be atraumatic at least at the distal end of the sheath.

**EXAMPLE 29:** The sheath of any examples herein, particularly examples **Error! Reference source not found.-Error! Reference source not found.,** wherein the elastomeric polymer comprises a styrene-based elastomer, polyurethane, latex, copolymers thereof, blends thereof, or co-extrudates thereof.

**EXAMPLE 30:** The sheath of any examples herein, particularly example **Error! Reference source not found.,** wherein the elastomeric polymer exhibit a Shore A durometer of less than 90.

**EXAMPLE 31:** The sheath of any examples herein, particularly examples **Error! Reference source not found.-Error! Reference source not found.,** wherein the braid or the coil is at least partially embedded within at least a portion of the layer of the elastomeric polymer.

**EXAMPLE 32:** The sheath of any examples herein, particularly examples **Error! Reference source not found.-Error! Reference source not found.,** wherein a hydrophilic coating layer is disposed on the outer surface of the outer layer.

**EXAMPLE 33:** A method of making a sheath having a proximal and a distal end and comprising: forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid or a coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 34:** The method of any examples herein, particularly example **Error! Reference source not found.,** wherein the step of forming the variable diameter inner liner comprises rolling the sheet over a mandrel having a predetermined diameter to form the spiral configuration, wherein the predetermined diameter of the mandrel is substantially identical to the predetermined diameter dᵣ of the inner liner.

**EXAMPLE 35:** The method of any examples herein, particularly examples **Error! Reference source not found.-Error! Reference source not found.,** wherein the rest diameter dᵣ is substantially uniform along the longitudinal axis of the lumen.

**EXAMPLE 36:** The method of any examples herein, particularly examples **Error! Reference source not found.-Error! Reference source not found.,** wherein the rest diameter *dᵣ* varies along the longitudinal axis of the lumen and wherein the rest diameter *dᵣ* at the proximal end that is larger than the rest diameter *dᵣ* at the distal end.

**EXAMPLE 37:** The method of any examples herein, particularly examples **Error! Reference source not found.-Error! Reference source not found.,** wherein the step of forming the outer layer comprises mounting the braid or the coil on the inner liner.

**EXAMPLE 38:** The method of any examples herein, particularly example **Error! Reference source not found.,** wherein the step of forming the outer layer further comprises mounting the elastomeric polymer on the braid.

**EXAMPLE 39:** The method of any examples herein, particularly examples **Error! Reference source not found.-Error! Reference source not found.,** further comprising at least partially embedding the braid or the coil within at least a portion of the layer of the elastomeric polymer.

**EXAMPLE 40:** The method of any examples herein, particularly examples **Error! Reference source not found.-Error! Reference source not found.,** wherein the step of forming the outer layer comprises mounting the layer of the elastic polymer on the braid or the coil and then mounting the layer of the elastic polymer and the braid or the coil on the inner liner positioned on the mandrel.

**EXAMPLE 41:** The method of any examples herein, particularly example **Error! Reference source not found.,** further comprising at least partially embedding the braid or the coil within at least a portion of the layer of the elastomeric polymer before mounting on the inner liner.

**EXAMPLE 42:** The method of any examples herein, particularly example **Error! Reference source not found.,** further comprising at least partially embedding the braid or the coil within at least a portion of the layer of the elastomeric polymer after mounting on the inner liner.

**EXAMPLE 43:** The method of any examples herein, particularly examples **Error! Reference source not found.-Error! Reference source not found.,** wherein the outer surface of the layer of the elastomeric polymer defines at least a portion of the outer surface of the outer layer.

**EXAMPLE 44:** The method of any examples herein, particularly examples **Error! Reference source not found.-Error! Reference source not found.,** wherein at least a portion of the inner surface of the layer of the elastomeric polymer defines at least a portion of the inner surface of the outer layer.

**EXAMPLE 45:** The method of any examples herein, particularly examples **Error! Reference source not found.-Error! Reference source not found.,** wherein at least a portion of the braid or the coil defines at least a portion of the inner surface of the outer layer.

**EXAMPLE 46:** The method of any examples herein, particularly examples **Error! Reference source not found.-Error! Reference source not found.,** further comprising bonding at least a portion of the inner surface of the layer of the elastomeric polymer to at least a portion of the outer surface of the sheet of the inner liner.

**EXAMPLE 47:** The method of any examples herein, particularly example **Error! Reference source not found.,** wherein the bonding is performed by heating at a temperature from about 350 °F to about 550 °F for a time period effective to form a bond between at least a portion of the outer layer and at least a portion of the inner liner.

**EXAMPLE 48:** The method of any examples herein, particularly example **Error! Reference source not found.** or **Error! Reference source not found.,** wherein a first strip of the elastomeric polymer is applied along at least a portion of the longitudinal axis of the lumen to at least a portion of the outer surface of the sheet that does not comprise the overlaying portion prior to or during the step of bonding the at least a portion of the inner surface of the layer of the elastomeric polymer to at least a portion of the outer surface of the sheet of the inner liner.

**EXAMPLE 49:** The method of any examples herein, particularly examples **Error! Reference source not found.-Error! Reference source not found.,** wherein a second strip of the elastomeric polymer is applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner.

**EXAMPLE 50:** The method of any examples herein, particularly examples **Error! Reference source not found.-Error! Reference source not found.,** wherein a third strip of the elastomeric polymer is applied to at least a portion of the outer surface of the sheet at the distal end of the sheath prior to or during the step of bonding the at least a portion of the inner surface of the layer of the elastomeric polymer to at least a portion of the outer surface of the sheet of the inner liner.

**EXAMPLE 51:** The method of any examples herein, particularly examples **Error! Reference source not found.-Error! Reference source not found.,** wherein an amount of a first lubricant is applied to at least a portion of the inner liner prior to the step of forming the outer layer such that the amount of the first lubricant is disposed between at least a portion of the inner liner and at least a portion of the outer liner in the sheath.

**EXAMPLE 52:** The method of any examples herein, particularly examples **Error! Reference source not found.-Error! Reference source not found.,** wherein an amount of a second lubricant is applied to at least a portion of the overlying and sliding portions of the sheet prior to the step of forming the outer layer.

**EXAMPLE 53:** The method of any examples herein, particularly examples **Error! Reference source not found.-Error! Reference source not found.,** wherein the sheet comprises a high density polyethylene, polypropylene, polyamide, fluoropolymer, copolymers thereof, or blends thereof.

**EXAMPLE 54:** The method of any examples herein, particularly example **Error! Reference source not found.,** wherein the sheet has a multilayer structure.

**EXAMPLE 55:** The method of any examples herein, particularly examples **Error! Reference source not found.-Error! Reference source not found.,** wherein the inner surface of the sheet is at least partially ribbed.

**EXAMPLE 56:** The method of any examples herein, particularly examples **Error! Reference source not found.-Error! Reference source not found.,** wherein the sheet is lubricious and has a coefficient of friction less than about 0.5.

**EXAMPLE 57:** The method of any examples herein, particularly examples **Error! Reference source not found.-Error! Reference source not found.,** wherein the braid or the coil is an expandable braid or an expandable coil.

**EXAMPLE 58:** The method of any examples herein, particularly examples **Error! Reference source not found.-Error! Reference source not found.,** wherein the braid or the coil comprises at least one filament comprising a stainless steel, nitinol, a polymer material, or a composite material.

**EXAMPLE 59:** The method of any examples herein, particularly example **Error! Reference source not found.,** wherein the filament is a round filament or a flat filament.

**EXAMPLE 60:** The method of any examples herein, particularly examples **Error! Reference source not found.** or **Error! Reference source not found.,** wherein the polymer material is a polyester or nylon.

**EXAMPLE 61:** The method of any examples herein, particularly examples **Error! Reference source not found.-Error! Reference source not found.,** wherein the round filament has a diameter of less than about 0.015".

**EXAMPLE 62:** The method of any examples herein, particularly examples **Error! Reference source not found.-Error! Reference source not found.,** wherein the flat filament has a height of less than about 0.006" and a width greater than about 0.003" to about 0.015".

**EXAMPLE 63:** The method of any examples herein, particularly examples **Error! Reference source not found.-Error! Reference source not found.,** wherein the braid has per inch crosses (PIC) count of less than 50.

**EXAMPLE 64:** The method of any examples herein, particularly example **Error! Reference source not found.,** wherein the PIC varies along the longitudinal axis of the lumen.

**EXAMPLE 65:** The method of any examples herein, particularly examples **Error! Reference source not found.-Error! Reference source not found.,** wherein the filament is nitinol and wherein the nitinol is heat set at *dₑ*.

**EXAMPLE 66:** The method of any examples herein, particularly examples **Error! Reference source not found.-Error! Reference source not found.,** where the filament comprises stainless steel or nitinol, the filament is configured to be atraumatic at least at the distal end of the sheath.

**EXAMPLE 67:** The method of any examples herein, particularly examples **Error! Reference source not found.-Error! Reference source not found.,** wherein the elastomeric polymer comprises a styrene-based elastomer, polyurethane, latex, copolymers thereof, blends thereof, or co-extrudates thereof.

**EXAMPLE 68:** The method of any examples herein, particularly example **Error! Reference source not found.,** wherein the elastomeric polymer exhibits a Shore A durometer of less than 90.

**EXAMPLE 69:** The method of any examples herein, particularly examples **Error! Reference source not found.-Error! Reference source not found.,** further comprising disposing a hydrophilic coating layer on the outer surface of the layer of the elastomeric polymer.

**EXAMPLE 70:** A sheath for delivering a medical device, wherein the sheath has a proximal and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; an outer layer having an inner surface and an outer surface and extending about at least a portion of the variable diameter inner liner such that the inner surface of the outer layer is positioned adjacent to the outer surface of the inner liner, wherein the outer layer comprises: at least one layer of a first elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 71:** The sheath of any examples herein, particularly example **Error! Reference source not found.,** wherein the rest diameter *dᵣ* is substantially uniform along the longitudinal axis of the lumen.

**EXAMPLE 72:** The sheath of any examples herein, particularly example **Error! Reference source not found.,** wherein the rest diameter *dᵣ* varies along the longitudinal axis of the lumen and wherein the rest diameter *dᵣ* at the proximal end that is larger than the rest diameter *dᵣ* at the distal end.

**EXAMPLE 73:** The sheath of any examples herein, particularly examples **Error! Reference source not found.-Error! Reference source not found.,** wherein the expanded diameter *dₑ* is configured to accommodate the medical device passing through the lumen.

**EXAMPLE 74:** The sheath of any examples herein, particularly examples **Error! Reference source not found.-Error! Reference source not found.,** wherein the sheath contracts to the predetermined rest diameter *dᵣ* after passage of the medical device through the lumen.

**EXAMPLE 75:** The sheath of any examples herein, particularly examples **Error! Reference source not found.-Error! Reference source not found.,** wherein the sheet comprises a high density polyethylene, polypropylene, polyamide, fluoropolymer, copolymers thereof, or blends thereof.

**EXAMPLE 76:** The sheath of any examples herein, particularly example **Error! Reference source not found.,** wherein the sheet has a multilayer structure.

**EXAMPLE 77:** The sheath of any examples herein, particularly examples **Error! Reference source not found.-Error! Reference source not found.,** wherein the inner surface of the sheet is at least partially ribbed.

**EXAMPLE 78:** The sheath of any examples herein, particularly examples **Error! Reference source not found.-Error! Reference source not found.,** wherein the sheet is lubricious and has a coefficient of friction less than about 0.5.

**EXAMPLE 79:** The sheath of any examples herein, particularly examples **Error! Reference source not found.-Error! Reference source not found.,** wherein an amount of a first lubricant is disposed between at least a portion of the inner liner and at least a portion of the outer layer.

**EXAMPLE 80:** The sheath of any examples herein, particularly examples **Error! Reference source not found.-Error! Reference source not found.,** wherein an amount of a second lubricant is disposed between at least a portion of the overlying portion of the sheet and at least a portion of the sliding portions of the sheet.

**EXAMPLE 81:** The sheath of any examples herein, particularly example **Error! Reference source not found.,** wherein the first and the second lubricants are the same or different.

**EXAMPLE 82:** The sheath of any examples herein, particularly examples **Error! Reference source not found.-Error! Reference source not found.,** wherein the outer surface of the at least one layer of the first elastomeric polymer defines at least a portion of the outer surface of the outer layer.

**EXAMPLE 83:** The sheath of any examples herein, particularly examples **Error! Reference source not found.-Error! Reference source not found.,** wherein at least a portion of the inner surface of the at least one layer of the first elastomeric polymer is at least partially bonded to at least a portion of the outer surface of the sheet of the inner liner.

**EXAMPLE 84:** The sheath of any examples herein, particularly examples **Error! Reference source not found.-Error! Reference source not found.,** wherein at least a portion of the inner surface of the at least one layer of the first elastomeric polymer defines at least a portion of the inner surface of the outer layer.

**EXAMPLE 85:** The sheath of any examples herein, particularly examples **Error! Reference source not found.-Error! Reference source not found.,** further comprising a braid or a coil positioned such that it covers at least a portion of the inner layer, wherein at least a portion of the braid or coil defines at least a portion of the inner surface of the outer layer.

**EXAMPLE 86:** The sheath of any examples herein, particularly examples **Error! Reference source not found.-Error! Reference source not found.,** further comprising at least one strip of a second elastomeric polymer.

**EXAMPLE 87:** The sheath of any examples herein, particularly example **Error! Reference source not found.,** wherein the second elastomeric polymer and the first elastomeric polymers are the same or different.

**EXAMPLE 88:** The sheath of any examples herein, particularly examples **Error! Reference source not found.** or **Error! Reference source not found.,** wherein the second elastomeric polymer has a higher Shore durometer than the first elastomeric polymer.

**EXAMPLE 89:** The sheath of any examples herein, particularly examples **Error! Reference source not found.-Error! Reference source not found.,** further comprising a first strip of a third elastomeric polymer disposed between at least a portion of the outer surface of the sheet that does not comprise the overlaying portion of the sheet and the inner surface of the outer layer.

**EXAMPLE 90:** The sheath of any examples herein, particularly examples **Error! Reference source not found.-Error! Reference source not found.,** further comprising a second strip of a fourth elastomeric polymer disposed between at least a portion of the outer surface of the sheet at the proximal end of the sheath and the inner surface of the outer layer.

**EXAMPLE 91:** The sheath of any examples herein, particularly examples **Error! Reference source not found.-Error! Reference source not found.,** further comprising a third strip of a fifth elastomeric polymer disposed between at least a portion of the outer surface of the sheet at the distal end of the sheath and the inner surface of the outer layer.

**EXAMPLE 92:** The sheath of any examples herein, particularly example **Error! Reference source not found.,** wherein the third, fourth, and/or fifth elastomeric polymers are the same or different.

**EXAMPLE 93:** The sheath of any examples herein, particularly example **Error! Reference source not found.,** wherein the third, fourth, and/or fifth elastomeric polymers comprise the first elastomeric polymer, the second elastomeric polymer or a combination thereof.

**EXAMPLE 94:** The sheath of any examples herein, particularly examples **Error! Reference source not found.-Error! Reference source not found.,** wherein the braid or coil is expandable.

**EXAMPLE 95:** The sheath of any examples herein, particularly examples **Error! Reference source not found.-Error! Reference source not found.,** wherein the braid or coil comprises at least one filament comprising stainless steel, nitinol, a polymer material, or a composite material.

**EXAMPLE 96:** The sheath of any examples herein, particularly example **Error! Reference source not found.,** wherein the filament is a round filament or a flat filament.

**EXAMPLE 97:** The sheath of any examples herein, particularly examples **Error! Reference source not found.** or **Error! Reference source not found.,** wherein the polymer material is polyester or nylon.

**EXAMPLE 98:** The sheath of any examples herein, particularly examples **Error! Reference source not found.-Error! Reference source not found.,** wherein the round filament has a diameter of less than about 0.015".

**EXAMPLE 99:** The sheath of any examples herein, particularly examples **Error! Reference source not found.-Error! Reference source not found.,** wherein the flat filament has a height of less than about 0.006" and a width from greater than about 0.003" to about 0.015".

**EXAMPLE 100:** The sheath of any examples herein, particularly examples **Error! Reference source not found.-Error! Reference source not found.,** wherein the braid has a per inch crosses (PIC) count of less than 50.

**EXAMPLE 101:** The sheath of any examples herein, particularly example **Error! Reference source not found.,** wherein the PIC varies along the longitudinal axis of the lumen.

**EXAMPLE 102:** The sheath of any examples herein, particularly examples **Error! Reference source not found.-Error! Reference source not found.,** wherein the filament is nitinol and wherein the nitinol is heat set at *dₑ*.

**EXAMPLE 103:** The sheath of any examples herein, particularly examples **Error! Reference source not found.-Error! Reference source not found.,** where the filament comprises stainless steel or nitinol, the filament is configured to be atraumatic at least at the distal end of the sheath.

**EXAMPLE 104:** The sheath of any examples herein, particularly examples **Error! Reference source not found.-Error! Reference source not found.,** wherein the first through fifth elastomeric polymer comprise a styrene-based elastomer, polyurethane, latex, copolymers thereof, blends thereof, or co-extrudates thereof, or a combination thereof.

**EXAMPLE 105:** The sheath of any examples herein, particularly example **Error! Reference source not found.,** wherein at least one of the first through fifth elastomeric polymers exhibit a Shore A durometer of less than 90.

**EXAMPLE 106:** The sheath of any examples herein, particularly examples **Error! Reference source not found.-Error! Reference source not found.,** wherein the braid or coil is at least partially embedded within at least a portion of the at least one layer of the first elastomeric polymer.

**EXAMPLE 107:** The sheath of any examples herein, particularly examples **Error! Reference source not found.-Error! Reference source not found.,** wherein a hydrophilic coating layer is disposed on the outer surface of the outer layer.

**EXAMPLE 108:** A method of making a sheath having a proximal and a distal end and comprising: forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: at least one layer of a first elastomeric polymer having a predetermined thickness and having an inner surface and outer surface; and wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 109:** The method of any examples herein, particularly example **Error! Reference source not found.,** wherein the step of forming the variable diameter inner liner comprises rolling the sheet over a mandrel having a predetermined diameter to form the spiral configuration, wherein the predetermined diameter of the mandrel is substantially identical to the predetermined diameter dᵣ of the inner liner.

**EXAMPLE 110:** The method of any examples herein, particularly examples **Error! Reference source not found.-Error! Reference source not found.,** wherein the rest diameter dᵣ is substantially uniform along the longitudinal axis of the lumen.

**EXAMPLE 111:** The method of any examples herein, particularly examples **Error! Reference source not found.-Error! Reference source not found.,** wherein the rest diameter *dᵣ* varies along the longitudinal axis of the lumen and wherein the rest diameter *dᵣ* at the proximal end that is larger than the rest diameter *dᵣ* at the distal end.

**EXAMPLE 112:** The method of any examples herein, particularly examples **Error! Reference source not found.-Error! Reference source not found.,** wherein the step of forming the outer layer further comprises mounting a braid or coil on at least some portion of the inner liner.

**EXAMPLE 113:** The method of any examples herein, particularly example **Error! Reference source not found.,** wherein the step of forming the outer layer further comprises mounting the at least one layer of the first elastomeric polymer on the braid or coil.

**EXAMPLE 114:** The method of any examples herein, particularly examples **Error! Reference source not found.-Error! Reference source not found.,** further comprising at least partially embedding the braid or coil within at least a portion of the at least one layer of the first elastomeric polymer.

**EXAMPLE 115:** The method of any examples herein, particularly examples **Error! Reference source not found.-Error! Reference source not found.,** wherein the step of forming the outer layer comprises mounting the at least one layer of the first elastic polymer on a braid or coil and then mounting the at least one layer of the first elastic polymer and the braid or coil on the inner liner positioned on the mandrel.

**EXAMPLE 116:** The method of any examples herein, particularly example **Error! Reference source not found.,** further comprising at least partially embedding the braid or coil within at least a portion of the at least one layer of the first elastomeric polymer before mounting on the inner liner.

**EXAMPLE 117:** The method of any examples herein, particularly example **Error! Reference source not found.,** further comprising at least partially embedding the braid or coil within at least a portion of the layer of the elastomeric polymer after mounting on the inner liner.

**EXAMPLE 118:** The method of any examples herein, particularly examples **Error! Reference source not found.-Error! Reference source not found.,** wherein the outer surface of the at least one layer of the first elastomeric polymer defines at least a portion of the outer surface of the outer layer.

**EXAMPLE 119:** The method of any examples herein, particularly examples **Error! Reference source not found.-Error! Reference source not found.,** wherein at least a portion of the inner surface of the at least one layer of the first elastomeric polymer defines at least a portion of the inner surface of the outer layer.

**EXAMPLE 120:** The method of any examples herein, particularly examples **Error! Reference source not found.-Error! Reference source not found.,** wherein at least a portion of the braid or coil defines at least a portion of the inner surface of the outer layer.

**EXAMPLE 121:** The method of any examples herein, particularly examples **Error! Reference source not found.-Error! Reference source not found.,** further comprising bonding at least a portion of the inner surface of the at least one layer of the first elastomeric polymer to at least a portion of the outer surface of the sheet of the inner liner.

**EXAMPLE 122:** The method of any examples herein, particularly example **Error! Reference source not found.,** wherein the bonding is performed by heating at a temperature from about 350 °F to about 550 °F for a time period effective to form a bond between at least a portion of the outer layer and at least a portion of the inner liner.

**EXAMPLE 123:** The method of any examples herein, particularly examples **Error! Reference source not found.-Error! Reference source not found.,** wherein the sheath further comprises at least one strip of a second elastomeric polymer.

**EXAMPLE 124:** The method of any examples herein, particularly example **Error! Reference source not found.,** wherein the second elastomeric polymer and the first elastomeric polymers are the same or different.

**EXAMPLE 125:** The method of any examples herein, particularly examples **Error! Reference source not found.** or **Error! Reference source not found.,** wherein the second elastomeric polymer has a higher Shore durometer than the first elastomeric polymer.

**EXAMPLE 126:** The method of any examples herein, particularly examples **Error! Reference source not found.-Error! Reference source not found.,** wherein a first strip of a third elastomeric polymer is applied along at least a portion of the longitudinal axis of the lumen to at least a portion of the outer surface of the sheet that does not comprise the overlaying portion prior to or during the step of bonding the at least a portion of the inner surface of the at least one layer of the first elastomeric polymer to at least a portion of the outer surface of the sheet of the inner liner.

**EXAMPLE 127:** The method of any examples herein, particularly examples **Error! Reference source not found.-Error! Reference source not found.,** wherein a second strip of a fourth elastomeric polymer is applied to at least a portion of the outer surface of the sheet at the proximal end of the sheath prior to or during the step of bonding the at least a portion of the inner surface of the at least one layer of the first elastomeric polymer to at least a portion of the outer surface of the sheet of the inner liner.

**EXAMPLE 128:** The method of any examples herein, particularly examples **Error! Reference source not found.-Error! Reference source not found.,** wherein a third strip of a fifth elastomeric polymer is applied to at least a portion of the outer surface of the sheet at the distal end of the sheath prior to or during the step of bonding the at least a portion of the inner surface of the at least one layer of the first elastomeric polymer to at least a portion of the outer surface of the sheet of the inner liner.

**EXAMPLE 129:** The method of any examples herein, particularly example **Error! Reference source not found.,** wherein the third, fourth, and/or fifth elastomeric polymers are the same or different.

**EXAMPLE 130:** The method of any examples herein, particularly example **Error! Reference source not found.,** wherein the third, fourth, and/or fifth elastomeric polymers comprise the first elastomeric polymer, the second elastomeric polymer or a combination thereof.

**EXAMPLE 131:** The method of any examples herein, particularly examples **Error! Reference source not found.-Error! Reference source not found.,** wherein an amount of a first lubricant is applied to at least a portion of the inner liner prior to the step of forming the outer layer such that the amount of the first lubricant is disposed between at least a portion of the inner liner and at least a portion of the outer liner in the sheath.

**EXAMPLE 132:** The method of any examples herein, particularly examples **Error! Reference source not found.-Error! Reference source not found.,** wherein an amount of a second lubricant is applied to at least a portion of the overlying and sliding portions of the sheet prior to the step of forming the outer layer.

**EXAMPLE 133:** The method of any examples herein, particularly example **Error! Reference source not found.,** wherein the first and the second lubricants are the same or different.

**EXAMPLE 134:** The method of any examples herein, particularly examples **Error! Reference source not found.-Error! Reference source not found.,** wherein the sheet comprises a high density polyethylene, polypropylene, polyamide, fluoropolymer, copolymers thereof, or blends thereof.

**EXAMPLE 135:** The method of any examples herein, particularly example **Error! Reference source not found.,** wherein the sheet has a multilayer structure.

**EXAMPLE 136:** The method of any examples herein, particularly examples **Error! Reference source not found.-Error! Reference source not found.,** wherein the inner surface of the sheet is at least partially ribbed.

**EXAMPLE 137:** The method of any examples herein, particularly examples **Error! Reference source not found.-Error! Reference source not found.,** wherein the sheet is lubricious and has a coefficient of friction less than about 0.5.

**EXAMPLE 138:** The method of any examples herein, particularly examples **Error! Reference source not found.-Error! Reference source not found.,** wherein the braid or coil is an expandable braid or coil.

**EXAMPLE 139:** The method of any examples herein, particularly examples **Error! Reference source not found.-Error! Reference source not found.,** wherein the braid or coil comprises at least one filament comprising a stainless steel, nitinol, a polymer material, or a composite material.

**EXAMPLE 140:** The method of any examples herein, particularly example **Error! Reference source not found.,** wherein the filament is a round filament or a flat filament.

**EXAMPLE 141:** The method of any examples herein, particularly examples **Error! Reference source not found.** or **Error! Reference source not found.,** wherein the polymer material is a polyester or nylon.

**EXAMPLE 142:** The method of any examples herein, particularly examples **Error! Reference source not found.-Error! Reference source not found.,** wherein the round filament has a diameter of less than about 0.015".

**EXAMPLE 143:** The method of any examples herein, particularly examples **Error! Reference source not found.-Error! Reference source not found.,** wherein the flat filament has a height of less than about 0.006" and a width greater than about 0.003" to about 0.015".

**EXAMPLE 144:** The method of any examples herein, particularly examples **Error! Reference source not found.-Error! Reference source not found.,** wherein the braid has per inch crosses (PIC) count of less than 50.

**EXAMPLE 145:** The method of any examples herein, particularly example **Error! Reference source not found.,** wherein the PIC varies along the longitudinal axis of the lumen.

**EXAMPLE 146:** The method of any examples herein, particularly examples **Error! Reference source not found.-Error! Reference source not found.,** wherein the filament is nitinol and wherein the nitinol is heat set at *dₑ*.

**EXAMPLE 147:** The method of any examples herein, particularly examples **Error! Reference source not found.-Error! Reference source not found.,** where the filament comprises stainless steel or nitinol, the filament is configured to be atraumatic at least at the distal end of the sheath.

**EXAMPLE 148:** The method of any examples herein, particularly examples **Error! Reference source not found.-Error! Reference source not found.,** the first through fifth elastomeric polymer comprises a styrene-based elastomer, polyurethane, latex, copolymers thereof, blends thereof, or co-extrudates thereof, or combination thereof.

**EXAMPLE 149:** The method of any examples herein, particularly example **Error! Reference source not found.,** wherein at least one of the first through fifth elastomeric polymers exhibit a Shore A durometer of less than 90.

**EXAMPLE 150:** The method of any examples herein, particularly examples **Error! Reference source not found.-Error! Reference source not found.,** further comprising disposing a hydrophilic coating layer on the outer surface of the layer of the elastomeric polymer.

**EXAMPLE 151:** A sheath for delivering a medical device, wherein the sheath has a proximal and a distal end and comprises: a) an inner liner defining a lumen having a first rest diameter *dᵣ* and a second expanded diameter *dₑ,* wherein the lumen is configured to receive and pass through a medical device, wherein the inner liner comprises a sheet comprising a first portion having a first surface and an opposite second surface, wherein a first end of the first portion splits into a first segment having a first surface and an opposite second surface and a third segment having a first surface and an opposite second surface, and wherein a second end of the first portion extends into a second segment having a first surface and an opposite second surface, wherein the sheet is rolled into a spiral configuration, such that at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment, wherein the first surface of the first portion extends into the first surface of the first segment, the second segment and the third segment, and wherein the second surface of the first portion extends into the second surface of the second and the third segments, wherein each segment is configured to slidably move along each other upon passage of the medical device through the lumen; wherein the sheet comprises a polymer layer; and b) an outer layer.

**EXAMPLE 152:The** sheath of any examples herein, particularly example **Error! Reference source not found.,** wherein the at least a portion of the first surface of the second segment overlaps the at least a portion of the second surface of the first segment such that a first gap is formed between the at least a portion of the first surface of the second segment and the at least a portion of the second surface of the first segment.

**EXAMPLE 153:** The sheath of any examples herein, particularly examples **Error! Reference source not found.** or **Error! Reference source not found.,** wherein the at least a portion of the first surface of the third segment overlaps the at least a portion of the second surface of the second segment such that a second gap is formed between the at least a portion of the first surface of the third segment and the at least a portion of the second surface of the second segment.

**EXAMPLE 154:** The sheath of any examples herein, particularly examples **Error! Reference source not found.-Error! Reference source not found.,** wherein the at least a portion of the first surface of the third segment overlaps the at least a portion of the second surface of the first segment such that a third gap is formed between the at least a portion of the first surface of the third segment and the at least a portion of the second surface of the first segment.

**EXAMPLE 155:** The sheath of any examples herein, particularly examples **Error! Reference source not found.-Error! Reference source not found.,** wherein the first gap has a substantially uniform width along overlapping portions.

**EXAMPLE 156:** The sheath of any examples herein, particularly examples **Error! Reference source not found.-Error! Reference source not found.,** wherein the first gap has a variable width along overlapping portions.

**EXAMPLE 157:** The sheath of any examples herein, particularly examples **Error! Reference source not found.-Error! Reference source not found.,** wherein the second gap has a substantially uniform width along overlapping portions.

**EXAMPLE 158:** The sheath of any examples herein, particularly examples **Error! Reference source not found.-Error! Reference source not found.,** wherein the second gap has a variable width along overlapping portions.

**EXAMPLE 159:** The sheath of any examples herein, particularly examples **Error! Reference source not found.-Error! Reference source not found.,** wherein the third gap has a substantially uniform width along overlapping portions.

**EXAMPLE 160:** The sheath of any examples herein, particularly examples **Error! Reference source not found.-Error! Reference source not found.,** wherein the third gap has a variable width along overlapping portions.

**EXAMPLE 161:** The sheath of any examples herein, particularly examples **Error! Reference source not found.-Error! Reference source not found.,** wherein at least a portion of the second segment is positioned within at least a portion of the third gap.

**EXAMPLE 162:** The sheath of any examples herein, particularly examples **Error! Reference source not found.-Error! Reference source not found.,** wherein when the medical device passes through the lumen, the first segment, the second segment, and the third segment are configured to slidably move along each other such that an overlapping portion between the first segment and the second segment and between the second segment and the third segment decreases, while an overlapping portion between the first segment and the third segment increases.

**EXAMPLE 163:** The sheath of any examples herein, particularly example **Error! Reference source not found.,** wherein a diameter of lumen increases from the first rest diameter *dᵣ* to the second expanded diameter *dₑ*.

**EXAMPLE 164:** The sheath of any examples herein, particularly examples **Error! Reference source not found.-Error! Reference source not found.,** wherein after passage of the medical device through the lumen, the first segment, the second segment, and the third segment are configured to slidably move back along each other such that the overlapping portion between the first segment and the second segment, between the second segment and the third segment, and between the first segment and the third segment increases.

**EXAMPLE 165:** The sheath of any examples herein, particularly example **Error! Reference source not found.,** wherein the diameter of lumen decreases from the second expanded diameter *dₑ* to a diameter that is substantially identical to the first rest diameter *dᵣ*.

**EXAMPLE 166:** A sheath for delivering a medical device, wherein the sheath has a proximal end and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; wherein the sheet comprises a polymer layer; an outer layer having a predetermined thickness and having an inner surface and outer surface, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 167:** The sheath of any examples herein, particularly example 1, wherein the first rest diameter *dᵣ* is substantially uniform along the longitudinal axis of the lumen.

**EXAMPLE 168:** The sheath of any examples herein, particularly example 1, wherein the rest diameter *dᵣ* varies along the longitudinal axis of the lumen, and wherein the rest diameter *dᵣ* at the proximal end is larger than the rest diameter *dᵣ* at the distal end.

**EXAMPLE 169:** The sheath of any examples herein, particularly examples 1-**Error! Reference source not found.,** wherein the expanded diameter *dₑ* is configured to accommodate the medical device passing through the lumen.

**EXAMPLE 170:** The sheath of any examples herein, particularly examples 1-**Error! Reference source not found.,** wherein the sheath contracts to the predetermined rest diameter *dᵣ* after passage of the medical device through the lumen.

**EXAMPLE 171:** The sheath of any examples herein, particularly examples 1-**Error! Reference source not found.,** wherein when the sheath is in an unexpanded rest state, the first and the second edges of the sheath are substantially aligned in a spaced relationship along a vertical axis passing through a thickness of the sheath.

**EXAMPLE 172:** The sheath of any examples herein, particularly example 2, wherein the spaced relationship comprises a portion of the sheet positioned between the first edge and the second edge along the vertical axis.

**EXAMPLE 173:** The sheath of any examples herein, particularly examples 2 or **Error! Reference source not found.,** wherein when the sheet is in an unexpanded rest state, the inner liner comprises at least two layers of the sheet overlaying each other at at least a portion of a circumference of the sheath.

**EXAMPLE 174:** The sheath of any examples herein, particularly example 3, wherein when the sheet is in an unexpanded rest state, the inner liner comprises three layers of the sheet overlaying each other at at least a portion of a circumference of the sheath.

**EXAMPLE 175:** The sheath of any examples herein, particularly examples 1-**Error! Reference source not found.,** wherein the sheath is in a rest state, the first edge of the sheet is substantially aligned with a vertical axis passing through a thickness of the sheath and the second edge circumferentially offset from the vertical axis.

**EXAMPLE 176:** The sheath of any examples herein, particularly example 4, wherein at at least a portion of a circumference of the sheath, the inner liner comprises one layer of the sheet without any overlaying portion.

**EXAMPLE 177:** The sheath of any examples herein, particularly examples **Error! Reference source not found.-Error! Reference source not found.,** wherein an amount of a lubricant is disposed over at least a portion of the first surface of the first portion, at least a portion of the first surface of the first segment, and/or at least a portion of the first surface of the second segment that does not overlap the at least a portion of the second surface of the first segment.

**EXAMPLE 178:** The sheath of any examples herein, particularly examples **Error! Reference source not found.-Error! Reference source not found.** or **Error! Reference source not found.,** wherein an amount of the lubricant is disposed over an overlapping portion between the at least a portion of the first surface of the second segment and the at least a portion of the second surface of the first segment.

**EXAMPLE 179:** The sheath of any examples herein, particularly examples **Error! Reference source not found.-Error! Reference source not found.** or **Error! Reference source not found.-Error! Reference source not found.,** wherein an amount of the lubricant is disposed in an overlapping portion between the at least a portion of the first surface of the third segment and the at least a portion of the second surface of the second segment.

**EXAMPLE 180:** The sheath of any examples herein, particularly examples **Error! Reference source not found.-Error! Reference source not found.** or **Error! Reference source not found.-Error! Reference source not found.,** wherein an amount of the lubricant is disposed in an overlapping portion between the at least a portion of the first surface of the third segment and the at least a portion of the second surface of the first segment.

**EXAMPLE 181:** The sheath of any examples herein, particularly example **Error! Reference source not found.,** wherein an amount of the lubricant is disposed in an overlapping portion between the at least a portion of the first surface of the third segment and the at least a portion of the second surface of the first segment.

**EXAMPLE 182:** The sheath of any examples herein, particularly examples **Error! Reference source not found.-Error! Reference source not found.** or **Error! Reference source not found.-Error! Reference source not found.,** wherein an amount of the lubricant is disposed on at least a portion of the second surface of the first portion, at least a portion of the second surface of the third segment, and/or at least a portion of the second surface of the second segment that is not overlapped by the at least a portion of the first surface of the third segment.

**EXAMPLE 183**:The sheath of any examples herein, particularly examples **Error! Reference source not found.-Error! Reference source not found.** or **Error! Reference source not found.-Error! Reference source not found.,** wherein an amount of the lubricant is disposed at at least a portion of an outermost surface of the sheet.

**EXAMPLE 184:** The sheath of any examples herein, particularly examples 1-5, wherein an amount of a lubricant is disposed between at least a portion of the overlying portion of the sheet and at least a portion of the sliding portions of the sheet.

**EXAMPLE 185:** The sheath of any examples herein, particularly example 6, wherein an amount of the lubricant is disposed along at least a portion of the inner surface of the sheet.

**EXAMPLE 186:** The sheath of any examples herein, particularly example 6 or **Error! Reference source not found.,** wherein an amount of the lubricant is disposed along at least a portion of the outer surface of the sheet.

**EXAMPLE 187:** The sheath of any examples herein, particularly examples **Error! Reference source not found.-Error! Reference source not found.,** wherein the lubricant comprises PTFE-based lubricant or a silicone-based lubricant.

**EXAMPLE 188:** The sheath of any examples herein, particularly examples **Error! Reference source not found.-Error! Reference source not found.** wherein the disposed lubricant has a predetermined pattern.

**EXAMPLE 189:** The sheath of any examples herein, particularly examples **Error! Reference source not found.-7,** wherein the lubricant is pad-printed.

**EXAMPLE 190:** The sheath of any examples herein, particularly examples **Error! Reference source not found.-Error! Reference source not found.,** wherein the lubricant is sprayed.

**EXAMPLE 191:** The sheath of any examples herein, particularly examples 7-**Error! Reference source not found.,** wherein a viscosity of the lubricant prior to disposing is about 600 to about 1,200 cP.

**EXAMPLE 192:** The sheath of any examples herein, particularly examples 7 or **Error! Reference source not found.,** wherein a viscosity of the lubricant prior to disposing is equal to or less than about 600 cP.

**EXAMPLE 193:** The sheath of any examples herein, particularly examples **Error! Reference source not found.-Error! Reference source not found.,** wherein the lubricant forms a film having a thickness of equal to or less than about 20 µm.

**EXAMPLE 194:** The sheath of any examples herein, particularly examples **Error! Reference source not found.-Error! Reference source not found.,** wherein the lubricant is cured.

**EXAMPLE 195:** The sheath of any examples herein, particularly examples **Error! Reference source not found.-Error! Reference source not found.,** wherein the polymer layer of the sheet comprises a polyolefin, a polyamide, a fluoropolymer, copolymers thereof, co-extrudates thereof, or blends thereof.

**EXAMPLE 196:** The sheath of any examples herein, particularly example **Error! Reference source not found.,** wherein the polyolefin comprises a high density polyethylene, polypropylene, or blends thereof.

**EXAMPLE 197:** The sheath of any examples herein, particularly examples **Error! Reference source not found.-Error! Reference source not found.,** wherein the sheet has a multilayer structure.

**EXAMPLE 198:** The sheath of any examples herein, particularly examples **Error! Reference source not found.-Error! Reference source not found.,** wherein the polymer layer of the sheet comprises a compound material comprising a polyolefin and a lubricious filler.

**EXAMPLE 199:** The sheath of any examples herein, particularly example 8, wherein the polyolefin is high-density polyethylene.

**EXAMPLE 200:** The sheath of any examples herein, particularly example 8 or **Error! Reference source not found.,** wherein the lubricous filler comprises a polytetrafluoroethylene (PTFE) filler.

**EXAMPLE 201:** The sheath of any examples herein, particularly examples 8-**Error! Reference source not found.,** wherein the lubricious filler is present in an amount from about 5 wt % to about 20 wt % of a total weight of the compound material.

**EXAMPLE 202**:The sheath of any examples herein, particularly examples 8-9, wherein the sheet is lubricious and has a coefficient of friction less than about 0.5.

**EXAMPLE 203:** The sheath of any examples herein, particularly examples 8-**Error! Reference source not found.,** wherein the polymer layer of the sheet comprises the compound material, the sheath is substantially free of a separately disposed lubricant.

**EXAMPLE 204:** The sheath of any examples herein, particularly example **Error! Reference source not found.,** wherein the sheath exhibits a push force that is comparable or smaller than a push force of a substantially identical reference sheath rolled in a spiral configuration, wherein an inner liner of the substantially identical reference sheath comprises a polymer layer substantially free of a lubricious filler and comprises an amount of a lubricant disposed between overlapping portions of the spiral configuration and/or an outermost surface of the inner liner.

**EXAMPLE 205:** A sheath for delivering a medical device, wherein the sheath has a proximal and a distal end and comprises: a variable diameter inner liner comprising a sheet comprising a polymer layer comprising a compound material, wherein the compound material comprises a polyolefin present in an amount from greater than 0 wt% to less than 100 wt % based on a total weight of the compound and a lubricious filler present in an amount from about 5 wt % to about 20 wt% of a total weight to the compound material; wherein the sheet is rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; an outer layer having a predetermined thickness and having an inner surface and outer surface, wherein the inner surface of the outer layer is disposed on the outer surface of the spiral configuration of the inner liner; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 206:** The sheath of any examples herein, particularly examples **Error! Reference source not found.-Error! Reference source not found.,** further comprising at least one tie layer.

**EXAMPLE 207:** The sheath of any examples herein, particularly example 10, wherein the tie layer is disposed over the first surface of the first portion, the first surface of the first segment, the first surface of the second segment, and the first surface of the third segment, such that when the sheet is in the spiral configuration, the tie layer is disposed between the overlapping portions and at at least an inner surface of the lumen.

**EXAMPLE 208:** The sheath of any examples herein, particularly example 10, wherein the tie layer is disposed at the inner surface of the sheet, such that when the sheet is in the spiral configuration, the tie layer is disposed between the overlapping portions and at at least an inner surface of the lumen.

**EXAMPLE 209:** The sheath of any examples herein, particularly examples **10-Error! Reference source not found.,** wherein the tie layer is disposed at the second surface of the first portion, the second surface of the first segment, the second surface of the second segment, and the second surface of the third segment, such that when the sheet is in the spiral configuration, the tie layer is disposed between the overlapping portions and at an outermost surface of the inner liner.

**EXAMPLE 210:** The sheath of any examples herein, particularly examples 10 or **Error! Reference source not found.,** wherein the tie layer is disposed at the outer surface of the sheet such that when the sheet is in the spiral configuration, the tie layer is disposed between the overlapping portions and at an outermost surface of the inner liner.

**EXAMPLE 211:** The sheath of any examples herein, particularly examples 10-11, wherein the tie layer is coextruded with the sheet.

**EXAMPLE 212:** The sheath of any examples herein, particularly examples 10-12, wherein the tie layer comprises polyurethane, maleic anhydride modified polyolefin, ethylene acrylic acid copolymer, ethylene acrylate copolymer, ethylene glycidyl methacrylate copolymer, ethylene acrylic esters glycidyl methacrylate terpolymer, ethylene acrylic esters, maleic anhydride terpolymer, or any copolymers or blends thereof.

**EXAMPLE 213:** The sheath of any examples herein, particularly examples 10-**Error! Reference source not found.,** wherein the sheet has a thickness from about 0.001" to about 0.020".

**EXAMPLE 214:** The sheath of any examples herein, particularly examples 10**-Error! Reference source not found.,** wherein the tie layer has a thickness from about 0.001" to about 0.003".

**EXAMPLE 215:** The sheath of any examples herein, particularly examples **Error! Reference source not found.-Error! Reference source not found.,** further comprising at least one lubricious liner.

**EXAMPLE 216:** The sheath of any examples herein, particularly example 13, wherein the at least one lubricious liner is disposed on the tie layer.

**EXAMPLE 217:** The sheath of any examples herein, particularly example 14, wherein the at least one lubricious liner is bonded to the sheet with the tie layer.

**EXAMPLE 218:** The sheath of any examples herein, particularly examples 13-**Error! Reference source not found.,** wherein the at least one lubricious liner comprises PTFE.

**EXAMPLE 219:** The sheath of any examples herein, particularly example **Error! Reference source not found.,** wherein the at least one lubricious liner is at least partially etched.

**EXAMPLE 220:** The sheath of any examples herein, particularly examples 13-**Error! Reference source not found.,** wherein the at least one lubricious liner has a thickness from about 0.001" to about 0.005".

**EXAMPLE 221:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; an outer layer having a predetermined thickness and having an inner surface and outer surface, wherein a lubricious liner is disposed between the outer surface of the inner liner and the inner surface of the outer layer, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 222:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; a tie layer disposed at the inner surface of the sheet, such that when the sheet is in the spiral configuration, the tie layer is disposed between the overlapping portions and at at least an innermost surface of the lumen; and/or a tie layer disposed at the outer surface of the sheet such that when the sheet is in the spiral configuration, the tie layer is disposed between the overlapping portions and at an outermost surface of the inner liner; at least one lubricious liner disposed on the tie layer; and an outer layer having a predetermined thickness and having an inner surface and outer surface, wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 223:** The sheath of any examples herein, particularly examples **Error! Reference source not found.-Error! Reference source not found.** wherein the outer layer comprises a polyether block amide, a styrene-based elastomer, polyurethane, latex, copolymers thereof, blends thereof, or extrudates thereof.

**EXAMPLE 224:** The sheath of any examples herein, particularly example 15, wherein the outer layer comprises one or more layers.

**EXAMPLE 225:** The sheath of any examples herein, particularly example 16, wherein at least one layer comprises the styrene-based elastomer.

**EXAMPLE 226:** The sheath of any examples herein, particularly example 16 or 17, wherein at least one layer comprises polyurethane.

**EXAMPLE 227:** The sheath of any examples herein, particularly example 16 or 17, wherein at least one layer comprises a blend of the styrene-based elastomer and polyurethane.

**EXAMPLE 228:** The sheath of any examples herein, particularly examples 17-19, wherein the styrene-based elastomer has a Shore A durometer between 20A to 50A.

**EXAMPLE 229:** The sheath of any examples herein, particularly examples **Error! Reference source not found**.-16, wherein the outer layer further comprises a first polymer layer comprising a first compound composition comprising: from greater than 0 wt% to less than 100 wt% of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition.

**EXAMPLE 230:** The sheath of any examples herein, particularly example 21, wherein the polymer in the first polymer layer composition has a Shore D from about 20D to about 35D.

**EXAMPLE 231:** The sheath of any examples herein, particularly example 21 or 22, wherein the polymer in the first polymer layer composition comprises PEBAX^{®}.

**EXAMPLE 232:** The sheath of any examples herein, particularly example 21 or 22, wherein the first polymer layer composition comprises polyurethane.

**EXAMPLE 233:** The sheath of any examples herein, particularly examples 21-**Error! Reference source not found.,** wherein the inorganic filler comprises bismuth oxychloride, barium sulfate, bismuth subcarbonate, calcium carbonate, aluminum trihydrate, barite, kaolin clay, limestone, or any combination thereof.

**EXAMPLE 234:** The sheath of any examples herein, particularly examples 21-23, wherein the inorganic filler is present in an amount of at least about 10 % based on a total weight of the first compound composition.

**EXAMPLE 235:** The sheath of any examples herein, particularly examples 21-24, wherein the inorganic filler is present in an amount of less than about 50 % based on a total weight of the first compound composition.

**EXAMPLE 236:** The sheath of any examples herein, particularly examples 21-**Error! Reference source not found.,** wherein the solid lubricant comprises a PTFE filler.

**EXAMPLE 237:** The sheath of any examples herein, particularly example **Error! Reference source not found.,** wherein the PTFE filler is a powder.

**EXAMPLE 238:** The sheath of any examples herein, particularly examples 21-**Error! Reference source not found.,** wherein the first compound composition further comprises at least one tackiness reducing compound.

**EXAMPLE 239:** The sheath of any examples herein, particularly example 25, wherein the at least one tackiness reducing compound is present in an amount from about 1 % to about 20 % based on a total weight of the first compound composition.

**EXAMPLE 240:** The sheath of any examples herein, particularly example 25 or 26, wherein the at least one tackiness reducing compound comprises ProPell^{™}.

**EXAMPLE 241:** The sheath of any examples herein, particularly examples 21-**Error! Reference source not found.,** wherein the outer layer comprises two or more polymer layers.

**EXAMPLE 242:** The sheath of any examples herein, particularly example **Error! Reference source not found.,** wherein the outer layer comprises a second polymer layer comprising a second compound composition comprising from greater than 0 wt% to 100 wt% of a second polymer comprising polyether block amide, a polyurethane, or a composition thereof.

**EXAMPLE 243:** The sheath of any examples herein, particularly example 27, wherein the second compound composition further comprises up to 20 % of tackiness reducing additive based on a total weight of the second compound composition.

**EXAMPLE 244:** The sheath of any examples herein, particularly example 27 or **Error! Reference source not found.,** wherein the second polymer layer composition comprises PEBAX^{®}.

**EXAMPLE 245:** The sheath of any examples herein, particularly examples 27-**Error! Reference source not found.,** wherein the second polymer layer composition comprises polyurethane.

**EXAMPLE 246:** The sheath of any examples herein, particularly examples 27-**Error! Reference source not found.,** wherein the second polymer has a Shore D Durometer from about 20D to about 35D.

**EXAMPLE 247:** The sheath of any examples herein, particularly example 28, wherein the second polymer has a Shore D Durometer of about 25D to about 35D.

**EXAMPLE 248:** The sheath of any examples herein, particularly examples 27-**Error! Reference source not found.,** wherein the second compound composition is substantially free of an inorganic filler.

**EXAMPLE 249:** The sheath of any examples herein, particularly examples 27-29, wherein the second compound composition is substantially free of a lubricant solid.

**EXAMPLE 250:** The sheath of any examples herein, particularly examples 27-30, wherein the outer layer has a predetermined thickness, and wherein at least about 50% of the predetermined thickness comprises the first and/or the second compound composition comprising the first and/or the second polymer having a Shore D Durometer from about 20D to about 35D.

**EXAMPLE 251:** The sheath of any examples herein, particularly example **Error! Reference source not found.,** wherein the predetermined thickness is up to 6 mils.

**EXAMPLE 252:** The sheath of any examples herein, particularly example **Error! Reference source not found.** or **Error! Reference source not found.,** wherein the predetermined thickness of the outer layer varies along the length of the sheath.

**EXAMPLE 253:** The sheath of any examples herein, particularly example **Error! Reference source not found.,** wherein the predetermined thickness of the outer layer is greater at the proximal end.

**EXAMPLE 254:** The sheath of any examples herein, particularly example **Error! Reference source not found.** or **Error! Reference source not found.,** wherein the predetermined thickness of the outer layer is smaller at the distal end as compared to the predetermined thickness of the outer layer at the proximal end.

**EXAMPLE 255:** The sheath of any examples herein, particularly examples 21-**Error! Reference source not found.,** wherein the first polymer layer has a thickness of about 0.001" to about 0.003".

**EXAMPLE 256:** The sheath of any examples herein, particularly examples 27-**Error! Reference source not found.,** wherein the second polymer layer has a thickness of about 0.002" to about 0.004".

**EXAMPLE 257:** The sheath of any one of any examples herein, particularly example s 27-**Error! Reference source not found.,** wherein the first polymer layer defines an inner surface of the outer layer.

**EXAMPLE 258:** The sheath of any examples herein, particularly examples 27-**Error! Reference source not found.,** wherein the second polymer layer defines an outer surface of the outer layer.

**EXAMPLE 259:** The sheath of any examples herein, particularly examples 27-**Error! Reference source not found.,** wherein the first polymer layer defines an outer surface of the outer layer.

**EXAMPLE 260:** The sheath of any examples herein, particularly example **Error! Reference source not found.,** wherein the second polymer layer defines an inner surface of the outer layer.

**EXAMPLE 261:** The sheath of any examples herein, particularly examples 27-**Error! Reference source not found.,** wherein one or more additional polymer layers are disposed between the first polymer layer and the second polymer layer.

**EXAMPLE 262:** The sheath of any examples herein, particularly examples 21-**Error! Reference source not found.** or 23-**Error! Reference source not found.,** wherein the outer layer further comprises a second polymer layer.

**EXAMPLE 263:** The sheath of any examples herein, particularly example **Error! Reference source not found.,** wherein the second polymer layer comprises polyurethane or polyurethane blend.

**EXAMPLE 264:** The sheath of any examples herein, particularly example **Error! Reference source not found.** or **Error! Reference source not found.,** wherein the second layer of polyurethane and the first polymer layer are coextruded to form a bump tubing.

**EXAMPLE 265:** The sheath of any examples herein, particularly example **Error! Reference source not found.,** wherein the bump tubing has a predetermined length substantially similar to a length of the sheath.

**EXAMPLE 266:** The sheath of any examples herein, particularly examples **Error! Reference source not found. -Error! Reference source not found.,** wherein the first polymer layer defines an inner surface of the outer layer.

**EXAMPLE 267:** The sheath of any examples herein, particularly example **Error! Reference source not found.,** wherein the second polymer layer defines an outer surface of the outer layer.

**EXAMPLE 268:** The sheath of any examples herein, particularly examples **Error! Reference source not found.-Error! Reference source not found.,** wherein the first polymer layer has a thickness from about 0.001" to about 0.010".

**EXAMPLE 269:** The sheath of any examples herein, particularly example **Error! Reference source not found.,** wherein the thickness of the first polymer layer is uniform along the length of the sheath.

**EXAMPLE 270:** The sheath of any examples herein, particularly example **Error! Reference source not found.,** wherein the thickness of the first polymer layer varies along the length of the sheath.

**EXAMPLE 271:** The sheath of any examples herein, particularly example **Error! Reference source not found.,** wherein the thickness of the first polymer layer is greater at the proximal end of the sheath as compared to a thickness of the first polymer layer along other portions of the sheath.

**EXAMPLE 272:** The sheath of any examples herein, particularly example **Error! Reference source not found.** or **Error! Reference source not found.,** wherein the thickness of the first polymer layer is smaller at the distal end of the sheath as compared to a thickness of the first polymer layer at the proximal end of the sheath.

**EXAMPLE 273:** The sheath of any examples herein, particularly examples **Error! Reference source not found.-Error! Reference source not found.,** wherein the second polymer layer has a thickness from about 0.001" to about 0.010".

**EXAMPLE 274:** The sheath of any examples herein, particularly example **Error! Reference source not found.,** wherein the thickness of the second polymer layer is uniform along the length of the sheath.

**EXAMPLE 275:** The sheath of any examples herein, particularly example **Error! Reference source not found.,** wherein the thickness of the second polymer layer varies along the length of the sheath.

**EXAMPLE 276:** The sheath of any examples herein, particularly example **Error! Reference source not found.,** wherein the thickness of the second polymer layer is greater at the proximal end of the sheath as compared to the thickness of the second polymer layer along other portions of the sheath.

**EXAMPLE 277:** The sheath of any examples herein, particularly example **Error! Reference source not found.** or **Error! Reference source not found.,** the thickness of the second polymer layer is smaller at the distal end of the sheath as compared to the thickness of the second polymer layer at the proximal end of the sheath.

**EXAMPLE 278:** The sheath of any examples herein, particularly examples **Error! Reference source not found.-Error! Reference source not found.,** wherein the second polymer layer has a Shore A durometer from about 30A to about 80A.

**EXAMPLE 279:** The sheath of any examples herein, particularly examples **Error! Reference source not found.-Error! Reference source not found.,** wherein the outer layer has a total predetermined thickness, and wherein at least about 50% of the total predetermined thickness comprises the first compound composition comprising the first polymer having a Shore D Durometer from about 20D to about 35D.

**EXAMPLE 280:** The sheath of any examples herein, particularly example **Error! Reference source not found.,** wherein the predetermined thickness is up to 0.01".

**EXAMPLE 281:** The sheath of any examples herein, particularly example **Error! Reference source not found.** or **Error! Reference source not found.,** wherein the total predetermined thickness of the outer layer varies along the length of the sheath.

**EXAMPLE 282:** The sheath of any examples herein, particularly example **Error! Reference source not found.,** wherein the total predetermined thickness of the outer layer is greater at the proximal end of the sheath.

**EXAMPLE 283:** The sheath of any examples herein, particularly example **Error! Reference source not found.** or **Error! Reference source not found.,** wherein the total predetermined thickness of the outer layer is smaller at the distal end of the sheath as compared to the predetermined thickness of the outer layer at the proximal end of the sheath.

**EXAMPLE 284:** The sheath of any examples herein, particularly examples **Error! Reference source not found.-Error! Reference source not found.,** wherein the first polymer layer and the second polymer layer are formed separately and wherein the second polymer layer at least partially overlies the first polymer layer.

**EXAMPLE 285:** The sheath of any examples herein, particularly example **Error! Reference source not found.,** wherein the first polymer layer and the second polymer layer have a different length.

**EXAMPLE 286:** The sheath of any examples herein, particularly example **Error! Reference source not found.,** wherein the first polymer layer has a length that is shorter than the length of the second polymer layer.

**EXAMPLE 287:** The sheath of any examples herein, particularly example **Error! Reference source not found.** or **Error! Reference source not found.,** wherein the first polymer layer is disposed at the proximal end of the sheath and has a length from about 5 cm to about 15 cm.

**EXAMPLE 288:** The sheath of any examples herein, particularly examples **Error! Reference source not found.-Error! Reference source not found.,** wherein the second polymer layer has a length substantially identical to the length of the sheath.

**EXAMPLE 289:** The sheath of any examples herein, particularly examples **Error! Reference source not found.-Error! Reference source not found.,** wherein the first polymer layer has a thickness that is uniform along the length of the first polymer layer.

**EXAMPLE 290:** The sheath of any examples herein, particularly examples **Error! Reference source not found.-Error! Reference source not found.,** wherein the first polymer layer has a thickness that varies along the length of the first polymer layer.

**EXAMPLE 291:** The sheath of any examples herein, particularly example **Error! Reference source not found.** or **Error! Reference source not found.,** wherein the thickness of the first polymer layer is from about 0.001" to about 0.006".

**EXAMPLE 292:** The sheath of any examples herein, particularly examples **Error! Reference source not found.-Error! Reference source not found.,** wherein the second polymer layer has a thickness that is uniform along the length of the second polymer layer.

**EXAMPLE 293:** The sheath of any examples herein, particularly examples **Error! Reference source not found.-Error! Reference source not found.,** wherein the second polymer layer has a thickness that varies along the length of the second polymer layer.

**EXAMPLE 294:** The sheath of any examples herein, particularly example **Error! Reference source not found.** or **Error! Reference source not found.,** wherein the thickness of the second polymer layer is from about 0.001" to about 0.010".

**EXAMPLE 295:** The sheath of any examples herein, particularly examples **Error! Reference source not found.-Error! Reference source not found.,** wherein a tie layer is disposed between the first polymer layer and the second polymer layer.

**EXAMPLE 296:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an elongated tube forming an outer layer of the sheath that is positioned at at least the proximal end of the sheath and extending along at least a portion of a length of the sheath, having an inner surface and an outer surface, and wherein the elongated tube comprises a) a first polymer layer, wherein the first polymer layer comprises a first compound composition comprising i) from greater than 0 wt% to less than 100 wt% of a polymer comprising a polyether block amide, a polyurethane, or a combination thereof based on a total weight of the first compound composition; ii) less than about 65% of an inorganic filler based on a total weight of the first compound composition; and iii) up to about 20 % of a solid lubricant filler based on a total weight of the first compound composition; and b) a second polymer layer comprising polyurethane; wherein the elongated tubing is a bump tubing and is a coextrusion of the first and the second polymers; wherein the variable diameter inner liner is configured to expand from a first rest diameter dᵣ to a second expanded diameter dₑ by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 297:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an elongated tube forming an outer layer of the sheath that is positioned at at least the proximal end of the sheath and extending along at least a portion of a length of the sheath, having an inner surface and an outer surface, and wherein the elongated tube comprises a) a first polymer layer, wherein the first polymer layer comprises a first compound composition comprising i) from greater than 0 wt% to less than 100 wt% of a polymer comprising a polyether block amide, a polyurethane, or a combination thereof based on a total weight of the first compound composition; ii) less than about 65% of an inorganic filler based on a total weight of the first compound composition; and iii) up to about 20 % of a solid lubricant filler based on a total weight of the first compound composition; and b) a second polymer layer comprising polyurethane; wherein the second polymer layer at least partially overlies the first polymer layer; and wherein the first polymer is disposed at the proximal end of the sheath and has a length that is shorter than the length of the second polymer layer; and wherein the variable diameter inner liner is configured to expand from a first rest diameter *dᵣ* to a second expanded diameter dₑ by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 298:** The sheath of any examples herein, particularly examples **Error! Reference source not found.-Error! Reference source not found.,** wherein at least a portion of an innermost surface of the outer layer is bonded to at least a portion of an outermost surface of the inner liner.

**EXAMPLE 299**:The sheath of any examples herein, particularly example **Error! Reference source not found.,** wherein the at least a portion of an innermost surface of the outer layer is bonded to the at least a portion of an outermost surface of the inner liner by laser welding, compression bonding, and/or selective ultrasonic welding.

**EXAMPLE 300:** The sheath of any examples herein, particularly example **Error! Reference source not found.,** wherein the at least a portion of an outermost surface the inner liner that is bonded to the at least a portion of an innermost surface of the outer layer does not comprise a lubricant or a lubricant liner prior to bonding or wherein the at least a portion of an outermost surface the inner liner bonded to the at least a portion of an innermost surface of the outer layer does not comprise a tie layer and/or a lubricant liner prior to bonding.

**EXAMPLE 301:** The sheath of any examples herein, particularly example **Error! Reference source not found.** or **Error! Reference source not found.,** wherein the at least a portion of an innermost surface of the outer layer bonded to the at least a portion of an outermost surface of the inner liner extends longitudinally along a length of the sheath.

**EXAMPLE 302:** The sheath of any examples herein, particularly example **Error! Reference source not found.** or **Error! Reference source not found.,** wherein two or more portions of the innermost surface of the outer layer bonded to two or more portions of the outermost surface, the inner liner extends longitudinally along a length of the sheath.

**EXAMPLE 303:** The sheath of any examples herein, particularly example **Error! Reference source not found.,** wherein the two or more portions of the innermost surface of the outer layer bonded to two or more portions of the outermost surface of the inner liner are disposed in a predetermined pattern.

**EXAMPLE 304:** The sheath of any examples herein, particularly examples **Error! Reference source not found.-Error! Reference source not found.,** wherein the at least one portion of the outermost surface of the inner liner bonded to the at least one portion of the innermost surface of the outer liner is positioned at a portion of the outer surface of the sheet adjacent to the second longitudinal edge of the sheet when the sheet is in the collapsed spiral configuration.

**EXAMPLE 305:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface, wherein at least a portion of an innermost surface of the outer layer is bonded to at least a portion of an outermost surface the inner liner, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 306:** The sheath of any examples herein, particularly examples **Error! Reference source not found.-Error! Reference source not found.,** wherein the outer layer extends from the proximal end of the sheath to the distal end of the sheath.

**EXAMPLE 307:** The sheath of any examples herein, particularly example **Error! Reference source not found.,** further comprising a reinforcing jacket having a proximal end and a distal end and wherein the reinforcing jacket is disposed over at least a portion of an outer surface of the outer layer.

**EXAMPLE 308:** The sheath of any examples herein, particularly example **Error! Reference source not found.,** wherein the proximal end of the reinforcing jacket is abutting the proximal end of the sheath.

**EXAMPLE 309:** The sheath of any examples herein, particularly example **Error! Reference source not found.** or **Error! Reference source not found.,** wherein the reinforcing jacket has a length of about 5 to about 15 cm.

**EXAMPLE 310:** The sheath of any examples herein, particularly examples **Error! Reference source not found.-Error! Reference source not found.,** wherein the distal end of the reinforcing jacket is substantially seamlessly bonded to the at least a portion of the outer surface of the outer layer.

**EXAMPLE 311:** The sheath of any examples herein, particularly examples **Error! Reference source not found.-Error! Reference source not found.,** wherein the reinforcing jacket comprises an elastomeric material having a Shore hardness of about 10A to about 80A.

**EXAMPLE 312:** The sheath of any examples herein, particularly examples **Error! Reference source not found.-Error! Reference source not found.,** wherein the elastomeric material comprises a polyether block amide, styrene-based elastomer, polyurethane, latex, copolymers thereof, blends thereof or co-extrudates thereof.

**EXAMPLE 313:** The sheath of any examples herein, particularly examples **Error! Reference source not found.-Error! Reference source not found.,** wherein the reinforcing jacket comprises a reinforcing element.

**EXAMPLE 314:** The sheath of any examples herein, particularly example **Error! Reference source not found.,** wherein the reinforcing element is embedded in the elastomeric material.

**EXAMPLE 315:** The sheath of any examples herein, particularly examples **Error! Reference source not found.** or **Error! Reference source not found.,** wherein the reinforcing element limits expansion of the outer layer to a predetermined diameter effective to prevent ballooning of the outer layer of the sheath and thereby to maintain hemostasis.

**EXAMPLE 316:** The sheath of any examples herein, particularly examples **Error! Reference source not found.-Error! Reference source not found.,** wherein the reinforcing element comprises a plurality of filaments arranged in a braid or coil configuration.

**EXAMPLE 317:** The sheath of any examples herein, particularly examples **Error! Reference source not found.-Error! Reference source not found.,** wherein the reinforcing element comprises a plurality of filaments disposed in a plurality of circumferential rows within the reinforcing jacket, wherein each of plurality has a sinusoidal form or any irregular form, or any combination thereof.

**EXAMPLE 318:** The sheath of any examples herein, particularly example **Error! Reference source not found.** or **Error! Reference source not found.,** wherein the plurality of filaments comprise stainless steel, nitinol, a polymer material, or composite material.

**EXAMPLE 319:** The sheath of any examples herein, particularly example **Error! Reference source not found.,** wherein the filament is a round filament or a flat filament.

**EXAMPLE 320:** The sheath of any examples herein, particularly example **Error! Reference source not found.** or **Error! Reference source not found.,** wherein the polymer material is polyester or nylon.

**EXAMPLE 321:** The sheath of any examples herein, particularly examples **Error! Reference source not found.-Error! Reference source not found.,** wherein the round filament has a diameter of less than about 0.015".

**EXAMPLE 322:** The sheath of any examples herein, particularly examples **Error! Reference source not found.-Error! Reference source not found.,** wherein the flat filament has a height of less than about 0.006" and a width from greater than about 0.003" to about 0.015".

**EXAMPLE 323:** The sheath of any examples herein, particularly examples **Error! Reference source not found.-Error! Reference source not found.,** wherein the braid has a per inch crosses (PIC) count of less than 50.

**EXAMPLE 324:** The sheath of any examples herein, particularly examples **Error! Reference source not found.-Error! Reference source not found.,** wherein the proximal end of the reinforcing jacket is not bonded to the outer layer.

**EXAMPLE 325:** The sheath of any examples herein, particularly examples **Error! Reference source not found.-Error! Reference source not found.,** wherein a tie layer is disposed between the outer layer and the reinforcing jacket.

**EXAMPLE 326:** The sheath of any examples herein, particularly examples **Error! Reference source not found.-Error! Reference source not found.,** wherein the outer layer does not extend to the proximal end of the sheath and wherein at least a portion of the inner liner at the proximal end of the sheath is substantially free of the outer layer.

**EXAMPLE 327:** The sheath of any examples herein, particularly example **Error! Reference source not found.,** wherein the at least a portion of the inner liner at the proximal end of the sheath that is substantially free of the outer layer is from about 5 cm to about 15 cm.

**EXAMPLE 328:** The sheath of any examples herein, particularly example **Error! Reference source not found.** or **Error! Reference source not found.,** wherein a proximal end of the outer layer is bonded to at least a portion of the outer surface of the inner liner.

**EXAMPLE 329:** The sheath of any examples herein, particularly examples **Error! Reference source not found.-Error! Reference source not found.,** further comprising a reinforcing jacket having a proximal end and a distal end and is disposed over the at least a portion of the outer surface of the inner liner at the proximal end of the sheath that is substantially free of the outer layer.

**EXAMPLE 330:** The sheath of any examples herein, particularly example **Error! Reference source not found.,** wherein the proximal end of the reinforcing jacket is abutting the proximal end of the sheath.

**EXAMPLE 331:** The sheath of any examples herein, particularly example **Error! Reference source not found.** or **Error! Reference source not found.,** wherein the proximal end of the reinforcing jacket is at least partially bonded to at least a portion of a proximal end of the outer surface of the inner liner.

**EXAMPLE 332:** The sheath of any examples herein, particularly examples **Error! Reference source not found.-Error! Reference source not found.,** wherein the reinforcing jacket has a length of about 5 to about 15 cm.

**EXAMPLE 333:** The sheath of any examples herein, particularly examples **Error! Reference source not found.-Error! Reference source not found.,** wherein the distal end of the reinforcing jacket is abutting or at least partially overlays a proximal portion of the outer layer.

**EXAMPLE 334:** The sheath of any examples herein, particularly example **Error! Reference source not found.,** wherein the distal end of the reinforcing jacket is seamlessly bonded to the at least a portion of the proximal portion of the outer surface of the outer layer.

**EXAMPLE 335:** The sheath of any examples herein, particularly examples **Error! Reference source not found.-Error! Reference source not found.,** wherein the reinforcing jacket comprises an elastomeric material having a Shore hardness of about 10A to about 80A.

**EXAMPLE 336:** The sheath of any examples herein, particularly examples **Error! Reference source not found.-Error! Reference source not found.,** wherein the elastomeric material comprises a polyether block amide, styrene-based elastomer, polyurethane, latex, copolymers thereof, blends thereof or co-extrudates thereof.

**EXAMPLE 337:** The sheath of any examples herein, particularly examples **Error! Reference source not found.-Error! Reference source not found.,** wherein the reinforcing jacket comprises a reinforcing element.

**EXAMPLE 338:** The sheath of any examples herein, particularly example **Error! Reference source not found.,** wherein the reinforcing element is embedded in the elastomeric material.

**EXAMPLE 339:** The sheath of any examples herein, particularly examples **Error! Reference source not found.** or **Error! Reference source not found.,** wherein the reinforcing element limits expansion of the outer layer to a predetermined diameter effective to prevent ballooning of the outer layer of the sheath.

**EXAMPLE 340:** The sheath of any examples herein, particularly examples **Error! Reference source not found.-Error! Reference source not found.,** wherein the reinforcing element comprises a plurality of filaments arranged in a braid or coil configuration.

**EXAMPLE 341:** The sheath of any examples herein, particularly examples **Error! Reference source not found.-Error! Reference source not found.,** wherein the reinforcing element comprises a plurality of filaments disposed in a plurality of circumferential rows within the reinforcing jacket, wherein each of plurality has a sinusoidal form or any irregular form, or any combination thereof.

**EXAMPLE 342:** The sheath of any examples herein, particularly example **Error! Reference source not found.** or **Error! Reference source not found.,** wherein the plurality of filaments comprise stainless steel, nitinol, a polymer material, or composite material.

**EXAMPLE 343:** The sheath of any examples herein, particularly example **Error! Reference source not found.,** wherein the filament is a round filament or a flat filament.

**EXAMPLE 344:** The sheath of any examples herein, particularly example **Error! Reference source not found.** or **Error! Reference source not found.,** wherein the polymer material is polyester or nylon.

**EXAMPLE 345:** The sheath of any examples herein, particularly examples **Error! Reference source not found.-Error! Reference source not found.,** wherein the round filament has a diameter of less than about 0.015".

**EXAMPLE 346:** The sheath of any examples herein, particularly examples **Error! Reference source not found.-Error! Reference source not found.,** wherein the flat filament has a height of less than about 0.006" and a width from greater than about 0.003" to about 0.015".

**EXAMPLE 347:** The sheath of any examples herein, particularly examples **Error! Reference source not found.-Error! Reference source not found.,** wherein the braid has a per inch crosses (PIC) count of less than 50.

**EXAMPLE 348:** The sheath of any examples herein, particularly examples **Error! Reference source not found.-Error! Reference source not found.,** wherein a tie layer is disposed between the inner liner and the reinforcing jacket.

**EXAMPLE 349:** The sheath of any examples herein, particularly examples **Error! Reference source not found.-Error! Reference source not found.,** wherein at least a portion of the reinforcing jacket is configured to be inserted into a subject's blood vessel.

**EXAMPLE 350:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface; and a reinforcing jacket having a proximal end and a distal end and wherein the reinforcing jacket is disposed over at least a portion of an outer surface of the outer layer; wherein the reinforcing jacket comprises an elastomer and a reinforcing element; and wherein the distal end of the reinforcing jacket substantially seamlessly bonded to at least a portion of the outer surface of the outer layer; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 351:** The sheath of any examples herein, particularly examples **Error! Reference source not found.-Error! Reference source not found.,** wherein the sheath further comprises a ballooning guard having a proximal end and a distal end and is disposed over at least a portion of the outer layer and wherein the ballooning guard is configured to remain outside of a subject's vessel and to substantially maintain hemostasis.

**EXAMPLE 352:** The sheath of any examples herein, particularly example **Error! Reference source not found.,** wherein the proximal end of the ballooning guard is connected to a most proximal portion of the outer layer and/or a hub of the sheath.

**EXAMPLE 353:** The sheath of any examples herein, particularly example **Error! Reference source not found.** or **Error! Reference source not found.,** wherein the distal end of the ballooning guard radially circumscribes at least a portion of the outer layer and wherein the distal end is not bonded to the outer layer.

**EXAMPLE 354:** The sheath of any examples herein, particularly examples **Error! Reference source not found.-Error! Reference source not found.,** wherein the distal end of the ballooning guard is configured to seal the sheath against a subject's skin.

**EXAMPLE 355:** The sheath of any examples herein, particularly examples **Error! Reference source not found.-Error! Reference source not found.,** wherein the ballooning guard is configured to adjust a length of the guard as a function of an insertion depth of the inner liner and outer layer of the sheath into a subject's vessel.

**EXAMPLE 356:** The sheath of any examples herein, particularly examples **Error! Reference source not found.-Error! Reference source not found.,** wherein the ballooning guard has a collapsible length.

**EXAMPLE 357:** The sheath of any examples herein, particularly examples **Error! Reference source not found.-Error! Reference source not found.,** wherein the ballooning guard has an inner diameter sized to have substantially no resistance to expansion of the outer layer to a predetermined diameter such that there is substantially no increase in a push force to advance the delivery system through the sheath.

**EXAMPLE 358:** The sheath of any examples herein, particularly example **Error! Reference source not found.,** wherein at the predetermined diameter of the outer layer, the ballooning guard is configured to resist to a blood pressure increase.

**EXAMPLE 359:** The sheath of any examples herein, particularly examples **Error! Reference source not found.-Error! Reference source not found.,** wherein the ballooning guard comprises a braided sleeve comprising a plurality of filaments.

**EXAMPLE 360:** The sheath of any examples herein, particularly example **Error! Reference source not found.,** wherein at least a portion of the filaments at the distal end of the ballooning guard are connected to each other.

**EXAMPLE 361:** The sheath of any examples herein, particularly examples **Error! Reference source not found.** or 360 wherein the braided or coiled sleeve comprises a polymeric sleeve.

**EXAMPLE 362:** The sheath of any examples herein, particularly examples **Error! Reference source not found.** or 360, wherein the braided or coiled sleeve comprises a fabric.

**EXAMPLE 363:** The sheath of any examples herein, particularly examples **Error! Reference source not found.-Error! Reference source not found.,** wherein the ballooning guard comprises the e-PTFE tubing, corrugated tubing, or any polymeric tubing having a shape that is configured to be compressed.

**EXAMPLE 364:** The sheath of any examples herein, particularly examples **Error! Reference source not found.-Error! Reference source not found.,** wherein the ballooning guard comprises a corrugated tubing.

**EXAMPLE 365:** A sheath for delivering a medical device, wherein the sheath has a proximal and a distal end and comprises: a variable diameter inner liner comprising a sheet rolled into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and an outer layer having a predetermined thickness and having an inner surface and outer surface and extending from the proximal end of the sheath to the distal end of the sheath; and a ballooning guard having a proximal end and a distal end and is disposed over at least a portion of the outer layer and wherein the ballooning guard is configured to remain outside of a subject's vessel and to maintain hemostasis; wherein the proximal end of the ballooning guard is connected to a most proximal portion of the outer layer and/or a hub of the sheath, wherein the distal end of the ballooning guard radially circumscribes at least a portion of the outer layer and wherein the distal end is not bonded to the outer layer; and wherein the ballooning guard is configured to adjust a length of the guard as a function of an insertion depth of the inner liner and outer layer of the sheath into a subject's vessel.

**EXAMPLE 366:** The sheath of any examples herein, particularly examples **Error! Reference source not found.-Error! Reference source not found.,** further comprising a hydrophilic coating disposed at an outermost surface of the sheath.

**EXAMPLE 367:** A method of making a sheath having a proximal and a distal end and comprising: a) forming an inner liner by: i) providing a double lumen tubing comprising two channels extruded in a single-tube configuration; wherein the double lumen comprises at least one polymer layer; wherein a first channel has an inner surface and an outer surface; wherein a second channel has an inner surface and an outer surface; wherein the second channel is positioned within the first channel such that at least a portion of a circumference of the first channel and at least a portion of a circumference of the second channel have at least one shared inner surface and at least one shared outer surface; and wherein the outer surface of the first channel defines an outer surface of the double-lumen tubing; ii) longitudinally cutting the first channel at a portion of the circumference of the first channel that is not shared with the circumference of the second channel to form a first sheet having a first edge and a second edge and the second channel disposed longitudinally along at least a portion of the first sheet; such that at least a portion of the circumference of the second channel has at least a portion that has a shared surface with the first sheet along a length of the sheet and along a length of the second channel; iii) longitudinally cutting the second channel at a portion of the circumference of the second channel that abuts the shared surface with the first sheet to form a second sheet having a first edge and a second edge, wherein the second edge is defined by a portion of the shared surface with the first sheet; iv) rolling the first and the second sheets into a spiral configuration such that: I) the shared surface between the second and the first sheets form a first portion of an inner liner having a first surface and an opposite second surface; II) at least a portion of the second sheet forms a first segment of an inner liner having a first surface and an opposite second surface; III) wherein a portion of the first sheet adjacent to the second end of the second sheet and extending to the first end of the first sheet forms a second segment of an inner liner having a first surface and an opposite second surface; and IV) wherein a portion of the first sheet adjacent the second end of the second sheet and extending to the second end of the first sheet forms a third segment of an inner liner having a first surface and an opposite second surface; wherein in the spiral configuration, at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment; wherein the first surface of the first portion extends into the first surface of the first segment, the second segment and the third segment, and wherein the second surface of the first portion extends into the second surface of the second and the third segments, and b) disposing an outer layer to form the sheath; wherein the formed sheath is configured to extend from a first rest diameter *dᵣ* to a second expanded diameter *dₑ* and back to a diameter substantially identical to the first rest diameter dᵣ upon passage of the medical device by slidably moving each segment along each other.

**EXAMPLE 368:** The method of any examples herein, particularly example **Error! Reference source not found.,** wherein the step of forming the variable diameter inner liner comprises rolling the sheet over a mandrel having a predetermined diameter to form the spiral configuration, wherein the predetermined diameter of the mandrel is substantially identical to the predetermined diameter dᵣ of the inner liner.

**EXAMPLE 369:** The method of any examples herein, particularly example **Error! Reference source not found.** or **Error! Reference source not found.,** wherein the at least a portion of the first surface of the second segment overlaps the at least a portion of the second surface of the first segment such that a first gap is formed between the at least a portion of the first surface of the second segment and the at least a portion of the second surface of the first segment.

**EXAMPLE 370:** The method of any examples herein, particularly examples **Error! Reference source not found.-Error! Reference source not found.,** wherein the at least a portion of the first surface of the third segment overlaps the at least a portion of the second surface of the second segment such that a second gap is formed between the at least a portion of the first surface of the third segment and the at least a portion of the second surface of the second segment.

**EXAMPLE 371:** The method of any examples herein, particularly examples **Error! Reference source not found.-Error! Reference source not found.,** wherein the at least a portion of the first surface of the third segment overlaps the at least a portion of the second surface of the first segment such that a third gap is formed between the at least a portion of the first surface of the third segment and the at least a portion of the second surface of the first segment.

**EXAMPLE 372:** The method of any examples herein, particularly examples **Error! Reference source not found.-Error! Reference source not found.,** wherein the first gap has a substantially uniform width along overlapping portions.

**EXAMPLE 373:** The method of any examples herein, particularly examples **Error! Reference source not found.-Error! Reference source not found.,** wherein the first gap has a variable width along overlapping portions.

**EXAMPLE 374:** The method of any examples herein, particularly examples **Error! Reference source not found.-Error! Reference source not found.,** wherein the second gap has a substantially uniform width along overlapping portions.

**EXAMPLE 375:** The method of any examples herein, particularly examples **Error! Reference source not found.-Error! Reference source not found.,** wherein the second gap has a variable width along overlapping portions.

**EXAMPLE 376:** The method of any examples herein, particularly examples **Error! Reference source not found.-Error! Reference source not found.,** wherein the third gap has a substantially uniform width along overlapping portions.

**EXAMPLE 377:** The method of any examples herein, particularly examples **Error! Reference source not found.-Error! Reference source not found.,** wherein the third gap has a variable width along overlapping portions.

**EXAMPLE 378:** The method of any examples herein, particularly examples **Error! Reference source not found.-Error! Reference source not found.,** wherein at least a portion of the second segment is positioned within at least a portion of the third gap.

**EXAMPLE 379:** The method of any examples herein, particularly examples **Error! Reference source not found.-Error! Reference source not found.,** wherein when the medical device passes through the sheath, the first segment, the second segment, and the third segment are configured to slidably move along each other such that an overlapping portion between the first segment and the second segment and between the second segment and the third segment decreases, while an overlapping portion between the first segment and the third segment increases.

**EXAMPLE 380:** The method of any examples herein, particularly example **Error! Reference source not found.,** wherein a diameter of the sheath increases from the first rest diameter *dᵣ* to the second expanded diameter *dₑ*.

**EXAMPLE 381:** The method of any examples herein, particularly examples **Error! Reference source not found.-Error! Reference source not found.,** wherein after passage of the medical device through the sheath, the first segment, the second segment, and the third segment are configured to slidably move back along each other such that the overlapping portion between the first segment and the second segment, between the second segment and the third segment, and between the first segment and the third segment increases.

**EXAMPLE 382:** The method of any examples herein, particularly example **Error! Reference source not found.,** wherein the diameter of the sheath decreases from the second expanded diameter *dₑ* to a diameter that is substantially identical to the first rest diameter *dᵣ*.

**EXAMPLE 383:** A method of making a sheath having a proximal and a distal end and comprising: a) forming a variable diameter inner liner by: i) providing an elongated single lumen tubing comprising at least one polymer layer; ii) longitudinally cutting at least a portion of a circumference of the elongated single lumen tubing to form a sheet having a first longitudinal edge and an opposite second longitudinal edge and having an inner surface and an outer surface; iii) forming a variable diameter inner liner by rolling the sheet in a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; and b) disposing an outer layer over at least a portion of the outer layer of the inner liner to form the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

**EXAMPLE 384:** The method of any examples herein, particularly example **Error! Reference source not found.,** wherein the step of forming the variable diameter inner liner comprises rolling the sheet over a mandrel having a predetermined diameter to form the spiral configuration, wherein the predetermined diameter of the mandrel is substantially identical to the predetermined diameter dᵣ of the inner liner.

**EXAMPLE 385:** The method of any examples herein, particularly example **Error! Reference source not found.,** wherein the first rest diameter *dᵣ* is substantially uniform along the longitudinal axis of the lumen.

**EXAMPLE 386:** The method of any examples herein, particularly example **Error! Reference source not found.,** wherein the rest diameter *dᵣ* varies along the longitudinal axis of the lumen and wherein the rest diameter *dᵣ* at the proximal end that is larger than the rest diameter *dᵣ* at the distal end.

**EXAMPLE 387:** The method of any examples herein, particularly examples **Error! Reference source not found.-Error! Reference source not found.,** wherein the expanded diameter *dₑ* is configured to accommodate the medical device passing through the lumen.

**EXAMPLE 388:** The method of any examples herein, particularly examples **Error! Reference source not found.-Error! Reference source not found.,** wherein the sheath contracts to the predetermined rest diameter *dᵣ* after passage of the medical device through the lumen.

**EXAMPLE 389:** The method of any examples herein, particularly examples **Error! Reference source not found.-Error! Reference source not found.,** wherein when the sheath is in an unexpanded rest state, the first and the second edges of the sheath are substantially aligned in a spaced relationship along a vertical axis passing through a thickness of the sheath.

**EXAMPLE 390:** The method of any examples herein, particularly example **Error! Reference source not found.,** wherein the spaced relationship comprises a portion of the sheet positioned between the first edge and the second edge along the vertical axis.

**EXAMPLE 391:** The method of any examples herein, particularly example **Error! Reference source not found.** or **Error! Reference source not found.,** wherein when the sheath is in an unexpanded rest state the sheet, the inner liner comprises at least two layers of the sheet overlaying each other along at least a portion of a sheath's circumference.

**EXAMPLE 392:** The method of any examples herein, particularly example **Error! Reference source not found.,** wherein the at least a portion of the sheath's circumference comprises three layers of the sheet overlaying each other.

**EXAMPLE 393:** The method of any examples herein, particularly examples **Error! Reference source not found.-Error! Reference source not found.,** wherein when the sheath is in a rest unexpended state, the first edge of the sheet is substantially aligned with a vertical axis passing through a thickness of the sheath and the second edge circumferentially offset from the vertical axis.

**EXAMPLE 394:** The method of any examples herein, particularly example **Error! Reference source not found.,** wherein at at least a portion of a circumference of the sheath, the inner liner comprises one layer of the sheet without any overlaying portion.

**EXAMPLE 395:** The method of any examples herein, particularly examples **Error! Reference source not found.-Error! Reference source not found.,** further comprising disposing an amount of a lubricant, wherein the lubricant is disposed at at least a portion of the outer surface of the double-lumen tubing prior to the step of cutting the at least a portion of the circumference of the first channel.

**EXAMPLE 396:** The method of any examples herein, particularly examples **Error! Reference source not found.-Error! Reference source not found.** or **Error! Reference source not found.,** further comprising disposing an amount of a lubricant, wherein the lubricant is disposed at at least a portion of the outer surface of the second channel prior to the step of cutting the at least a portion of the circumference of the second channel.

**EXAMPLE 397:** The method of any examples herein, particularly examples **Error! Reference source not found.-Error! Reference source not found.,** further comprising disposing an amount of a lubricant, wherein the lubricant is disposed at at least a portion of the outer surface of the elongated tubing prior to the step of cutting the at least a portion of the circumference of the tubing.

**EXAMPLE 398:** The method of any examples herein, particularly examples **Error! Reference source not found.-Error! Reference source not found.,** wherein the step of disposing of the amount of the lubricant comprises disposing the lubricant in a predetermined pattern.

**EXAMPLE 399:** The method of any examples herein, particularly example **Error! Reference source not found.,** wherein prior to the step of disposing the amount of lubricant, the double lumen tubing, the first channel, or the elongated tubing are positioned on a mandrel.

**EXAMPLE 400:** The method of any examples herein, particularly example **Error! Reference source not found.,** wherein the step of disposing the amount of lubricant comprises: first disposing the lubricant into at least one recessed conduit on a plate; wherein the recessed conduit has a predetermined length, width, and depth, then a) contacting the plate with a pad, thereby transferring the lubricant on the pad; and then b) contacting the pad with a predetermined portion of the outer surface of the double lumen tubing, the first channel, or the elongated tubing under a predetermined pressure to transfer the lubricant to the predetermined portion of the outer surface.

**EXAMPLE 401:** The method of any examples herein, particularly example **Error! Reference source not found.,** wherein at least step c) is repeated to form the predetermined pattern of the disposed lubricant.

**EXAMPLE 402:** The method of any examples herein, particularly examples **Error! Reference source not found.-Error! Reference source not found.,** wherein the lubricant disposed into the conduit on the plate, has a viscosity between about 600 cP to about 1,200 cP.

**EXAMPLE 403:** The method of any examples herein, particularly examples **Error! Reference source not found.-Error! Reference source not found.,** further comprises a step of curing the lubricant.

**EXAMPLE 404:** The method of any examples herein, particularly example **Error! Reference source not found.,** wherein the amount of the lubricant is sprayed on a predetermined portion of the outer surface of the double lumen tubing, the first channel, or the elongated tubing.

**EXAMPLE 405:** The method of any examples herein, particularly example **Error! Reference source not found.,** wherein the lubricant has a viscosity of equal to or less than about 600 cP.

**EXAMPLE 406:** The method of any examples herein, particularly example **Error! Reference source not found.** or **Error! Reference source not found.,** wherein the lubricant is sprayed in a predetermined pattern.

**EXAMPLE 407:** The method of any examples herein, particularly examples **Error! Reference source not found.-Error! Reference source not found.,** further comprising a step of curing the lubricant.

**EXAMPLE 408:** The method of any examples herein, particularly examples **Error! Reference source not found.-Error! Reference source not found.,** wherein the lubricant comprises a PTFE-based lubricant or a silicone-based lubricant.

**EXAMPLE 409:** The method of any examples herein, particularly examples **Error! Reference source not found.-Error! Reference source not found.** or **Error! Reference source not found.-Error! Reference source not found.,** further comprises disposing an amount of a lubricant, wherein the lubricant is disposed at at least a portion of the outer surface of the second channel after the step of cutting the at least a portion of the circumference of the first channel and prior to the step of cutting the at least a portion of the circumference of the second channel.

**EXAMPLE 410:** The method of any examples herein, particularly examples **Error! Reference source not found.-Error! Reference source not found.** or **Error! Reference source not found.-Error! Reference source not found.,** wherein when the inner liner is in the spiral configuration, the lubricant is disposed on at least a portion of the first surface of the first portion, at least a portion of the first surface of the first segment, and/or at least a portion of the first surface of the second segment that does not overlap the at least a portion of the second surface of the first segment.

**EXAMPLE 411:** The method of any examples herein, particularly examples **Error! Reference source not found.-Error! Reference source not found.** or **Error! Reference source not found.-Error! Reference source not found.,** wherein when the inner liner is in the spiral configuration, the lubricant is disposed in an overlapping portion between the at least a portion of the first surface of the second segment and the at least a portion of the second surface of the first segment.

**EXAMPLE 412:** The method of any examples herein, particularly examples **Error! Reference source not found.-Error! Reference source not found.** or **Error! Reference source not found.-Error! Reference source not found.,** wherein when the inner liner is in the spiral configuration, the lubricant is disposed in an overlapping portion between the at least a portion of the first surface of the third segment and the at least a portion of the second surface of the second segment.

**EXAMPLE 413:** The method of any examples herein, particularly examples **Error! Reference source not found.-Error! Reference source not found.** or **Error! Reference source not found.-Error! Reference source not found.,** wherein when the inner liner is in the spiral configuration, the lubricant is disposed in an overlapping portion between the at least a portion of the first surface of the third segment and the at least a portion of the second surface of the first segment.

**EXAMPLE 414:** The method of any examples herein, particularly example **Error! Reference source not found.,** wherein the lubricant is disposed in an overlapping portion between the at least a portion of the first surface of the third segment and the at least a portion of the second surface of the first segment.

**EXAMPLE 415:** The method of any examples herein, particularly examples **Error! Reference source not found.-Error! Reference source not found.** or **Error! Reference source not found.-Error! Reference source not found.,** wherein the lubricant is disposed on at least a portion of the second surface of the first portion, at least a portion of the second surface of the third segment, and/or at least a portion of the second surface of the second segment that is not overlapped by the at least a portion of the first surface of the third segment.

**EXAMPLE 416:** The method of any examples herein, particularly examples **Error! Reference source not found.-Error! Reference source not found.,** wherein the lubricant is disposed at at least a portion of an outermost surface of the sheet.

**EXAMPLE 417:** The method of any examples herein, particularly examples **Error! Reference source not found.-Error! Reference source not found.** or **Error! Reference source not found.,** the lubricant is disposed between at least a portion of the overlying portion of the sheet and at least a portion of the sliding portions of the sheet.

**EXAMPLE 418:** The method of any examples herein, particularly example **Error! Reference source not found.,** wherein the lubricant is disposed along at least a portion of the inner surface of the sheet.

**EXAMPLE 419:** The method of any examples herein, particularly examples **Error! Reference source not found.-Error! Reference source not found.** or **Error! Reference source not found.-Error! Reference source not found.,** wherein the lubricant is disposed along at least a portion of the outer surface of the sheet.

**EXAMPLE 420:** The method of any examples herein, particularly examples **Error! Reference source not found.-Error! Reference source not found.,** wherein the lubricant forms a film having a thickness of equal to or less than about 20 µm.

**EXAMPLE 421:** A method of forming a sheath comprising: a) positioning an elongated tubing on a mandrel; b) disposing an amount of a lubricant on an outer surface of the elongated tubing in a predetermined pattern; c) curing the lubricant; d) cutting an elongated tubing at a portion of a circumference of the tubing along a length of the tubing to form a sheet having an outer surface and inner surface and a first longitudinal edge and a second longitudinal edge; wherein the inner surface forms a sheath lumen, and at least a portion of the outer surface comprises the lubricant disposed in the predetermined pattern; e) rolling the sheet into a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet; f) disposing an outer layer having an inner surface and an outer surface over the outer surface of the inner liner such that the lubricant is disposed between at least a portion of the inner surface of the outer layer and at least a portion of the outer surface of the inner layer to form the sheath; and wherein the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

**EXAMPLE 422:** The method of any examples herein, particularly examples **Error! Reference source not found.-Error! Reference source not found.,** wherein the sheet comprises the at least one polymer comprising a polyolefin, a polyamide, a fluoropolymer, copolymers thereof, co-extrudates thereof, or blends thereof.

**EXAMPLE 423:** The method of any examples herein, particularly examples **Error! Reference source not found.-Error! Reference source not found.,** wherein the sheet comprises the at least one polymer layer comprising a compound material comprising a polyolefin and a lubricious filler.

**EXAMPLE 424:** The method of any examples herein, particularly example **Error! Reference source not found.** or **Error! Reference source not found.,** wherein the polyolefin is high-density polyethylene.

**EXAMPLE 425:** The method of any examples herein, particularly example **Error! Reference source not found.** or **Error! Reference source not found.,** wherein the lubricous filler comprises a polytetrafluoroethylene (PTFE) filler.

**EXAMPLE 426:** The method of any examples herein, particularly examples **Error! Reference source not found.-Error! Reference source not found.,** wherein the lubricious filler is present in an amount from about 5 wt % to about 20 wt % of a total weight of the compound material.

**EXAMPLE 427:** The method of any examples herein, particularly examples **Error! Reference source not found.-Error! Reference source not found.,** wherein the sheet is lubricious and has a coefficient of friction less than about 0.5.

**EXAMPLE 428:** The method of any examples herein, particularly examples **Error! Reference source not found.-Error! Reference source not found.,** wherein the polymer layer of the sheet comprises the compound material, the sheath is substantially free of a separately disposed lubricant.

**EXAMPLE 429:** The method of any examples herein, particularly example **Error! Reference source not found.,** wherein the sheath exhibits a push force that is comparable or smaller than a push force of a substantially identical reference sheath rolled in a spiral configuration, wherein an inner liner of the substantially identical reference sheath comprises a polymer layer substantially free of a lubricious filler and comprises an amount of a lubricant disposed between overlapping portions of the spiral configuration and/or an outermost surface of the inner liner.

**EXAMPLE 430:** A method of making a sheath having a proximal end and a distal end and comprising: a) extruding a tubular body to form an elongated tubing comprising a compound material, wherein the compound material comprises a polyolefin present in an amount from greater than 0 wt% to less than 100 wt % based on a total weight of the compound and a lubricious filler present in an amount from about 5 wt % to about 20 wt% of a total weight to the compound material; b) cutting the elongated tubing at at least a portion of the circumference along a length of the elongated tubing to form a sheet; c) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; d) positioning an outer layer having a predetermined thickness and having an inner surface and outer surface, wherein the inner surface of the outer layer is disposed on the outer surface of the spiral configuration of the inner liner; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 431:** The method of any examples herein, particularly examples **Error! Reference source not found.-Error! Reference source not found.,** wherein the at least one polymer layer of the sheet is coextruded with at least one tie layer.

**EXAMPLE 432:** The method of any examples herein, particularly example **Error! Reference source not found.,** wherein the coextruded tie layer is disposed at the first surface of the first portion, the first surface of the first segment, the first surface of the second segment, and the first surface of the third segment, such that when the sheet is in the spiral configuration, the tie layer is disposed between the overlapping portions and at at least an inner surface of the lumen.

**EXAMPLE 433:** The method of any examples herein, particularly example **Error! Reference source not found.** or **Error! Reference source not found.,** wherein the coextruded tie layer is disposed at the inner surface of the sheet, such that when the sheet is in the spiral configuration, the tie layer is disposed between the overlapping portions and at at least an inner surface of the lumen.

**EXAMPLE 434:** The method of any examples herein, particularly examples **Error! Reference source not found.-Error! Reference source not found.,** wherein the coextruded tie layer is disposed at the second surface of the first portion, the second surface of the first segment, the second surface of the second segment, and the second surface of the third segment, such that when the sheet is in the spiral configuration, the tie layer is disposed between the overlapping portions and at an outermost surface of the inner liner.

**EXAMPLE 435:** The method of any examples herein, particularly examples **Error! Reference source not found.** or **Error! Reference source not found.,** wherein the coextruded tie layer is disposed at the outer surface of the sheet such that when the sheet is in the spiral configuration, the tie layer is disposed between the overlapping portions and at an outermost surface of the inner liner.

**EXAMPLE 436:** The method of any examples herein, particularly examples **Error! Reference source not found.-Error! Reference source not found.,** wherein the tie layer comprises polyurethane, maleic anhydride modified polyolefin, ethylene acrylic acid copolymer, ethylene acrylate copolymer, ethylene glycidyl methacrylate copolymer, ethylene acrylic esters glycidyl methacrylate terpolymer, ethylene acrylic esters, maleic anhydride terpolymer, or any copolymers or blends thereof.

**EXAMPLE 437:** The method of any examples herein, particularly examples **Error! Reference source not found.-Error! Reference source not found.,** wherein the sheet has a thickness from about 0.001" to about 0.020".

**EXAMPLE 438:** The method of any examples herein, particularly examples **Error! Reference source not found.-Error! Reference source not found.,** wherein the tie layer has a thickness from about 0.001" to about 0.003".

**EXAMPLE 439:** The method of any examples herein, particularly examples **Error! Reference source not found.-Error! Reference source not found.,** further comprising disposing at least one lubricious liner between at least a portion of an outermost surface of the inner liner at at least a portion of an innermost surface of the outer layer.

**EXAMPLE 440:** The method of any examples herein, particularly example **Error! Reference source not found.,** wherein the at least one lubricious liner is disposed on the tie layer.

**EXAMPLE 441:** The method of any examples herein, particularly example **Error! Reference source not found.,** wherein the at least one lubricious liner is bonded to the sheet with the tie layer.

**EXAMPLE 442:** The method of any examples herein, particularly examples **Error! Reference source not found.-Error! Reference source not found.,** wherein the at least one lubricious liner comprises PTFE.

**EXAMPLE 443:** The method of any examples herein, particularly example **Error! Reference source not found.,** wherein the at least one lubricious liner is at least partially etched.

**EXAMPLE 444:** The method of any examples herein, particularly examples **Error! Reference source not found.-Error! Reference source not found.,** wherein the at least one lubricious liner has a thickness from about 0.001" to about 0.005".

**EXAMPLE 445:** A method of making a sheath having a proximal end and a distal end and comprising: a) co-extruding a polymer layer and a tie layer to form an elongated tubing; b) positioning at least one lubricious liner at an outer surface of the inner liner; c) cutting the elongated tubing at at least a portion of the circumference along a length of the elongated tubing to form a sheet; d) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; e) positioning an outer layer having a predetermined thickness and having an inner surface and outer surface on the least one lubricious liner to form the sheath; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

**EXAMPLE 446:** A method of making a sheath having a proximal end and a distal end and comprising: a) co-extruding a polymer layer and a tie layer to form an elongated tubing; b) positioning at least one lubricious liner on the tie layer; c) cutting the elongated tubing at at least a portion of the circumference along a length of the elongated tubing to form a sheet; d) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; such that when the sheet is in the spiral configuration, the tie layer is disposed between the overlapping portions and at at least an inner surface of the lumen; and/or such that when the sheet is in the spiral configuration, the tie layer is disposed between the overlapping portions and at an outermost surface of the inner liner; e) positioning an outer layer having a predetermined thickness and having an inner surface and outer surface to form the sheath, and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 447:** The method of any examples herein, particularly examples **Error! Reference source not found.-Error! Reference source not found.** wherein the outer layer comprises a polyether block amide, a styrene-based elastomer, polyurethane, latex, copolymers thereof, blends thereof, or extrudates thereof.

**EXAMPLE 448:** The method of any examples herein, particularly example **Error! Reference source not found.,** wherein the outer layer comprises one or more layers.

**EXAMPLE 449:** The method of any examples herein, particularly example **Error! Reference source not found.** or **Error! Reference source not found.,** wherein at least one layer comprises the styrene-based elastomer.

**EXAMPLE 450:** The method of any examples herein, particularly example **Error! Reference source not found.** or **Error! Reference source not found.,** wherein at least one layer comprises polyurethane.

**EXAMPLE 451:** The method of any examples herein, particularly example **Error! Reference source not found.** or **Error! Reference source not found.,** wherein at least one layer comprises a blend of the styrene-based elastomer and polyurethane.

**EXAMPLE 452:** The method of any examples herein, particularly examples **Error! Reference source not found.-Error! Reference source not found.,** wherein the styrene-based elastomer has a Shore A durometer between 20A to 50A.

**EXAMPLE 453:** The method wherein the outer layer of any examples herein, particularly examples **Error! Reference source not found.-Error! Reference source not found.,** further comprises a first polymer layer comprising a first compound composition comprising: from greater than 0 wt% to less than 100 wt% of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition.

**EXAMPLE 454:** The method of any examples herein, particularly example **Error! Reference source not found.,** wherein the polymer in the first polymer layer composition has a Shore D from about 20D to about 35D.

**EXAMPLE 455:** The method of any examples herein, particularly example **Error! Reference source not found.** or **Error! Reference source not found.,** wherein the polymer in the first polymer layer composition comprises PEBAX^{®}.

**EXAMPLE 456:** The method of any examples herein, particularly example **Error! Reference source not found.** or **Error! Reference source not found.,** wherein the first polymer layer composition comprises polyurethane.

**EXAMPLE 457:** The method of any examples herein, particularly examples **Error! Reference source not found.-Error! Reference source not found.,** wherein the inorganic filler comprises bismuth oxychloride, barium sulfate, bismuth subcarbonate, calcium carbonate, aluminum trihydrate, barite, kaolin clay, limestone, or any combination thereof.

**EXAMPLE 458:** The method of any examples herein, particularly examples **Error! Reference source not found.-Error! Reference source not found.,** wherein the inorganic filler is present in an amount of at least about 10 % based on a total weight of the first compound composition.

**EXAMPLE 459:** The method of any examples herein, particularly examples **Error! Reference source not found.-Error! Reference source not found.,** wherein the inorganic filler is present in an amount of less than about 50 % based on a total weight of the first compound composition.

**EXAMPLE 460:** The method of any examples herein, particularly examples **Error! Reference source not found.-Error! Reference source not found.,** wherein the solid lubricant comprises a PTFE filler.

**EXAMPLE 461:** The method of any examples herein, particularly example **Error! Reference source not found.** wherein the PTFE filler is a powder.

**EXAMPLE 462:** The method of any examples herein, particularly examples **Error! Reference source not found.-Error! Reference source not found.,** wherein the first compound composition further comprises at least one tackiness reducing compound

**EXAMPLE 463:** The method of any examples herein, particularly example **Error! Reference source not found.,** wherein the at least one tackiness reducing compound is present in an amount from about 1 % to about 20 % based on a total weight of the first compound composition.

**EXAMPLE 464:** The method of any examples herein, particularly example **Error! Reference source not found.** or **Error! Reference source not found.,** wherein the at least one tackiness reducing compound comprises ProPell^{™}.

**EXAMPLE 465:** The method of any examples herein, particularly examples **Error! Reference source not found.-Error! Reference source not found.,** wherein the outer layer comprises two or more polymer layers.

**EXAMPLE 466:** The method of any examples herein, particularly example **Error! Reference source not found.,** wherein the outer layer comprises a second polymer layer comprising a second compound composition comprising from greater than 0 wt% to 100 wt% of a second polymer comprising polyether block amide, a polyurethane, or a composition thereof.

**EXAMPLE 467:** The method of any examples herein, particularly example **Error! Reference source not found.,** wherein the second compound composition further comprises up to 20 % of tackiness reducing additive based on a total weight of the second compound composition.

**EXAMPLE 468:** The method of any examples herein, particularly example **Error! Reference source not found.** or **Error! Reference source not found.,** wherein the second polymer layer composition comprises PEBAX^{®}.

**EXAMPLE 469:** The method of any examples herein, particularly examples **Error! Reference source not found.-Error! Reference source not found.,** wherein the second polymer layer composition comprises polyurethane.

**EXAMPLE 470:** The method of any examples herein, particularly examples **Error! Reference source not found.-Error! Reference source not found.,** wherein the second polymer has a Shore D Durometer from about 20D to about 35D.

**EXAMPLE 471:** The method of any examples herein, particularly example **Error! Reference source not found.,** wherein the second polymer has a Shore D Durometer of about 25D to about 35D.

**EXAMPLE 472:** The method of any examples herein, particularly examples **Error! Reference source not found.-Error! Reference source not found.,** wherein the second compound composition is substantially free of an inorganic filler.

**EXAMPLE 473:** The method of any examples herein, particularly examples **Error! Reference source not found.-Error! Reference source not found.,** wherein the second compound composition is substantially free of a lubricant solid.

**EXAMPLE 474:** The method of any examples herein, particularly examples **Error! Reference source not found.-Error! Reference source not found.,** wherein the outer layer has a predetermined thickness, and wherein at least about 50% of the predetermined thickness comprises the first and/or the second compound composition comprising the first and/or the second polymer having a Shore D Durometer from about 20D to about 35D.

**EXAMPLE 475:** The method of any examples herein, particularly example **Error! Reference source not found.,** wherein the predetermined thickness is up to 6 mils.

**EXAMPLE 476:** The method of any examples herein, particularly example **Error! Reference source not found.** or **Error! Reference source not found.,** wherein the predetermined thickness of the outer layer varies along the length of the sheath.

**EXAMPLE 477:** The method of any examples herein, particularly example **Error! Reference source not found.,** wherein the predetermined thickness of the outer layer is greater at the proximal end.

**EXAMPLE 478:** The method of any examples herein, particularly example **Error! Reference source not found.** or **Error! Reference source not found.,** wherein the predetermined thickness of the outer layer is smaller at the distal end as compared to the predetermined thickness of the outer layer at the proximal end.

**EXAMPLE 479:** The method of any examples herein, particularly examples **Error! Reference source not found.-Error! Reference source not found.,** wherein the first polymer layer has a thickness of about 0.001" to about 0.003".

**EXAMPLE 480:** The method of any examples herein, particularly examples **Error! Reference source not found.-Error! Reference source not found.,** wherein the second polymer layer has a thickness of about 0.002" to about 0.004".

**EXAMPLE 481:** The method of any examples herein, particularly examples **Error! Reference source not found.-Error! Reference source not found.,** wherein the first polymer layer defines an inner surface of the outer layer.

**EXAMPLE 482:** The method of any examples herein, particularly examples **Error! Reference source not found.-Error! Reference source not found.,** wherein the second polymer layer defines an outer surface of the outer layer.

**EXAMPLE 483:** The method of any examples herein, particularly examples **Error! Reference source not found.-Error! Reference source not found.,** wherein the first polymer layer defines an outer surface of the outer layer.

**EXAMPLE 484:** The method of any examples herein, particularly example **Error! Reference source not found.,** wherein the second polymer layer defines an inner surface of the outer layer.

**EXAMPLE 485:** The method of any examples herein, particularly examples **Error! Reference source not found.-Error! Reference source not found.,** wherein one or more additional polymer layers are disposed between the first polymer layer and the second polymer layer.

**EXAMPLE 486:** The method of any examples herein, particularly examples **Error! Reference source not found.-Error! Reference source not found.** or **Error! Reference source not found.-Error! Reference source not found.,** wherein the outer layer further comprises a second polymer layer.

**EXAMPLE 487:** The method of any examples herein, particularly example **Error! Reference source not found.,** wherein the second polymer layer comprises polyurethane.

**EXAMPLE 488:** The method of any examples herein, particularly example **Error! Reference source not found.** or **Error! Reference source not found.,** wherein the second layer of polyurethane and the first polymer layer are coextruded to form a bump tubing.

**EXAMPLE 489:** The method of any examples herein, particularly example **Error! Reference source not found.,** wherein the bump tubing has a predetermined length substantially similar to a length of the sheath.

**EXAMPLE 490:** The method of any examples herein, particularly examples **Error! Reference source not found. -Error! Reference source not found.,** wherein the first polymer layer defines an inner surface of the outer layer.

**EXAMPLE 491:** The method of any examples herein, particularly example **Error! Reference source not found.,** wherein the second polymer layer defines an outer surface of the outer layer.

**EXAMPLE 492:** The method of any examples herein, particularly examples **Error! Reference source not found.-Error! Reference source not found.,** wherein the first polymer layer has a thickness from about 0.001" to about 0.010".

**EXAMPLE 493:** The method of any examples herein, particularly example **Error! Reference source not found.,** wherein the thickness of the first polymer layer is uniform along the length of the sheath.

**EXAMPLE 494:** The method of any examples herein, particularly example **Error! Reference source not found.,** wherein the thickness of the first polymer layer varies along the length of the sheath.

**EXAMPLE 495:** The method of any examples herein, particularly example **Error! Reference source not found.,** wherein the thickness of the first polymer layer is greater at the proximal end as compared to the thickness of the first polymer layer along other portions of the sheath.

**EXAMPLE 496:** The method of any examples herein, particularly example **Error! Reference source not found.** or **Error! Reference source not found.,** the thickness of the first polymer layer is smaller at the distal end as compared to the thickness of the first polymer layer at the proximal end.

**EXAMPLE 497:** The method of any examples herein, particularly examples **Error! Reference source not found.-Error! Reference source not found.,** wherein the second polymer layer has a thickness from about 0.001" to about 0.010".

**EXAMPLE 498:** The method of any examples herein, particularly example **Error! Reference source not found.,** wherein the thickness of the second polymer layer is uniform along the length of the sheath.

**EXAMPLE 499:** The method of any examples herein, particularly example **Error! Reference source not found.,** wherein the thickness of the second polymer layer varies along the length of the sheath.

**EXAMPLE 500:** The method of any examples herein, particularly example **Error! Reference source not found.,** wherein the thickness of the second polymer layer is greater at the proximal end as compared to the thickness of the second polymer layer along other portions of the sheath.

**EXAMPLE 501:** The method of any examples herein, particularly example **Error! Reference source not found.** or **Error! Reference source not found.,** the thickness of the second polymer layer is smaller at the distal end as compared to the thickness of the second polymer layer at the proximal end.

**EXAMPLE 502:** The method of any examples herein, particularly examples **Error! Reference source not found.-Error! Reference source not found.,** wherein the second polymer layer has a Shore A durometer from about 30A to about 80A.

**EXAMPLE 503:** The method of any examples herein, particularly examples **Error! Reference source not found.-Error! Reference source not found.,** wherein the outer layer has a total predetermined thickness, and wherein at least about 50% of the total predetermined thickness comprises the first compound composition comprising the first polymer having a Shore D Durometer from about 20D to about 35D.

**EXAMPLE 504:** The method of any examples herein, particularly example **Error! Reference source not found.,** wherein the predetermined thickness is up to 0.01".

**EXAMPLE 505:** The method of any examples herein, particularly example **Error! Reference source not found.** or **Error! Reference source not found.,** wherein the total predetermined thickness of the outer layer varies along the length of the sheath.

**EXAMPLE 506:** The method of any examples herein, particularly example **Error! Reference source not found.,** wherein the total predetermined thickness of the outer layer is greater at the proximal end.

**EXAMPLE 507:** The method of any examples herein, particularly example **Error! Reference source not found.** or **Error! Reference source not found.,** wherein the total predetermined thickness of the outer layer is smaller at the distal end as compared to the predetermined thickness of the outer layer at the proximal end.

**EXAMPLE 508:** The method of any examples herein, particularly examples **Error! Reference source not found.-Error! Reference source not found.,** wherein the first polymer layer and the second polymer layers are formed separately and wherein the second polymer layer at least partially overlies the first polymer layer.

**EXAMPLE 509:** The method of any examples herein, particularly example **Error! Reference source not found.,** wherein the first polymer layer and the second polymer layer have a different length.

**EXAMPLE 510:** The method of any examples herein, particularly example **Error! Reference source not found.,** wherein the first polymer layer has a length that is shorter than the length of the second polymer layer.

**EXAMPLE 511:** The method of any examples herein, particularly example **Error! Reference source not found.** or **Error! Reference source not found.,** wherein the first polymer layer is disposed at the proximal end of the sheath and has a length from about 5 cm to about 15 cm.

**EXAMPLE 512:** The method of any examples herein, particularly examples **Error! Reference source not found.-Error! Reference source not found.,** wherein the second polymer layer has a length substantially identical to the length of the sheath.

**EXAMPLE 513:** The method of any examples herein, particularly examples **Error! Reference source not found.-Error! Reference source not found.,** wherein the first polymer layer has a thickness that is uniform along the length of the first polymer layer.

**EXAMPLE 514:** The method of any examples herein, particularly examples **Error! Reference source not found.-Error! Reference source not found.,** wherein the first polymer layer has a thickness that varies along the length of the first polymer layer.

**EXAMPLE 515:** The method of any examples herein, particularly example **Error! Reference source not found.** or **Error! Reference source not found.,** wherein the thickness of the first polymer layer is from about 0.001" to about 0.006".

**EXAMPLE 516:** The method of any examples herein, particularly examples **Error! Reference source not found.-Error! Reference source not found.,** wherein the second polymer layer has a thickness that is uniform along the length of the second polymer layer.

**EXAMPLE 517:** The method of any examples herein, particularly examples **Error! Reference source not found.-Error! Reference source not found.,** wherein the second polymer layer has a thickness that varies along the length of the second polymer layer.

**EXAMPLE 518:** The method of any examples herein, particularly example **Error! Reference source not found.** or **Error! Reference source not found.,** wherein the thickness of the second polymer layer is from about 0.001" to about 0.010".

**EXAMPLE 519:** The method of any examples herein, particularly examples **Error! Reference source not found.-Error! Reference source not found.,** wherein a tie layer is disposed between the first polymer layer and the second polymer layer.

**EXAMPLE 520:** A method of making a sheath having a proximal end and a distal end and comprising: a) co-extruding an elongated bump tubing comprising a first polymer layer and a second polymer layer; wherein i) the first polymer layer comprises: a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; ii) the second polymer layer comprises a polyurethane; wherein the first polymer layer defines an inner surface of the tubing and the second polymer layer defines an outer surface of the tubing; b) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; and c) disposing the elongated bump tubing on the sheath such that the elongated bump tubing forms an outer layer of the sheath, and wherein the elongated tube is positioned at at least the proximal end of the sheath and extending along at least a portion of a length of the sheath, wherein the elongated bump tubing is configured to reversibly expand from an initial diameter d₀ in an unexpended position to an expanded diameter dₑ in an expanded position upon passage of a medical device; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 521:** A method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing a first polymer layer comprising a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; wherein the first polymer layer is disposed over a proximal portion of the inner liner and has a length from about 5 cm to about 15 cm; c) disposing a second polymer layer comprising polyurethane over the first polymer layer, wherein the second polymer layer extends along a length of the sheath; wherein the first polymer layer and the second polymer layer together form an outer layer of the sheath that is configured to reversibly expand from an initial diameter d₀ in an unexpended position to an expanded diameter dₑ in an expanded position upon passage of a medical device; and wherein the variable diameter inner liner is configured to expand from a first rest diameter *dᵣ* to a second expanded diameter dₑ by sliding the first edge of the sheet along at least a portion of the inner surface and sliding the second edge of the sheet along the at least a portion of the outer surface, during application of a radial force.

**EXAMPLE 522:** The method of any examples herein, particularly examples **Error! Reference source not found.-Error! Reference source not found.,** further comprising a step of bonding at least a portion of an innermost surface of the outer layer to at least a portion of an outermost surface of the inner liner.

**EXAMPLE 523:** The method of any examples herein, particularly example **Error! Reference source not found.,** wherein the step of bonding comprises laser welding, compression bonding, and/or selective ultrasonic welding.

**EXAMPLE 524:** The method of any examples herein, particularly example **Error! Reference source not found.** or **Error! Reference source not found.,** wherein prior to the step of bonding, the formed sheath in the rest unexpanded configuration is disposed on a mandrel.

**EXAMPLE 525:** The method of any examples herein, particularly example **Error! Reference source not found.** or **Error! Reference source not found.,** wherein a laser beam is positioned such that a bond having a predetermined size is formed at a predetermined portion of the sheath and wherein the laser beam is configured to move along a longitudinal axis of the sheath to a predetermined distance at conditions effective to form the bond.

**EXAMPLE 526:** The method of any examples herein, particularly example **Error! Reference source not found.,** wherein the conditions effective to form the predetermined bond, comprise a predetermined laser power, a predetermined laser focus, a predetermined focus position, a predetermined weld start angle, and/or welding distance.

**EXAMPLE 527:** The method of any examples herein, particularly example **Error! Reference source not found.** or **Error! Reference source not found.,** wherein the mandrel is configured to rotate the sheath such that the predetermined portion of the sheath is exposed to the laser beam.

**EXAMPLE 528:** The method of any examples herein, particularly examples **Error! Reference source not found.-Error! Reference source not found.,** wherein the predetermined portion where the bond is formed is substantially free of a lubricant and/or a tie layer between at least a portion of an outermost surface of the inner liner and at least a portion of an innermost surface of the outer layer.

**EXAMPLE 529:** The method of any examples herein, particularly examples **Error! Reference source not found.-Error! Reference source not found.,** wherein the predetermined portion extends longitudinally along a length of the sheath.

**EXAMPLE 530:** The method of any examples herein, particularly examples **Error! Reference source not found.-Error! Reference source not found.,** wherein two or more portions of an innermost surface of the outer layer are bonded to two or more portions of an outermost surface, the inner liner extends longitudinally along a length of the sheath.

**EXAMPLE 531:** The method of any examples herein, particularly example **Error! Reference source not found.,** wherein the two or more portions of an innermost surface of the outer layer bonded to two or more portions of an outermost surface of the inner liner disposed in a predetermined pattern.

**EXAMPLE 532:** The method of any examples herein, particularly examples **Error! Reference source not found.-Error! Reference source not found.,** wherein at least a portion of the mandrel with the formed sheath in the rest unexpended configuration is inserted into a radial compression head bonder comprising a collapsible aperture configured to compress the sheath to a predetermined diameter.

**EXAMPLE 533:** The method of any examples herein, particularly example **Error! Reference source not found.,** wherein the radial compression head bonder comprises a plurality of dies, wherein at least one of a plurality of dies is heated to form the bond at the preterminal portion of the sheath.

**EXAMPLE 534:** The method of any examples herein, particularly example 374, wherein the sheath is configured to move horizontally, and compression/heating bonding is repeated.

**EXAMPLE 535:** The method of any examples herein, particularly examples **Error! Reference source not found.-Error! Reference source not found.,** wherein the compression force is from about 0 to about 10 pounds.

**EXAMPLE 536:** The method of any examples herein, particularly examples **Error! Reference source not found.-Error! Reference source not found.,** wherein the predetermined portion is substantially free of a lubricant and/or a tie layer between at least a portion of an outermost surface of the inner liner and at least a portion of an innermost surface of the outer layer.

**EXAMPLE 537:** The method of any examples herein, particularly example **Error! Reference source not found.,** wherein the bond is formed by the selective ultrasonic welding using an ultrasonic horn.

**EXAMPLE 538:** A method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing an outer layer over the inner liner to form the sheath; c) positioning the sheath on a mandrel configured to rotate; d) aligning the mandrel with a laser beam configured to move along a longitudinal axis of the sheath to a predetermined distance at conditions effective to form the bond; e) forming a bond at a predetermined portion of the sheath between at least a portion of an outermost surface of the inner liner and at least a portion of an innermost surface of the outer layer, wherein the predetermined portion is substantially free of a lubricant and/or a tie layer; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 539:** A method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing an outer layer over the inner liner to form the sheath; c) positioning the sheath on a mandrel configured to rotate; d) inserting the mandrel into a radial compression head bonder comprising a collapsible aperture configured to compress the sheath to a predetermined diameter and wherein the radial compression head bonder comprises a plurality of dies, wherein at least one of a plurality of dies is heated to form the bond at the preterminal portion of the sheath; e) forming a bond at a predetermined portion of the sheath between at least a portion of an outermost surface of the inner liner and at least a portion of an innermost surface of the outer layer, wherein the predetermined portion is substantially free of a lubricant and/or a tie layer; and wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 540:** The method of any examples herein, particularly examples **Error! Reference source not found.-Error! Reference source not found.,** wherein the outer layer extends from the proximal end of the sheath to the distal end of the sheath.

**EXAMPLE 541:** The method of any examples herein, particularly example **Error! Reference source not found.,** further comprising a step of disposing a reinforcing jacket having a proximal end and a distal end over at least a portion of an outer surface of the outer layer.

**EXAMPLE 542:** The method of any examples herein, particularly example **Error! Reference source not found.,** wherein the proximal end of the reinforcing jacket is abutting the proximal end of the sheath.

**EXAMPLE 543:** The method of any examples herein, particularly example **Error! Reference source not found.** or **Error! Reference source not found.,** wherein the reinforcing jacket has a length of about 5 to about 15 cm.

**EXAMPLE 544:** The method of any examples herein, particularly examples **Error! Reference source not found.-Error! Reference source not found.,** further comprising substantially seamlessly bonding the distal end of the reinforcing jacket to the at least a portion of the outer surface of the outer layer.

**EXAMPLE 545:** The method of any examples herein, particularly examples **Error! Reference source not found.-Error! Reference source not found.,** wherein the reinforcing jacket comprises an elastomeric material having a Shore hardness of about 10A to about 80A.

**EXAMPLE 546:** The method of any examples herein, particularly examples **Error! Reference source not found.-Error! Reference source not found.,** wherein the elastomeric material comprises a polyether block amide, styrene-based elastomer, polyurethane, latex, copolymers thereof, blends thereof or co-extrudates thereof.

**EXAMPLE 547:** The method of any examples herein, particularly examples **Error! Reference source not found.-Error! Reference source not found.,** wherein the reinforcing jacket comprises a reinforcing element.

**EXAMPLE 548:** The method of any examples herein, particularly example **Error! Reference source not found.,** wherein the reinforcing element is embedded in the elastomeric material.

**EXAMPLE 549:** The method of any examples herein, particularly examples **Error! Reference source not found.** or **Error! Reference source not found.,** wherein the reinforcing element limits expansion of the outer layer to a predetermined diameter effective to prevent ballooning of the outer layer of the sheath.

**EXAMPLE 550:The** method of any examples herein, particularly examples **Error! Reference source not found.-Error! Reference source not found.,** wherein the reinforcing element comprises a plurality of filaments arranged in a braid or coil configuration.

**EXAMPLE 551:** The method of any examples herein, particularly examples **Error! Reference source not found.-Error! Reference source not found.,** wherein the reinforcing element comprises a plurality of filaments disposed in a plurality of circumferential rows within the reinforcing jacket, wherein each of plurality has a sinusoidal form or any irregular form, or any combination thereof.

**EXAMPLE 552:** The method of any examples herein, particularly example **Error! Reference source not found.** or **Error! Reference source not found.,** wherein the plurality of filaments comprise stainless steel, nitinol, a polymer material, or composite material.

**EXAMPLE 553:** The method of any examples herein, particularly example **Error! Reference source not found.,** wherein the filament is a round filament or a flat filament.

**EXAMPLE 554:** The method of any examples herein, particularly example **Error! Reference source not found.** or **Error! Reference source not found.,** wherein the polymer material is polyester or nylon.

**EXAMPLE 555:** The method of any examples herein, particularly examples **Error! Reference source not found.-Error! Reference source not found.,** wherein the round filament has a diameter of less than about 0.015".

**EXAMPLE 556:** The method of any examples herein, particularly examples **Error! Reference source not found.-Error! Reference source not found.,** wherein the flat filament has a height of less than about 0.006" and a width from greater than about 0.003" to about 0.015".

**EXAMPLE 557:** The method of any examples herein, particularly examples **Error! Reference source not found.-Error! Reference source not found.,** wherein the braid has a per inch crosses (PIC) count of less than 50.

**EXAMPLE 558:** The method of any examples herein, particularly examples **Error! Reference source not found.-Error! Reference source not found.,** wherein the proximal end of the reinforcing jacket is not bonded to the outer layer.

**EXAMPLE 559:** The method of any examples herein, particularly examples **Error! Reference source not found.-Error! Reference source not found.,** further comprising disposing a tie layer, the outer layer and the reinforcing jacket.

**EXAMPLE 560:** The method of any examples herein, particularly examples **Error! Reference source not found.-Error! Reference source not found.,** wherein the outer layer does not extend to the proximal end of the sheath and wherein at least a portion of the inner liner at the proximal end of the sheath is substantially free of the outer layer.

**EXAMPLE 561:** The method of any examples herein, particularly example **Error! Reference source not found.,** wherein the at least a portion of the inner liner at the proximal end of the sheath that is substantially free of the outer layer is from about 5 cm to about 15 cm.

**EXAMPLE 562:** The method of any examples herein, particularly example **Error! Reference source not found.** or **Error! Reference source not found.,** wherein a proximal end of the outer layer is bonded to at least a portion of the outer surface of the inner liner.

**EXAMPLE 563:** The method of any examples herein, particularly examples **Error! Reference source not found.-Error! Reference source not found.,** further comprising disposing a reinforcing jacket having a proximal end and a distal end over the at least a portion of the outer surface of the inner liner at the proximal end of the sheath that is not overlaid by the outer layer.

**EXAMPLE 564:** The method of any examples herein, particularly example **Error! Reference source not found.,** wherein the proximal end of the reinforcing jacket is abutting the proximal end of the sheath.

**EXAMPLE 565:** The method of any examples herein, particularly example **Error! Reference source not found.** or **Error! Reference source not found.,** wherein the proximal end of the reinforcing jacket is at least partially bonded to at least a portion of a proximal end of the outer surface of the inner liner.

**EXAMPLE 566:** The method of any examples herein, particularly examples **Error! Reference source not found.-Error! Reference source not found.,** wherein the reinforcing jacket has a length of about 5 to about 15 cm.

**EXAMPLE 567:** The method of any examples herein, particularly examples **Error! Reference source not found.-Error! Reference source not found.,** wherein the distal end of the reinforcing jacket is abutting or at least partially overlays a proximal portion of the outer layer.

**EXAMPLE 568:** The method of any examples herein, particularly example **Error! Reference source not found.,** wherein the distal end of the reinforcing jacket is substantially seamlessly bonded to the at least a portion of the proximal portion of the outer surface of the outer layer.

**EXAMPLE 569:** The method of any examples herein, particularly examples **Error! Reference source not found.-Error! Reference source not found.,** wherein the reinforcing jacket comprises an elastomeric material having a Shore hardness of about 10A to about 80A.

**EXAMPLE 570:** The method of any examples herein, particularly examples **Error! Reference source not found.-Error! Reference source not found.,** wherein the elastomeric material comprises a polyether block amide, styrene-based elastomer, polyurethane, latex, copolymers thereof, blends thereof or co-extrudates thereof.

**EXAMPLE 571:** The method of any examples herein, particularly examples **Error! Reference source not found.-Error! Reference source not found.,** wherein the reinforcing jacket comprises a reinforcing element.

**EXAMPLE 572:** The method of any examples herein, particularly example **Error! Reference source not found.,** wherein the reinforcing element is embedded in the elastomeric material.

**EXAMPLE 573:** The method of any examples herein, particularly examples **Error! Reference source not found.** or **Error! Reference source not found.,** wherein the reinforcing element limits expansion of the outer layer to a predetermined diameter effective to prevent ballooning of the outer layer of the sheath.

**EXAMPLE 574:** The method of any examples herein, particularly examples **Error! Reference source not found.-Error! Reference source not found.,** wherein the reinforcing element comprises a plurality of filaments arranged in a braid or coil configuration.

**EXAMPLE 575:** The method of any examples herein, particularly examples **Error! Reference source not found.-Error! Reference source not found.,** wherein the reinforcing element comprises a plurality of filaments disposed in a plurality of circumferential rows within the reinforcing jacket, wherein each of plurality has a sinusoidal form or any irregular form, or any combination thereof.

**EXAMPLE 576:** The method of any examples herein, particularly example **Error! Reference source not found.** or **Error! Reference source not found.,** wherein the plurality of filaments comprise stainless steel, nitinol, a polymer material, or composite material.

**EXAMPLE 577:** The method of any examples herein, particularly example **Error! Reference source not found.,** wherein the filament is a round filament or a flat filament.

**EXAMPLE 578:** The method of any examples herein, particularly example **Error! Reference source not found.** or **Error! Reference source not found.,** wherein the polymer material is polyester or nylon.

**EXAMPLE 579:** The method of any examples herein, particularly examples **Error! Reference source not found.-Error! Reference source not found.,** wherein the round filament has a diameter of less than about 0.015".

**EXAMPLE 580:** The method of any examples herein, particularly examples **Error! Reference source not found.-Error! Reference source not found.,** wherein the flat filament has a height of less than about 0.006" and a width from greater than about 0.003" to about 0.015".

**EXAMPLE 581:** The method of any examples herein, particularly examples **Error! Reference source not found.-Error! Reference source not found.,** wherein the braid has a per inch crosses (PIC) count of less than 50.

**EXAMPLE 582:** The method of any examples herein, particularly examples **Error! Reference source not found.-Error! Reference source not found.,** wherein a tie layer is disposed between the inner liner and the reinforcing jacket.

**EXAMPLE 583:** A method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing an outer layer over at least a portion of the inner liner to form the sheath; c) disposing a reinforcing jacket having a proximal end and a distal end over at least a portion of the outer layer; wherein the reinforcing jacket comprises an elastomer and a reinforcing element; and d) substantially seamlessly bonding the distal end of the reinforcing jacket to at least a portion of the outer layer; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 584:** The method of any examples herein, particularly examples **Error! Reference source not found.-Error! Reference source not found.,** further comprising disposing a ballooning guard having a proximal end and a distal end over at least a portion of the outer layer, wherein the ballooning guard is configured to remain outside of a subject's vessel and to maintain hemostasis.

**EXAMPLE 585:** The method of any examples herein, particularly example **Error! Reference source not found.,** further comprising connecting the proximal end of the ballooning guard to a most proximal portion of the outer layer and/or a hub of the sheath.

**EXAMPLE 586:** The method of any examples herein, particularly example **Error! Reference source not found.** or **Error! Reference source not found.,** wherein the distal end of the ballooning guard radially circumscribes at least a portion of the outer layer and wherein the distal end is not bonded to the outer layer.

**EXAMPLE 587:** The method of any examples herein, particularly examples **Error! Reference source not found.-Error! Reference source not found.,** wherein the distal end is configured to seal the sheath against a subject's skin.

**EXAMPLE 588:** The method of any examples herein, particularly examples **Error! Reference source not found.-Error! Reference source not found.,** wherein the ballooning guard is configured to adjust a length of the guard as a function of an insertion depth of the inner liner and outer layer of the sheath into a subject's vessel.

**EXAMPLE 589:** The method of any examples herein, particularly examples **Error! Reference source not found.-Error! Reference source not found.,** wherein the ballooning guard has a collapsible length.

**EXAMPLE 590:** The method of any examples herein, particularly examples **Error! Reference source not found.-Error! Reference source not found.,** wherein the ballooning guard has an inner diameter sized to have substantially no resistance to expansion of the outer layer to a predetermined diameter such that there is substantially no increase in a push force to advance the delivery system through the sheath.

**EXAMPLE 591:** The method of any examples herein, particularly example **Error! Reference source not found.,** wherein at the predetermined diameter of the outer layer, the ballooning guard is configured to resist to a blood pressure increase.

**EXAMPLE 592:** The method of any examples herein, particularly examples **Error! Reference source not found.-Error! Reference source not found.,** wherein the ballooning guard comprises a braided sleeve comprising a plurality of filaments.

**EXAMPLE 593:** The method of any examples herein, particularly example **Error! Reference source not found.,** wherein at least a portion of the filaments at the distal end of the ballooning guard are connected to each other.

**EXAMPLE 594:** The method of any examples herein, particularly examples **Error! Reference source not found.** or **Error! Reference source not found.,** wherein the braided or coiled sleeve comprises a polymeric sleeve.

**EXAMPLE 595:** The method of any examples herein, particularly examples **Error! Reference source not found.** or **Error! Reference source not found.,** wherein the braided or coiled sleeve comprises a fabric.

**EXAMPLE 596:** The method of any examples herein, particularly examples **Error! Reference source not found.-Error! Reference source not found.,** wherein the ballooning guard comprises the e-PTFE tubing, the ballooning guard comprises the e-PTFE tubing, corrugated tubing, or any polymeric tubing having a shape that is configured to be compressed.

**EXAMPLE 597:** The method of any examples herein, particularly examples **Error! Reference source not found.-Error! Reference source not found.,** wherein the ballooning guard comprises a corrugated tubing.

**EXAMPLE 598:** A method of making a sheath having a proximal end and a distal end and comprising: a) forming an inner liner by rolling the sheet into a spiral configuration such that at least a portion of an inner surface of the sheet overlays at least a portion of an outer surface of the sheet and wherein a first longitudinal edge of the sheet is slidable along at least a portion the inner surface of the sheet and a second longitudinal edge is slidable along at least a portion of the outer surface of the sheet, wherein the inner surface of the sheet defines a lumen of the sheath having a longitudinal axis; b) disposing an outer layer over at least a portion of the inner liner to form the sheath; c) disposing a ballooning guard having a proximal end and a distal end over at least a portion of the outer layer, wherein the ballooning guard is configured to remain outside of a subject's vessel and to maintain hemostasis; d) connecting the proximal end of the ballooning guard to a most proximal portion of the outer layer and/or a hub of the sheath, wherein the distal end of the ballooning guard radially circumscribes at least a portion of the outer layer and wherein the distal end is not bonded to the outer layer; and wherein the ballooning guard is configured to adjust a length of the guard as a function of an insertion depth of the inner liner and outer layer of the sheath into a subject's vessel; wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial force.

**EXAMPLE 599:** The method of any examples herein, particularly examples **Error! Reference source not found.-Error! Reference source not found.,** further comprising disposing a hydrophilic coating over at least a portion of an outermost surface of the sheath.

**EXAMPLE 600:** A method of delivering a medical device through a sheath, the method comprising: introducing the medical device into the sheath of any examples herein, particularly examples **Error! Reference source not found.-32;** advancing the medical device through the sheath such that the medical device exerts a radially outward force on an inner surface of the sheath, locally expanding the sheath by sliding the overlapping portions of the sheath to locally expanding at least a portion of the sheath; and locally contracting the expanded sheath back to an unexpanded configuration by radially compressing the expanded portion with a radially inward bias of the outer layer of the sheath.

**EXAMPLE 601:** The method of any examples herein, particularly example **Error! Reference source not found.,** wherein the medical device is a prosthetic heart valve mounted in a radially crimped state on a delivery apparatus, and the act of advancing the medical device through the sheath comprises advancing the delivery apparatus and the prosthetic heart valve into the vasculature of a patient.

It is understood that the aspects disclosed herein that comprise braid can be substituted or used in addition or alternative with aspects comprising coil.

In view of the many possible aspects to which the principles of the described disclosure can be applied, it should be recognized that the illustrated aspects are only some examples of the disclosure and should not be taken as limiting the scope of the disclosure. Rather, the scope of the disclosure is defined by the following claims. We, therefore, claim as our disclosure all that comes within the scope and spirit of these claims.

**The** application further comprises the following embodiments:
1. A sheath for delivering a medical device, wherein the sheath has a proximal end and a distal end and comprises:
   a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; wherein the sheet comprises a polymer layer;
   an outer layer having a predetermined thickness and having an inner surface and outer surface, and
   wherein the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.
2. The sheath of embodiment 1, wherein when the sheath is in an unexpanded rest state, the first and the second edges of the sheath are substantially aligned in a spaced relationship along a vertical axis passing through a thickness of the sheath.
3. The sheath of embodiment 1 or 2, wherein when the sheet is in an unexpanded rest state, the inner liner comprises at least two layers of the sheet overlaying each other at at least a portion of a circumference of the sheath.
4. The sheath of any one of embodiments 1-3, wherein the sheath is in a rest state, the first edge of the sheet is substantially aligned with a vertical axis passing through a thickness of the sheath and the second edge circumferentially offset from the vertical axis.
5. The sheath of embodiment 4, wherein at at least a portion of a circumference of the sheath, the inner liner comprises one layer of the sheet without any overlaying portion.
6. The sheath of any one of embodiments 1-5, wherein an amount of a lubricant is disposed between at least a portion of the overlying portion of the sheet and at least a portion of the sliding portions of the sheet.
7. The sheath of any one of embodiments 1-6 wherein the disposed lubricant has a predetermined pattern.
8. The sheath of any one of embodiments 1-7, wherein the polymer layer of the sheet comprises a compound material comprising a polyolefin and a lubricious filler.
9. The sheath of embodiment 8, wherein the lubricious filler is present in an amount from about 5 wt % to about 20 wt % of a total weight of the compound material.
10.The sheath of any one of embodiments 1-9, further comprising at least one tie layer.
11.The sheath of embodiment 10, wherein the tie layer is disposed at the outer surface of the sheet such that when the sheet is in the spiral configuration, the tie layer is disposed between the overlapping portions and at an outermost surface of the inner liner.
12.The sheath of any one of embodiments 10-11, wherein the tie layer is coextruded with the sheet.
13.The sheath of any one of embodiments 1-12 further comprising at least one lubricious liner.
14.The sheath of embodiment 13, wherein the at least one lubricious liner is disposed on the tie layer.
15.The sheath of any one of embodiments 1-14, wherein the outer layer comprises a polyether block amide, a styrene-based elastomer, polyurethane, latex, copolymers thereof, blends thereof or extrudates thereof.
16.The sheath of embodiment 15, wherein the outer layer comprises one or more layers.
17.The sheath of embodiment 16, wherein at least one layer comprises the styrene-based elastomer.
18.The sheath of embodiment 16 or 17, wherein at least one layer comprises polyurethane.
19.The sheath of embodiment 16, wherein at least one layer comprises a blend of the styrene-based elastomer and polyurethane.
20.The sheath of any one of embodiments 17-19, wherein the styrene-based elastomer has a Shore A durometer between 20A to 50A.
21.The sheath of any one of embodiments 1-16, wherein the outer layer further comprises a first polymer layer comprising a first compound composition comprising:
   from greater than 0 wt% to less than 100 wt% of a polymer comprising a polyether block amide, a polyurethane, or a combination thereof based on a total weight of the first compound composition;
   less than about 65% of an inorganic filler based on a total weight of the first compound composition; and
   up to about 20 % of a solid lubricant filler based on a total weight of the first compound composition.
22.The sheath of embodiment 21, wherein the polymer in the first polymer layer composition has a Shore D from about 20D to about 35D.
23.The sheath of any one of embodiments 21-22, wherein the inorganic filler comprises bismuth oxychloride, barium sulfate, bismuth subcarbonate, calcium carbonate, aluminum trihydrate, barite, kaolin clay, limestone, or any combination thereof.
24.The sheath of any one of embodiments 21-23, wherein the inorganic filler is present in an amount of at least about 10 % based on a total weight of the first compound composition.
25.The sheath of any one of embodiments 21-24, wherein the first compound composition further comprises at least one tackiness reducing compound.
26.The sheath of embodiment 25, wherein the at least one tackiness reducing compound is present in an amount from about 1 % to about 20 % based on a total weight of the first compound composition.
27.The sheath of any one of embodiments 21-26, wherein the outer layer comprises a second polymer layer comprising a second compound composition comprising from greater than 0 wt% to 100 wt% of a second polymer comprising polyether block amide, a polyurethane, or a composition thereof.
28.The sheath of embodiment 27, wherein the second polymer has a Shore D Durometer from about 20D to about 35D.
29.The sheath of any one of embodiments 27-28, wherein the second compound composition is substantially free of an inorganic filler.
30.The sheath of any one of embodiments 27-29, wherein the second compound composition is substantially free of a solid lubricant filler.
31.The sheath of any one of embodiments 27-30, wherein one or more additional polymer layers are disposed between the first polymer layer and the second polymer layer.
32.The sheath of any one of embodiments 1-31, further comprising a hydrophilic coating disposed at an outermost surface of the sheath.

## Claims

1. A sheath (8; 100; 600C) for delivering a medical device, wherein the sheath (8; 100; 600C) comprises:
a variable diameter inner liner (108; 202) comprising:
a sheet having a first edge (202a) and a second edge (202b) and defined by an inner surface and an outer surface,
wherein the sheet is wound in a spiral configuration such that at least a portion of the inner surface of the sheet overlays at least a portion of the outer surface of the sheet,
wherein the first edge (202a) of the sheet is slidable along at least a portion of the inner surface of the sheet and the second edge (202b) is slidable along at least a portion of the outer surface of the sheet,
wherein the inner surface of the sheet defines a lumen (201) of the sheath (8; 600C; 2502) having a longitudinal axis, and
an outer layer (110; 206) having an inner surface and an outer surface and extending about at least a portion of the variable diameter inner liner (202) such that the inner surface of the outer layer (110; 206) is positioned adjacent to the outer surface of the inner liner (108; 202),
wherein the outer layer comprises:
at least one layer of a first elastomeric polymer having a predetermined thickness and having an inner surface and outer surface,
wherein the variable diameter inner liner (108; 202) is configured to expand from a predetermined rest diameter (dᵣ) to an expanded diameter (dₑ) by sliding the first edge (202a) of the sheet along at least a portion of the inner surface and sliding the second edge (202b) of the sheet along the at least a portion of the outer surface, during application of a radial outward force by passage of a medical device through the lumen of the inner liner (108; 202) and
a lubricant disposed along at least a portion of the outer surface of the inner liner.

2. The sheath (8; 100; 600C) of claim 1, wherein the rest diameter dᵣ, varies along the longitudinal axis of the lumen of the inner liner (108; 202).

3. The sheath (8; 100; 600C) of claim 2, wherein the rest diameter dᵣₜ at the proximal end (110; 312) is larger than the rest diameter dᵣ₂ at the distal end (104; 310).

4. The sheath (8; 100; 600C) of claims 1 to 3, wherein the outer layer (110; 206) conforms to the shape of the inner liner (108; 202).

5. The sheath (8; 100; 600C) of any of claims 1 to 4, wherein the sheath (8; 100; 600C) is configured to be inserted into a vessel by passing through the skin of a patient, such that a soft tip portion (102) at a distal end (104; 310) of the sheath (8; 100; 600C) is inserted into the vessel.

6. The sheath (8; 100; 600C) of any of claims 1 to 5, wherein the sheath (8; 100; 600C) includes a proximal flared end portion (114) to facilitate mating with an introducer housing (101).

7. The sheath (8; 100; 600C) of claims 5 or 6, wherein the soft tip portion (102) comprises low density polyethylene (LDPE) and is configured to minimize trauma or damage to the patient's vessels as the sheath is navigated through the vasculature.

8. The sheath (8; 100; 600C) of claims 5 to 7, wherein the soft tip portion (102) is slightly tapered.

9. The sheath (8; 100; 600C) of any of claims 5 to 8, wherein soft tip portion (102) is secured to the distal end (104; 310) of the sheath (8; 100; 600C), by thermally bonding the soft tip portion (102) to the inner and outer layers (108, 110; 202, 206) of the sheath (8; 100; 600C).

10. The sheath (8; 100; 600C) of any of claims 1 to 9, wherein the inner liner (108; 202) comprises a compound material.

11. The sheath (8; 100; 600C) of claim 10, wherein a polymer layer of the sheet used to form the inner liner (108; 202) comprises a compound material comprising a polyolefin and a lubricious filler.

12. The sheath (8; 100; 600C) of claim 11, wherein the lubricious filler comprises one or more of graphene, reduced graphene oxide, carbon black, boron nitride, silicones, talc, polytetrafluorethylene (PTFE), fluorinated ethylene propylene, and the like.

13. The sheath (8; 100; 600C) of any of claims 1 to 12, wherein the lubricant comprises a PTFE-based lubricant or a silicone-based lubricant.

14. The sheath (8; 100; 600C) of any of claims 1 to 13 comprising at least one radiopaque marker (112) positioned near the distal end (104; 310) of the sheath (100).

15. The sheath (8; 100; 600C) of claim 14, wherein the marker (112) is associated with inner liner (108; 202) and/or outer layer (110; 206) of the sheath (8; 100; 600C).
